(19) Europäisches Patentamt
European Patent Office
Office européen des brevets

(11) EP 2 308 994 A1

(12) EUROPEAN PATENT APPLICATION

(43) Date of publication:
**13.04.2011 Bulletin 2011/15**

(21) Application number: **10181096.8**

(22) Date of filing: **21.03.2002**

(51) Int Cl.:
***C12Q 1/68*** *(2006.01)* ***C12P 19/34*** *(2006.01)*
***C07H 21/02*** *(2006.01)* ***C07H 21/04*** *(2006.01)*

(84) Designated Contracting States:
**AT BE CH CY DE DK ES FI FR GB GR IE IT LI LU MC NL PT SE TR**
Designated Extension States:
**AL LT LV MK RO SI**

(30) Priority: **23.03.2001 US 815015**
**09.07.2001 US 303338 P**

(62) Document number(s) of the earlier application(s) in accordance with Art. 76 EPC:
**02707078.8 / 1 430 145**

(27) Previously filed application:
**21.03.2002 PCT/IL02/00231**

(71) Applicant: **YEDA RESEARCH AND DEVELOPMENT CO., LTD.**
**76100 Rehovot (IL)**

(72) Inventors:
- **Livneh, Zvi**
**76100 Rechovot (IL)**
- **Paz-Elizur, Tamar**
**76100 Rechovot (IL)**
- **Blumenstein, Sara**
**52524 Ramat Gan (IL)**

(74) Representative: **Fichter, Robert Arno**
**Dennemeyer & Associates S.A.**
**Patent Department**
**55, rue des Bruyères**
**1274 Howald (LU)**

Remarks:
This application was filed on 28-09-2010 as a divisional application to the application mentioned under INID code 62.

(54) **Methods for determining a risk to develop cancer**

(57) The present invention concerns a method of determining a risk of a subject to develop cancer, the method comprising determining a level of catalytic activity of a DNA repair enzyme in a biological sample of the subject, wherein said DNA repair enzyme is selected from the group consisting of 8-oxoguanine DNA glycosylase (OGG1), AP endonuclease1 (APE1), methylpurine DNA glycosylase (MFG), uracil DNA glycosylase1 (UNG1), uracil DNA glycosylase2 (UNG2), SMUG1, MBD4, mismatch-specific thymine/uracil glycosylase (TDG), enodonuclease III (NTH1), adenine-specific mismatch DNA glycosylase (MYH), 8-oxo-GTPase/8-oxodGTPase (MTH1), dUTPase (DUT), AP endonuclease2 (APE2), deoxyribose phosphate lyase (POLB) and wherein a level of said activity below a predetermined value is indicative of an increased risk of the subject to develop said cancer.

EP 2 308 994 A1

SEQUENCE LISTING

<110> Yeda Research And Development Co. Ltd.
<120> METHODS AND KITS FOR DETERMINING A RISK TO DEVELOP CANCER, FOR EVALUATING AN EFFECTIVENESS AND DOSAGE OF CANCER THERAPY AND FOR CORRELATING BETWEEN AN ACTIVITY OF A DNA REPAIR ENZYME AND A CANCER
<130> FSS-12416-EP

<140> 02707078.8

<141> 21 March 2002

<150> US 09/815,015

<151> 23 March 2001
<160> 14
<170> PatentIn version 3.1
<210> 1
<211> 32
<212> DNA
<213> Artificial sequence

<220>
<223> synthetic oligonucleotide

<220>
<221> misc_feature
<222> (18)..(18)
<223> 8-oxoguanine (8-oxoG)

<400> 1
ccggtgcatg acactgtnac ctatcctcag cg 32

<210> 2
<211> 32
<212> DNA
<213> Artificial sequence

<220>
<223> synthetic oligonucleotide

<400> 2
cgctgaggat aggtcacagt gtcatgcacc gg 32

<210> 3
<211> 2062
<212> DNA
<213> Homo sapiens

<400> 3
ctcccagccc gtctccccgc tccagtttag aacctaattc ccaattcccg gaccgggccc 60

agccctgggc tcttactgtc cgcttttgct gggacctgtt ccacaaatgg gcgtcttctg 120

ccttgggccg tggggttgg gccggaagct gcggacgcct gggaaggggc cgctgcagct 180

cttgagccgc ctctgcgggg accacttgca ggccatccca gccaagaagg ccccggctgg 240

gcaggaggag cctgggacgc cgccctcctc gccgctgagt gccgagcagt tggaccggat 300

ccagaggaac aaggccgcgg ccctgctcag actcgcggcc cgcaacgtgc ccgtgggctt 360

tggagagagc tggaagaagc acctcagcgg ggagttcggg aaaccgtatt ttatcaagct 420

aatgggattt gttgcagaag aaagaaagca ttacactgtt tatccacccc cacaccaagt 480

~

cttcacctgg acccagatgt gtgacataaa agatgtgaag gttgtcatcc tgggacagga 540

tccatatcat ggacctaatc aagctcacgg gctctgcttt agtgttcaaa ggcctgttcc 600

gcctccgccc agtttggaga acatttataa agagttgtct acagacatag aggattttgt 660

tcatcctggc catggagatt tatctgggtg ggccaagcaa ggtgttctcc ttctcaacgc 720

tgtcctcacg gttcgtgccc atcaagccaa ctctcataag gagcgaggct gggagcagtt 780

cactgatgca gttgtgtcct ggctaaatca gaactcgaat ggccttgttt tcttgctctg 840

gggctcttat gctcagaaga agggcagtgc cattgatagg aagcggcacc atgtactaca 900

gacggctcat ccctcccctt tgtcagtgta tagagggttc tttggatgta gacacttttc 960

aaagaccaat gagctgctgc agaagtctgg caagaagccc attgactgga aggagctgtg 1020

atcatcagct gaggggtggc ctttgagaag ctgctgttaa cgtatttgcc agttacgaag 1080

ttccactgaa aattttccta ttaattctta agtactctgc ataaggggga aaagcttcca 1140

gaaagcagcc atgaaccagg ctgtccagga atggcagctg tatccaacca caaacaacaa 1200

aggctaccct ttgaccaaat gtctttctct gcaacatggc ttcggcctaa aatatgcaga 1260

agacagatga ggtcaaatac tcagttggct ctctttatct cccttgcctt tatggtgaaa 1320

caggggagat gtgcaccttt caggcacagc cctagtttgg cgcctgctgc tccttggttt 1380

tgcctggtta gactttcagt gacagatgtt ggggtgtttt tgcttagaaa ggtccccttg 1440

tctcagcctt gcagggcagg catgccagtc tctgccagtt ccactgcccc cttgatcttt 1500

gaaggagtcc tcaggcccct cgcagcataa ggatgttttg caactttcca gaatctggcc 1560

cagaaattag ggctcaattt cctgattgta gtagaggtta agattgctgt gagctttatc 1620

agataagaga ccgagagaag taagctgggt cttgttattc cttgggtgtt ggtggaataa 1680

gcagtggaat ttgaacaagg aagaggagaa aagggaattt tgtctttatg gggtggggtg 1740

attttctcct agggttatgt ccagttgggg tttttaaggc agcacagact gccaagtact 1800

gtttttttta accgactgaa atcactttgg gatatttttt cctgcaacac tggaaagttt 1860

tagtttttta agaagtactc atgcagatat atatatatat atttttccca gtcctttttt 1920

taagagacgg tctttattgg gtctgcacct ccatccttga tcttgttagc aatgctgttt 1980

ttgctgttag tcgggttaga gttggctcta cgcgaggttt gttaataaaa gtttgttaaa 2040

agttcaaaaa aaaaaaaaaa aa 2062

<210> 4
<211> 1570
<212> DNA
<213> Homo sapiens

<400> 4
aacgggatgg ggagctggac cagcagatta tgagcttaca gaaagcctgg cctacatttt 60

actcttttttg gatttcttcc tcatcaagag actgctgcag tgcctgtcat gtgacagcgg 120

catggacata tgccccaggc tttcctgctg ggtccatcc atgagcctgc aggtgccctc 180

atggagcccc agccctgccc tggaagcttg gctgagagct tcctggagga ggagcttcgg 240
ctcaatgctg agctgagcca gctgcagttt tcggagcctg tgggcatcat ctacaatccc 300
gtggagtatg catgggagcc acatcgcaac tacgtgactc gctactgcca gggccccaag 360
gaagtactct tcctgggcat gaaccctgga ccttttggca tggcccagac tggggtgccc 420
tttggggaag taagcatggt ccgggactgg ttgggcattg tggggcctgt gctgacccct 480
ccccaagagc atcctaaacg accagtgctg ggactggagt gcccacagtc agaagtgagt 540
ggtgcccgat tctggggctt tttccggaac ctctgtggac agcctgaggt cttcttccat 600
cactgttttg tccacaatct atgccctctg cttttcctgg ctcccagcgg gcgcaacctt 660
actcctgctg agctgcctgc caagcagcga gaacagcttc ttgggatctg tgatgcagcc 720
ctctgccggc aggtgcagct gctgggggtg cggctggtgg tgggagttgg gcgactggca 780
gagcagcggg cacgacgggc tctggcaggc ctgatgccag aggtccaggt ggaagggctc 840
ctgcatccct ctccccgtaa cccacaggcc aacaagggct gggaggcagt ggccaaggaa 900
agattgaatg agctggggct gctgccactg ctgttgaaat gagtgccctt ggggccttgc 960
atgggacaca ttcaagacct cgaagtcatt cttggccaag cagatgacaa cacatctcct 1020
ggactggagc aaaaggtcct tctgtgcacc ctggtcgctg ggaaacgtat tctttgatct 1080
gttgaactgt cttccaacct gccatggcag ttttgacact actcctgttt gccctcctga 1140
ttcctgcttt ctttaccttt taacattgcc cctttcaggg gaccccactt tgtagggaat 1200
ctgcagaagg tgtgcttttg cacttgcaga ctgctctacc tcagtgtttc cttgggagac 1260
tttattcagc tgagagtgcc ctagacagta acttctaagg tcacgtttac tatttcagag 1320
gaaatatctt gccaggatac ctacccatcc ttatagaaca gttaccttta gctgacccct 1380
ttcctcacag ggaccaagac aaagcatggg acatgaaatt aagagtgaac ttcttatggg 1440
aggctgcagc tggatcagag gaaaaatcca gtgtgacaga gtgcaagtca gaagacctgg 1500
cttttcatcc cagctttgaa acttggaact ttttgattga caaattaata aacctctcta 1560
tgcctcaggc 1570

<210> 5
<211> 2470
<212> DNA
<213> Homo sapiens

<400> 5
ggcggctgta gccgaggggg cggccggaaa gcagcggcgg cgtctggggc gctttcgcaa 60
cattcagacc tcggttgcag cccggtgccg tgagctgaag aggtttcaca tcttactccg 120
ccccacaccc tgggcgttgc ggcgctgggc tcgttgctgc agccggaccc tgctcgatgg 180
gcacgactgg gctggagagt ctgagtctgg gggaccgcgg agctgccccc accgtcacct 240
ctagtgagcg cctagtccca gacccgccga atgacctccg caaagaagat gttgctatgg 300
aattggaaag agtgggagaa gatgaggaac aaatgatgat aaaaagaagc agtgaatgta 360
atcccttgct acaagaaccc atcgcttctg ctcagtttgg tgctactgca ggaacagaat 420

~

```
gccgtaagtc tgtcccatgt ggatgggaaa gagttgtgaa gcaaaggtta tttgggaaga     480
cagcaggaag atttgatgtg tactttatca gcccacaagg actgaagttc agatccaaaa     540
gttcacttgc taattatctt cacaaaaatg gagagacttc tcttaagcca gaagattttg     600
attttactgt actttctaaa aggggtatca agtcaagata taaagactgc agcatggcag     660
ccctgacatc ccatctacaa aaccaaagta acaattcaaa ctggaacctc aggacccgaa     720
gcaagtgcaa aaaggatgtg tttatgccgc caagtagtag ttcagagttg caggagagca     780
gaggactctc taactttact tccactcatt tgcttttgaa agaagatgag ggtgttgatg     840
atgttaactt cagaaaggtt agaaagccca aaggaaaggt gactattttg aaaggaatcc     900
caattaagaa aactaaaaaa ggatgtagga agagctgttc aggttttgtt caaagtgata     960
gcaaaagaga atctgtgtgt aataaagcag atgctgaaag tgaacctgtt gcacaaaaaa    1020
gtcagcttga tagaactgtc tgcatttctg atgctggagc atgtggtgag accctcagtg    1080
tgaccagtga agaaaacagc cttgtaaaaa aaaaagaaag atcattgagt tcaggatcaa    1140
atttttgttc tgaacaaaaa acttctggca tcataaacaa attttgttca gccaaagact    1200
cagaacacaa cgagaagtat gaggatacct ttttagaatc tgaagaaatc ggaacaaaag    1260
tagaagttgt ggaaaggaaa gaacatttgc atactgacat tttaaaacgt ggctctgaaa    1320
tggacaacaa ctgctcacca accaggaaag acttcactgg tgagaaaata tttcaagaag    1380
ataccatccc acgaacacag atagaaagaa ggaaaacaag cctgtatttt tccagcaaat    1440
ataacaaaga agctcttagc cccccacgac gtaaagcctt taagaaatgg acacctcctc    1500
ggtcaccttt taatctcgtt caagaaacac tttttcatga tccatggaag cttctcatcg    1560
ctactatatt tctcaatcgg acctcaggca aaatggcaat acctgtgctt tggaagtttc    1620
tggagaagta tccttcagct gaggtagcaa gaaccgcaga ctggagagat gtgtcagaac    1680
ttcttaaacc tcttggtctc tacgatcttc gggcaaaaac cattgtcaag ttctcagatg    1740
aatacctgac aaagcagtgg aagtatccaa ttgagcttca tgggattggt aaatatggca    1800
acgactctta ccgaattttt tgtgtcaatg agtggaagca ggtgcaccct gaagaccaca    1860
aattaaataa atatcatgac tggctttggg aaaatcatga aaaattaagt ctatcttaaa    1920
ctctgcagct ttcaagctca tctgttatgc atagctttgc acttcaaaaa agcttaatta    1980
agtacaacca accacctttc cagccataga gattttaatt agcccaacta gaagcctagt    2040
gtgtgtgctt tcttaatgtg tgtgccaatg gtggatcttt gctactgaat gtgtttgaac    2100
atgttttgag attttttaa aataaattat tatttgacaa caatccaaaa aaaatacggc     2160
ttttccaatg atgaaatata atcagaagat gaaaaatagt tttaaactat caataataca    2220
aagcaaattt ctatcagcct tgctaaagct aggggcccac taaatatttt tatcggctag    2280
gcgtggtggt gcatgcctgt aatctcggaa ggctgaggca ggaggatcat ttgagctcat    2340
gagggcccag gaggtcaagg cttcagtgag ccatgatcat gccactgcac tccagtctgg    2400
atgacagaga gagaccctgt ctcaaaaaat atatatttaa aaaataaaaa taaaagctga    2460
```

~

ccccaaagac 2470

<210> 6
<211> 3410
<212> DNA
<213> Homo sapiens

<400> 6
gcaccaggcg cccagtggag ccgtttggga gaattgcctg cgccacgcag cggggccgga 60

caggcggtaa ggatctgatt aggctttcga acttgagttt gactgatgtc ttctgtgtgg 120

tgtccgctaa atcccacagc atataggatc agtcgcattg gttataaggt ttgcttctgg 180

ctgggtgcgg tggctcatgc ctgtaatcca acattgggag gccaaggcag gcggaccacc 240

tgaagtcggg agcttgagtc cagccactgt ctgggtactg ccagccatcg ggcccaggtc 300

tctggggttg tcttaccgca gtgagtacca cgcggtacta cagagaccgg ctgcccgtgt 360

gcccggcagg tggagccgcc gcatcagcgg cctcggggaa tggaagcgga gaacgcgggc 420

agctattccc ttcagcaagc tcaagctttt tatacgtttc catttcaaca actgatggct 480

gaagctccta atatggcagt tgtgaatgaa cagcaaatgc cagaagaagt tccagcccca 540

gctcctgctc aggaaccagt gcaagaggct ccaaaaggaa gaaaaagaaa acccagaaca 600

acagaaccaa aacaaccagt ggaacccaaa aaacctgttg agtcaaaaaa atctggcaag 660

tctgcaaaac caaaagaaaa acaagaaaaa attacagaca catttaaagt aaaaagaaaa 720

gtagaccgtt ttaatggtgt ttcagaagct gaacttctga ccaagactct ccccgatatt 780

ttgaccttca atctggacat tgtcattatt ggcataaacc cgggactaat ggctgcttac 840

aaagggcatc attaccctgg acctggaaac catttttgga agtgtttgtt tatgtcaggg 900

ctcagtgagg tccagctgaa ccatatggat gatcacactc taccagggaa gtatggtatt 960

ggatttacca acatggtgga aaggaccacg cccggcagca aagatctctc cagtaaagaa 1020

tttcgtgaag gaggacgtat tctagtacag aaattacaga aatatcagcc acgaatagca 1080

gtgtttaatg gaaaatgtat ttatgaaatt tttagtaaag aagtttttgg agtaaaggtt 1140

aagaacttgg aatttgggct tcagccccat aagattccag acacagaaac tctctgctat 1200

gttatgccat catccagtgc aagatgtgct cagtttcctc gagcccaaga caaagttcat 1260

tactacataa aactgaagga cttaagagat cagttgaaag gcattgaacg aaatatggac 1320

gttcaagagg tgcaatatac atttgaccta cagcttgccc aagaggatgc aaagaagatg 1380

gctgttaagg aagaaaaata tgatccaggt tatgaggcag catatggtgg tgcttacgga 1440

gaaaatccat gcagcagtga accttgtggc ttctcttcaa atgggctaat tgagagcgtg 1500

gagttaagag gagaatcagc tttcagtggc attcctaatg ggcagtggat gacccagtca 1560

tttacagacc aaattccttc ctttagtaat cactgtggaa cacaagaaca ggaagaagaa 1620

agccatgctt aagaatggtg cttctcagct ctgcttaaat gctgcagttt taatgcagtt 1680

gtcaacaagt agaacctcag tttgctaact gaagtgtttt attagtattt tactctagtg 1740

gtgtaattgt aatgtagaac agttgtgtgg tagtgtgaac cgtatgaacc taagtagttt 1800

ggaagaaaaa gtagggtttt tgtatactag cttttgtatt tgaattaatt atcattccag 1860

ctttttatat actatatttc atttatgaag aaattgattt tcttttggga gtcactttta 1920

atctgtaatt ttaaaataca agtctgaata tttatagttg attcttaact gtgcataaac 1980

ctagatatac cattatccct tttataccta agaagggcat gctaataatt accactgtca 2040

aagaggcaaa ggtgttgatt tttgtatata agttaagcct cagtggagtc tcatttgtta 2100

gtttttagtg gtaactaagg gtaaactcag ggttccctga gctatatgca cactcagacc 2160

tctttgcttt accagtggtg tttgtgagtt gctcagtagt aaaaactggc ccttacctga 2220

cagagccctg gctttgacct gctcagccct gtgtgttaat cctctagtag ccaattaact 2280

actctggggt ggcaggttcc agagaatcga gtagaccttt tgccactcat ctgtgtttta 2340

cttgagacat gtaaatatga tagggaagga actgaatttc tccattcata tttataacca 2400

ttctagtttt atcttccttg gctttaagag tgtgccatgg aaagtgataa gaaatgaact 2460

tctaggctaa gcaaaaagat gctggagata tttgatactc tcatttaaac tggtgcttta 2520

tgtacatgag atgtactaaa ataagtaata tagaattttt cttgctaggt aaatccagta 2580

agccaataat tttaaagatt ctttatctgc atcattgctg tttgttacta taaattaaat 2640

gaacctcatg gaaaggttga ggtgtatacc tttgtgattt tctaatgagt tttccatggt 2700

gctacaaata atccagacta ccaggtctgg tagatattaa agctgggtac taagaaatgt 2760

tatttgcatc ctctcagtta ctcctgaata ttctgatttc atacgtaccc agggagcatg 2820

ctgttttgtc aatcaatata aaatatttat gaggtctccc ccacccccag gaggttatat 2880

gattgctctt ctctttataa taagagaaac aaattcttat tgtgaatctt aacatgcttt 2940

ttagctgtgg ctatgatgga ttttattttt tcctaggtca agctgtgtaa aagtcattta 3000

tgttatttaa atgatgtact gtactgctgt ttacatggac gttttgtgcg ggtgctttga 3060

agtgccttgc atcagggatt aggagcaatt aaattatttt ttcacgggac tgtgtaaagc 3120

atgtaactag gtattgcttt ggtatataac tattgtagct ttacaagaga ttgttttatt 3180

tgaatgggga aaataccctt taaattatga cggacatcca ctagagatgg gtttgaggat 3240

tttccaagcg tgtaataatg atgtttttcc taacatgaca gatgagtagt aaatgttgat 3300

atatcctata catgacagtg tgagactttt tcattaaata atattgaaag attttaaaat 3360

tcatttgaaa gtctgatggc ttttacaata aaagatatta agaattgtta 3410

<210> 7
<211> 1108
<212> DNA
<213> Homo sapiens

<400> 7
cctgggcccc catgcccgtg cagctcgcac atatgtgggg cagagcagcc accctgcccc 60

cagcagcagc cgtccatcgt cagacgtgat catttcctga ggcctcgagt gtgtcagggt 120

gtttgtgcct cataacaacc cacaggatgg tcaccccccgc tttgcagatg aagaaaccaa 180

~

agcagttttg ccgacggatg gggcaaaaga agcagcgacc agctagagca gggcagccac 240

acagctcgtc cgacgcagcc caggcacctg cagagcagcc acacagctcg tccgatgcag 300

cccaggcacc ttgccccagg gagcgctgct tgggaccgcc caccactccg ggcccatacc 360

gcagcatcta tttctcaagc ccaaagggcc accttacccg actggggttg gagttcttcg 420

accagccggc agtcccctg gcccgggcat ttctgggaca ggtcctagtc cggcgacttc 480

ctaatggcac agaactccga ggccgcatcg tggagaccga ggcatacctg ggccagagg 540

atgaaccggc ccactcaagg ggtggccggc agaccccccg caaccgaggc atgttcatga 600

agccggggac cctgtacgtg tacatcattt acggcatgta cttctgcatg aacatctcca 660

gccaggggga cggggcttgc gtcttgctgc gagcactgga gcccctggaa ggtctggaga 720

ccatgcgtca cgttcgcagc accctccgga aaggcaccgc cagccgtgtc ctcaaggacc 780

gcgagctctg cagtggcccc tccaagctgt gccaggccct ggccatcaac aagagctttg 840

accagaggga cctggcacag gatgaagctg tatggctgga gcgtggtccc ctggagccca 900

gtgagccggc tgtagtggca gcagcccggg tgggcgtcgg ccatgcaggg gagtgggccc 960

ggaaacccct ccgcttctat gtccggggca gcccctgggt cagtgtggtc gacagagtgg 1020

ctgagcagga cacacaggcc tgagcaaagg gcctgcccag acaagatttt ttaattgttt 1080

aaaaaccgaa taaatgtttt atttctag 1108

<210> 8
<211> 939
<212> DNA
<213> Homo sapiens

<400> 8
atgtgtagtc cgcaggagtc cggcatgacc gccttgagcg cgaggatgct gacccggagc 60

cggagcctgg gacccggggc tgggccgcgg gggtgtaggg aggagcccgg gcctctccgg 120

agaagagagg ctgcagcaga agcgaggaaa agccacagcc ccgtgaagcg tccgcggaaa 180

gcacagagac tgcgtgtggc ctatgagggc tcggacagtg agaaaggtga gggggctgag 240

cccctcaagg tgccagtctg ggagccccag gactggcagc aacagctggt caacatccgt 300

gccatgagga acaaaaagga tgcacctgtg gaccatctgg ggactgagca ctgctatgac 360

tccagtgccc ccccaaaggt acgcaggtac caggtgctgc tgtcactgat gctctccagc 420

caaaccaaag accaggtgac ggcgggcgcc atgcagcgac tgcgggcgcg gggcctgacg 480

gtggacagca tcctgcagac agatgatgcc acgctgggca agctcatcta ccccgtcggt 540

ttctggagga gcaaggtgaa atacatcaag cagaccagcg ccatcctgca gcagcactac 600

ggtggggaca tcccagcctc tgtggccgag ctggtggcgc tgccgggtgt tgggcccaag 660

atggcacacc tggctatggc tgtggcctgg ggcactgtgt caggcattgc agtggacacg 720

catgtgcaca gaatcgccaa caggctgagg tggaccaaga aggcaaccaa gtccccagag 780

gagacccgcg ccgccctgga ggagtggctg cctagggagc tgtggcacga gatcaatgga 840

ctcttggtgg gcttcggcca gcagacctgt ctgcctgtgc accctcgctg ccacgcctgc 900

ctcaaccaag ccctctgccc ggccgcccag ggtctctga 939

<210> 9
<211> 1854
<212> DNA
<213> Homo sapiens

<400> 9
ggagcctcta gaactatgag cccgaggcct tcccctctcc cagagcgcag aggctttgaa 60

ggctacctct gggaagccgc tcaccgtcgg aagctgcggg agctgaaact gcgccatcgt 120

cactgtcggc ggccatgaca ccgctcgtct cccgcctgag tcgtctgtgg gccatcatga 180

ggaagccacg agcagccgtg ggaagtggtc acaggaagca ggcagccagc caggaaggga 240

ggcagaagca tgctaagaac aacagtcagg ccaagccttc tgcctgtgat gggatgattg 300

ctgagtgtcc tggggcccca gcaggcctgg ccaggcagcc ggaagaggtg gtattgcagg 360

cctctgtctc ctcataccat ctattcagag acgtagctga agtcacagcc ttccgaggga 420

gcctgctaag ctggtacgac caagagaaac gggacctacc atggagaaga cgggcagaag 480

atgagatgga cctggacagg cgggcatatg ctgtgtgggt ctcagaggtc atgctgcagc 540

agacccaggt tgccactgtg atcaactact ataccggatg gatgcagaag tggcctacac 600

tgcaggacct ggccagtgct tccctggagg aggtgaatca actctgggct ggcctgggct 660

actattctcg tggccggcgg ctgcaggagg gagctcggaa ggtggtagag gagctagggg 720

gccacatgcc acgtacagca gagaccctgc agcagctcct gcctggcgtg ggcgctaca 780

cagctggggc cattgcctct atcgcctttg gccaggcaac cggtgtggtg gatggcaacg 840

tagcacgggt gctgtgccgt gtccgagcca ttggtgctga tcccagcagc acccttgttt 900

cccagcagct ctggggtcta gcccagcagc tggtggaccc agcccggcca ggagatttca 960

accaagcagc catggagcta ggggccacag tgtgtacccc acagcgccca ctgtgcagcc 1020

agtgccctgt ggagagcctg tgccgggcac gccagagagt ggagcaggaa cagctcttag 1080

cctcagggag cctgtcgggc agtcctgacg tggaggagtg tgctcccaac actggacagt 1140

gccacctgtg cctgcctccc tcggagccct gggaccagac cctgggagtg gtcaacttcc 1200

ccagaaaggc cagccgcaag ccccccaggg aggagagctc tgccacctgt gttctggaac 1260

agcctggggc ccttggggcc caaattctgc tggtgcagag gcccaactca ggtctgctgg 1320

caggactgtg ggagttcccg tccgtgacct gggagccctc agagcagctt cagcgcaagg 1380

ccctgctgca ggaactacag cgttgggctg ggcccctccc agccacgcac ctccggcacc 1440

ttggggaggt tgtccacacc ttctctcaca tcaagctgac atatcaagta tatgggctgg 1500

ccttggaagg gcagacccca gtgaccaccg taccaccagg tgctcgctgg ctgacgcagg 1560

aggaatttca caccgcagct gtttccaccg ccatgaaaaa ggttttccgt gtgtatcagg 1620

gccaacagcc agggacctgt atgggttcca aaaggtccca ggtgtcctct ccgtgcagtc 1680

ggaaaaagcc ccgcatgggc cagcaagtcc tggataattt ctttcggtct cacatctcca 1740

~

```
ctgatgcaca cagcctcaac agtgcagccc agtgacacct ctgaaagccc ccattccctg   1800

agaatcctgt tgttagtaaa gtgcttattt ttgtagttaa aaaaaaaaaa aaaa         1854


<210>  10
<211>  2557
<212>  DNA
<213>  Homo sapiens

<220>
<221>  misc_feature
<222>  (200)..(200)
<223>  any nucleotide


<400>  10
ttcgcttgaa cccgggaggc ggagcttgca gtgagccgag atcgcgccat cacactccag     60

ctcaggcgac agagtgagac tccgtctcaa agaaaaaaaa cttgcagcct gatagttaag    120

atacagcaac cccaaatccc tatgctaaaa ggtgagaatg gcccagataa aggtcatgtc    180

tcctagctcc ctgctttttn atgccatcct ccagaaggga agaaattaaa taatccatcc    240

tcctactcca ggcgactaga aggcaggctg cctcagggcc acacactggg acttggactc    300

aacctgatgg gcttctgggc ccagccccag acaaaccccc ggcaaacgtc ccattccgag    360

gaaagcatga gcagatggag tatggaagaa atgcccaaga cggcaggcag cagctgtggc    420

ggccggcggg acgacaatcc gaggagaggc ctctgatgtc ctgaggtctc agaggacgcc    480

taaaggcctt gaatgggaca agcttagcgg gcgggcgcag aagagaataa tactctggag    540

acacttcccg agggctctgg ggccggagct gtgttcgctc cggttcttgg tgaagacagg    600

gttcgtggga ggcggcccaa ggagggcgaa cgcctaagac tgcaaaggct cgggggagaa    660

cggctctcgg agaacgggct ggggaaggac gtggctctga agacggacag ccctgaggaa    720

ccgcggggcg cccagatgga actcgttagc gccccgagtg cagacaatcc cggagggggga   780

aaggcgagca gctggcagag agcccagtgc cggccaaccg cgcgagcgcc tcagaacggc    840

ccgcccaccc tgatttctca ttggcgcctc ctacctcctc ctcggattgg ctacctctag    900

gtgaaatgag cggtggttga gccctacttc cggtggtgct gtggtctgcc cctggagaac    960

ccagaagaac acagctgtgc gcgcccacag gctctggggg cgggagaaga taagtcgcaa   1020

ggaggggggcg ggacctacac ctcaggaaag ccggagaatt ggggcacgaa gcggggcttt  1080

gatgacccgc aaagggcgag gcatgcagga ggtggaggaa ttaagtgaaa cagggaaggt   1140

tgttaaacag caccgtgtgg gcgaggcctt aagggtcgtg gtccttgtct gggcggggtc   1200

tttgggcgtc gacgaggcct ggttctgggt aggcggggct actacggggc ggtgcctgct   1260

gtggaaatgc ctgcccgcgc gcttctgccc aggcgcatgg ggcatcgtac tctagcctcc   1320

actcctgccc tgtgggcctc catcccgtgc cctcgctctg agctgcgcct ggacctggtt   1380

ctgccttctg gacaatcttt ccggtggagg gagcaaagtc ctgcacactg gagtggtgta   1440

ctagcggatc aagtatggac actgactcag actgaggagc agctccactg cactgtgtac   1500
```

cgaggagaca agagccaggc tagcaggccc acaccagacg agctggaggc cgtgcgcaag 1560

tacttccagc tagatgttac cctggctcaa ctgtatcacc actggggttc cgtggactcc 1620

cacttccaag aggtggctca gaaattccaa ggtgtgcgac tgctgcgaca agaccccatc 1680

gaatgccttt tctcttttat ctgttcctcc aacaacaaca tcgcccgcat cactggcatg 1740

gtggagcggc tgtgccaggc ttttggacct cggctcatcc agcttgatga tgtcacctac 1800

catggcttcc ccagcctgca ggccctggct gggccagagg tggaggctca tctcaggaag 1860

ctgggcctgg gctatcgtgc ccgttacgtg agtgccagtg cccgagccat cctggaagaa 1920

cagggcgggc tagcctggct gcagcagcta cgagagtcct catatgagga ggcccacaag 1980

gccctctgca tcctgcctgg agtgggcacc aaggtggctg actgcatctg cctgatggcc 2040

ctagacaagc cccaggctgt gcccgtggat gtccatatgt ggcacattgc ccaacgtgac 2100

tacagctggc accctaccac gtcccaggcg aagggaccga gcccccagac caacaaggaa 2160

ctgggaaact tttccggag cctgtgggga ccttatgctg gctgggccca agcggtgctg 2220

ttcagtgccg acctgcgcca atcccgccat gctcaggagc caccagcaaa gcgcagaaag 2280

ggttccaaag ggccggaagg ctagatgggg caccctggac aaagaaattc cccaagcacc 2340

ttcccctcca ttccccactt ctctctcccc atccccaccc agtctcatgt tggggagggg 2400

cctccctgtg actacctcaa aggccaggca cccccaaatc aagcagtcag tttgcacaac 2460

aagatggggt gggggatatt gagggagaca gcgctaagga tggttttatc ttccctttat 2520

tacaagaagg aacaataaaa tagaaacatt tgtatgg 2557

<210> 11
<211> 643
<212> DNA
<213> Homo sapiens

<400> 11
gagcggcggt gcagaaccca gggaccatgg gcgcctccag gctctatacc ctggtgctgg 60

tcctgcagcc tcagcgagtt ctcctgggca tgaaaaagcg aggcttcggg gccggccggt 120

ggaatggctt tgggggcaaa gtgcaagaag gagagaccat cgaggatggg gctaggaggg 180

agctgcagga ggagagcggt ctgacagtgg acgccctgca caaggtgggc cagatcgtgt 240

ttgagttcgt gggcgagcct gagctcatgg acgtgcatgt cttctgcaca gacagcatcc 300

aggggacccc cgtggagagc gacgaaatgc gcccatgctg gttccagctg gatcagatcc 360

ccttcaagga catgtggccc gacgacagct actggtttcc actcctgctt cagaagaaga 420

aattccacgg gtacttcaag ttccagggtc aggacaccat cctggactac acactccgcg 480

aggtggacac ggtctagcgg gagcccaggg cagcccctgg gcaggagacg tggctgctga 540

acagctgcaa accatcttca cctgggggca ttgagtggcg cagagccggg tttcatctgg 600

aattaactgg atggaaggga aaataaagct atctagcggt gaa 643

<210> 12
<211> 1006

~

<212> DNA
<213> Homo sapiens

<400> 12
cgtctcctcg ctcgccttct ggctctgcca tgccctgctc tgaagagaca cccgccattt 60

cacccagtaa gcgggcccgg cctgcggagg tgggcggcat gcagctccgc tttgcccggc 120

tctccgagca cgccacggcc cccacccggg gctccgcgcg cgccgcgggc tacgacctgt 180

acagtgccta tgattacaca ataccaccta tggagaaagc tgttgtgaaa acggacattc 240

agatagcgct cccttctggg tgttatggaa gagtggctcc acggtcaggc ttggctgcaa 300

aacactttat tgatgtagga gctggtgtca tagatgaaga ttatagagga aatgttggtg 360

ttgtactgtt taattttggc aaagaaaagt ttgaagtcaa aaaaggtgat cgaattgcac 420

agctcatttg cgaacggatt ttttatccag aaatagaaga agttcaagcc ttggatgaca 480

ccgaaagggg ttcaggaggt tttggttcca ctggaaagaa ttaaaattta tgccaagaac 540

agaaaacaag aagtcatacc tttttcttaa aaaaaaaaaa agtttttgct tcaagtgttt 600

tggtgttttg cacttctgta aacttactag ctttaccttc taaaagtact gcattttttta 660

cttttttttta tgatcaagga aaagatcatt aaaaaaaaac acaaaagaag tttttctttg 720

tgtttggatc aaaaagaaac tttgtttttc cgcaattgaa ggttgtatgt aaatctgctt 780

tgtggtgacc tgatgtaaac agtgtcttct taaaatcaaa tgtaaatcaa ttacagatta 840

aaaaaaaaaa gcctgtattt aactcatatg atctcccttc agcaacttat tttgctttaa 900

ttgctttaaa tcttaagcaa tattttttat tcagtaaaca aattctttca caaggtacaa 960

aatcttgcat aagctgaact aaaataaaaa tgaaaaggag agatta 1006

<210> 13
<211> 1420
<212> DNA
<213> Homo sapiens

<400> 13
tgccatcggg ccggtgcaga tacggggttg ctcttttgct cataagaggg gcttcgctgg 60

cagtctgaac ggcaagcttg agtcaggacc cttaattaag atcctcaatt ggctggaggg 120

cagatctcgc gagtagggca acgcggtaaa aatattgctt cggtgggtga cgcggtacag 180

ctgcccaagg gcgttcgtaa cgggaatgcc gaagcgtggg aaaaagggag cggtggcgga 240

agacggggat gagctcagga cagagccaga ggccaagaag agtaagacgg ccgcaaagaa 300

aaatgacaaa gaggcagcag gagagggccc agccctgtat gaggaccccc cagatcacaa 360

aacctcaccc agtggcaaac ctgccacact caagatctgc tcttggaatg tggatgggct 420

tcgagcctgg attaagaaga aaggattaga ttgggtaaag gaagaagccc cagatatact 480

gtgccttcaa gagaccaaat gttcagagaa caaactacca gctgaacttc aggagctgcc 540

tggactctct catcaatact ggtcagctcc ttcggacaag gaagggtaca gtggcgtggg 600

cctgctttcc cgccagtgcc cactcaaagt ttcttacggc ataggcgatg aggagcatga 660

tcaggaaggc cgggtgattg tggctgaatt tgactcgttt gtgctggtaa cagcatatgt 720

~

acctaatgca ggccgaggtc tggtacgact ggagtaccgg cagcgctggg atgaagcctt 780

tcgcaagttc ctgaagggcc tggcttcccg aaagcccctt gtgctgtgtg gagacctcaa 840

tgtggcacat gaagaaattg accttcgcaa ccccaagggg aacaaaaaga atgctggctt 900

cacgccacaa gagcgccaag gcttcgggga attactgcag gctgtgccac tggctgacag 960

ctttaggcac ctctacccca acacaccccta tgcctacacc ttttggactt atatgatgaa 1020

tgctcgatcc aagaatgttg gttggcgcct tgattacttt ttgttgtccc actctctgtt 1080

acctgcattg tgtgacagca agatccgttc caaggccctc ggcagtgatc actgtcctat 1140

caccctatac ctagcactgt gacaccaccc ctaaatcact ttgagcctgg gaaataagcc 1200

ccctcaacta ccattccttc tttaaacact cttcagagaa atctgcattc tatttctcat 1260

gtataaaact aggaatcctc caaccaggct cctgtgatag agttctttta agcccaagat 1320

tttttatttg agggtttttt gtttttttaaa aaaaaattga acaaagacta ctaatgactt 1380

tgtttgaatt atccacatga aaataaagag ccatagtttc 1420

<210> 14
<211> 1259
<212> DNA
<213> Homo sapiens

<400> 14
ccggagctgg gttgctcctg ctcccgtctc caagtcctgg tacctccttc aagctgggag 60

agggctctag tccctggttc tgaacactct ggggttctcg ggtgcaggcc gccatgagca 120

aacggaaggc gccgcaggag actctcaacg ggggaatcac cgacatgctc acagaactcg 180

caaactttga gaagaacgtg agccaagcta tccacaagta caatgcttac agaaaagcag 240

catctgttat agcaaaatac ccacacaaaa taaagagtgg agctgaagct aagaaattgc 300

ctggagtagg aacaaaaatt gctgaaaaga ttgatgagtt tttagcaact ggaaaattac 360

gtaaactgga aaagattcgg caggatgata cgagttcatc catcaatttc ctgactcgag 420

ttagtggcat tggtccatct gctgcaagga agtttgtaga tgaaggaatt aaaacactag 480

aagatctcag aaaaaatgaa gataaattga accatcatca gcgaattggg ctgaaatatt 540

ttggggactt tgaaaaaaga attcctcgtg aagagatgtt acaaatgcaa gatattgtac 600

taaatgaagt taaaaaagtg gattctgaat acattgctac agtctgtggc agtttcagaa 660

gaggtgcaga gtccagtggt gacatggatg ttctcctgac ccatcccagc ttcacttcag 720

aatcaaccaa acagccaaaa ctgttacatc aggttgtgga gcagttacaa aaggttcatt 780

ttatcacaga taccctgtca aagggtgaga caaagttcat gggtgtttgc cagcttccca 840

gtaaaaatga tgaaaaagaa tatccacaca gaagaattga tatcaggttg atacccaaag 900

atcagtatta ctgtggtgtt ctctatttca ctgggagtga tattttcaat aagaatatga 960

gggctcatgc cctagaaaag ggtttcacaa tcaatgagta caccatccgt cccttgggag 1020

tcactggagt tgcaggagaa cccctgccag tggatagtga aaaagacatc tttgattaca 1080

```
tccagtggaa ataccgggaa cccaaggacc ggagcgaatg aggcctgtat cctccctggc   1140
agacacaacc caataggagt cttaatttat ttcttaacct ttgctatgta agggtctttg   1200
gtgtttttaa atgattgttt cttcttcatg cttttgcttg caatgtagtc aataaaacc    1259
```

## Description

FIELD AND BACKGROUND OF THE INVENTION

The present invention relates to the field of diagnosis and prognosis. More particularly, the present invention relates to methods of and kits for (i) determining a risk of a subject to develop cancer; (ii) evaluating an effectiveness and preferred dosage of cancer therapy administered to a cancer patient; and (iii) determining a presence of correlation or non-correlation between an activity of at least one DNA repair enzyme and at least one cancer.

The DNA in each cell of a body is constantly subjected to damage caused by both internal (e.g., reactive oxygen species) and external DNA damaging agents (e.g., sunlight, X- and γ-rays, smoke) (Friedberg, *et al.,* 1995). Most lesions are eliminated from DNA by one of several pathways of DNA repair (Friedberg, *et al.,* 1995, Hanawalt, 1994, Modrich, 1994, Sancar, 1994). When unrepaired DNA lesions are replicated, they cause mutations because of their miscoding nature (Echols and Goodman, 1991, Livneh, *et al.,* 1993, Strauss, 1985). The occurrence of such mutations in critical genes, e.g., oncogenes and tumor suppressor genes, may lead to the development of cancer (Bishop, 1995, Vogelstein and Kinzler, 1993, Weinberg, 1989). Indeed, DNA repair has emerged in recent years as a critical factor in cancer pathogenesis, as a growing number of cancer predisposition syndromes have been shown to be caused by mutations in genes involved in DNA repair and the regulation of genome stability. These include Xeroderma Pigmentosum (Weeda, *et al.,* 1993), Hereditary nonpolyposis colon cancer (Fishel, *et al.,* 1993, Leach, *et al.,* 1993, Modrich, 1994, Parsons, *et al.,* 1993), Ataxia Telangiectasia (Savitsky, *et al.,* 1995), Li-Fraumeni syndrome (Srivastava, *et al.,* 1990), and the BRCA1 (Gowen, *et al.,* 1998, Scully, *et al.,* 1997) and BRCA2 genes (Connor, *et al.,* 1997, Patel, *et al.,* 1998, Sharan, *et al.,* 1997). In these cases, which represent a minority of the cancer cases, gene mutations have caused malfunction, leading to a strong reduction in DNA repair.

A possible extension of the role of DNA repair in hereditary cancer, would be a role for DNA repair in sporadic cancer. Several studies suggested that inter-individual variability in DNA repair correlates with variation in cancer susceptibility, with low repair correlated to higher cancer risk (Athas, *et al.,* 1991, Helzlsouer, *et al.,* 1996, Jyothish, *et al.,* 1998, Parshad, *et al.,* 1996, Patel, *et al.,* 1997, Sagher, *et al.,* 1988, Wei, *et al.,* 1996, Wei, *et al.,* 1993, Wei, *et al.,* 1994).

7, 8-dihydro-8-oxoguanine (also termed 8-oxoguanine or 8-hydroxyguanine; dubbed 8-OxoG) is formed in DNA by two major pathways: (a) Modification of guanine in DNA by reactive oxygen species formed by intracellular metabolism, oxidative stress, cigarette smoke, or by radiation (Asami, *et al.,* 1997, Gajewski, *et al.,* 1990, Hutchinson, 1985, Leanderson and Tagesson, 1992). (b) Incorporation into DNA by DNA polymerases of 8-oxo-dGTP, which is formed by oxidation of intracellular dGTP (Maki and Sekiguchi, 1992). Once in DNA, 8-oxoG is replicated by DNA polymerases with the misinsertion of dAMP, causing characteristic GC to TA transversions (Shibutani, *et al.,* 1991, Wood, *et al.,* 1990). When the modified dGTP is used as a substrate by DNA polymerases, it is often misinserted opposite an A in the template, causing AT to CG transversions (Pavlov, *et al.,* 1994).

The major route for removing 8-oxoG from DNA is base excision repair, initiated by 8-oxoguanine DNA N-glycosylase, product of the *OGG1* gene (in humans termed also *hOGG1;* (Aburatani, *et al.,* 1997, Arai, *et al.,* 1997, Bjoras, *et al.,* 1997, Radicella, *et al.,* 1997, Roldan-Arjona, *et al.,* 1997, Rosenquist, *et al.,* 1997). The *OGG1* gene was recently knocked-out in mice, such that the effects on carcinogenesis can now be examined in this organism (Klungland, *et al.,* 1999, Minowa, *et al.,* 2000). Expression of the *E. coli* enzyme in Chinese hamster cells reduced 4-fold the mutagenicity of γ radiation (Laval, 1994), indicating that the repair of 8-oxoG is important in negating the mutagenic activity of γ radiation. The following observations associate OGG1 with cancer: (i) *OGG1* was mapped to chromosome 3p25, a site frequently lost in human lung and kidney cancers (Arai, *et al.,* 1997, Audebert, *et al.,* 2000, Ishida, *et al.,* 1999, Lu, *et al.,* 1997, Wikman, *et al.,* 2000). (ii) *OGG1* was found to be mutated in 2 out of 25 lung tumors (Chevillard, *et al.,* 1998), and in 4 out of 99 renal tumors (Audebert, *et al.,* 2000). (iii) *OGG1* was found to be mutated in a leukemic cell line (Hyun, *et al.,* 2000) and in a gastric cell line (Shinmura, *et al.,* 1998). (iv) Analysis of p53 mutations in human lung, breast, and kidney tumors revealed a substantial occurrence of GC to TA mutations, a mutation type produced by unrepaired 8-oxoG (Hollstein *et al.,* 1996; Hemandez-Boussard, *et al.,* 1999).

Since preventive measures which reduce the risk of developing cancer, such as, but not limited to, the use of anti-oxidants, diet, avoiding cigarette smoking, refraining from occupational exposure to cancer causing agents, are known and further since periodic testing and therefore early detection of cancer offers improved cure rates, there is a great need for, and it would be highly advantageous to have methods and kits for determining a risk of a subject to develop cancer.

Since the effectiveness of cancer therapy depends on the sensitivity of cells to genotoxic (mutageic) agents, there is a great need for, and it would be highly advantageous to have methods and kits for evaluating an effectiveness and preferred dosage of cancer therapy administered to a cancer patient.

There is also a great need for, and it would be highly advantageous to have methods and kits for determining a presence of correlation or non-correlation between an activity of at least one DNA repair enzyme and at least one cancer, so as to allow to determine a risk of a subject to develop cancer and to evaluate an effectiveness and preferred dosage of cancer therapy administered to a cancer patient.

## SUMMARY OF THE INVENTION

According to one aspect of the present invention there is provided method of determining a risk (e.g., odds ratio, relative risk) of a subject to develop cancer, the method comprising determining a level of a parameter indicative of a level of activity of a DNA repair/damage preventing enzyme in a tissue of the subject, and, according to the level, determining the risk of the subject to develop the cancer.

According to another aspect of the present invention there is provided a method of determining a risk of a subject to develop cancer, the method comprising determining (a) a presence or absence of exposure to environmental conditions, such as smoking and occupational exposure to smoke or ionizing radiation, associated with increased risk of developing cancer; and (b) a level of a parameter indicative of a level of activity of a DNA repair/damage preventing enzyme in a tissue of the subject; and according to the presence or absence and the level, determining the risk of the subject to develop the cancer.

According to still another aspect of the present invention there is provided a method of determining a presence of correlation or non-correlation between an activity of at least one DNA repair/damage preventing enzyme and at least one cancer, the method comprising determining a level of a parameter indicative of a level of activity of at least one DNA repair/damage preventing enzyme in tissue derived from a plurality of cancer patients and a plurality of apparently normal individuals, and, according to the level determining the correlation or non-correlation between the activity of the at least one DNA repair/damage preventing enzyme and the at least one cancer.

According to further features in preferred embodiments of the invention described below, the parameter is selected from the group consisting of a protein level of said DNA repair/damage preventing enzyme, a level of a RNA encoding said DNA repair/damage preventing enzyme and a level of catalytic activity of said DNA repair/damage preventing enzyme.

According to still further features in the described preferred embodiments the cancer is selected from the group consisting of lung cancer, blood cancers, colorectal cancer, breast cancer, prostate cancer, ovary cancer and head and neck cancer.

According to still further features in the described preferred embodiments the tissue is selected from the group consisting of blood cells, scraped cells and biopsies.

According to still further features in the described preferred embodiments the DNA repair/damage preventing enzyme is selected from the group consisting of a DNA N-glycosylase, deoxyribose phosphate lyase and AP endonuclease.

According to still further features in the described preferred embodiments the DNA N-glycosylase is selected from the group consisting of Uracil DNA glycosylase, hSMUG1, hMBD4, Mismatch-specific thymine/uracil glycosylase, Methylpurine DNA glycosylase, hNTH1, Adenine-specific mismatch DNA glycosylase and 8-oxoguanine DNA glycosylase.

According to still further features in the described preferred embodiments the risk is expressed as a fold risk increase as is compared to a normal, apparently healthy, population, or a reference control group.

According to still further features in the described preferred embodiments the risk is expressed in enzyme specific activity units.

According to still further features in the described preferred embodiments the risk is expressed as a magnitude of a scale.

According to still further features in the described preferred embodiments determining the level of catalytic activity of the DNA repair/damage preventing enzyme is effected using a DNA substrate having at least one lesion therein.

According to still further features in the described preferred embodiments the at least one lesion is at a predetermined site in the DNA substrate.

According to still further features in the described preferred embodiments the lesion is selected from the group consisting of uracil, 5-fluorouracil, 5-hydroxyuracil, isodialuric acid, alloxan, uracil or thymine in U/TpG:5meCpG, uracil (U:G), 3,$N^4$-ethenocytosine, (eC:G), T (T:G), 3-methyladenine, 7-methyladenine, 3-methylguanine, 7-methylguanine, hypoxanthine, 1, N6-ethenoadenine, 1,N2-ethenoguanine, thymine glycol, cytosine glycol, dihydrouracil, formamidopyrimidine urea, adenine from A:G; A:8-oxoG; C:A, 2-hydroxyadenine, 2,5-amino-5-formamidopyrimidine, 7,8-dihydro-8-oxoguanine (also termed 8-oxoguanine) and abasic site.

According to still further features in the described preferred embodiments the substrate includes at least two different lesions of at least two types.

According to still further features in the described preferred embodiments the substrate includes a single lesion.

According to still further features in the described preferred embodiments the substrate includes at least two different lesions of a single type.

According to still further features in the described preferred embodiments the subject is known to be, or is about to be, exposed to environmental conditions associated with increased risk of developing cancer.

According to yet another aspect of the present invention there is provided a method of predicting the efficacy of a mutagenic anti-cancer treatment, such as chemotherapy and/or radiotherapy, in a subject, the method comprising determining a level of a factor indicative of a level of activity of a DNA repair/damage preventing enzyme in a tissue of the subject, and, according to the level, predicting the efficacy of the mutagenic anti-cancer treatment in the subj ect.

According to still another aspect of the present invention there is provided a method of selecting dosage of a mutagenic anti-cancer treatment, such as chemotherapy and/or radiotherapy, for treating a subject, the method comprising determining a level of a factor indicative of a level of activity of a DNA repair/damage preventing enzyme in a tissue of the subject, and, according to the level, selecting dosage of the mutagenic anti-cancer treatment for treating the subject.

According to an additional aspect of the present invention there is provided a kit for determining a level of activity of a DNA repair/damage preventing enzyme in a tissue of a subject, the kit comprising, a package including, contained in sealable containers, a DNA substrate having at least one lesion therein and a reaction buffer.

According to further features in preferred embodiments of the invention described below, the kit, further comprising test tubes for separating lymphocytes.

According to still further features in the described preferred embodiments the test tubes are prepackaged with an anti-coagulant.

According to still further features in the described preferred embodiments the kit further comprising a liquid having a specific gravity selected effective in separating lymphocytes from red blood cells via centrifugation.

According to still further features in the described preferred embodiments the kit further comprising a solution having osmolarity selected effective in lysing red blood cells.

According to still further features in the described preferred embodiments the kit further comprising a protein extraction buffer.

According to still further features in the described preferred embodiments the kit further comprising reagents for conducting protein determinations.

According to still further features in the described preferred embodiments the kit further comprising a purified DNA repair/damage preventing enzyme, which serves as a control for such activity.

The present invention successfully addresses the shortcomings of the presently known configurations by providing, methods, kits and reagents useful in determining a risk of a subject to develop cancer and for evaluating an effectiveness and individual dosage of cancer therapy administered to a cancer patient.

## BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

FIG. 1a shows an outline of an OGGA nicking assay according to the present invention. In the assay a 32 base pair synthetic DNA is cleaved at an 8-oxoG lesion (indicated by a circle), generating, after denaturation, a radiolabeled 17-mer. The asterisk represents a radiolabeled phosphate group.

FIGs. 1b-c represent a time course of the OGGA nicking assay of the present invention, performed under standard conditions, with a protein extract prepared from peripheral blood lymphocytes from a healthy donor. Figure 1b shows a phosphorimage of the reaction products fractionated by urea-PAGE, and Figure 1c shows the quantification of the images. GO, the DNA substrate with a site-specific 8-oxoG; G, a control substrate with a G instead of 8-oxoG.

FIGs. 2a-b show protein titration in the OGGA nicking assay. The assay was performed under standard conditions, with the indicated amounts of protein extract prepared from peripheral blood lymphocytes from a healthy donor. Figure 2a shows a phosphorimage of the reaction products fractionated by urea-PAGE, and Figure 2b shows the quantification of the images. GO, the DNA substrate with a site-specific 8-oxoG; G, a control substrate with a G instead of 8-oxoG.

FIGs. 3a-b show analysis of the specificity of the OGGA nicking assay of the present invention. The assay was performed under standard conditions, except that the reaction mixture contained 2 pmol of radiolabeled substrate containing 8-oxoG, and the indicated amounts of unlabeled competing DNA. Figure 3a shows a phosphorimage of the reaction products fractionated by urea-PAGE, and Figure 3b shows the quantification of the images. The protein extract was from a healthy donor Hx, G and GO represent unlabeled competing DNAs, which were similar to the radiolabeled substrate, and contained either hypoxanthine, guanine or 8-oxoG in the same location.

FIG. 4 shows the OGGA distribution in healthy individuals (i.e., control subjects). The OGGA nicking assay of the present invention was performed with blood samples from 123 healthy donors. OGGA ≤ 5.5 is defined as Low (less than 4 % of the control group) OGGA > 5.5 is defined as Normal.

FIG. 5 shows a comparison of OGGA in males and females. The OGGA distribution of the 123 individuals shown in Figure 4, was plotted separately for males (N=53) and females (N=70).

FIG. 6 shows a comparison of OGGA in smokers and non-smokers. The OGGA distribution of the 123 individuals shown in Figure 4, was plotted separately for smokers (N=35) and non-smokers (N=88).

FIG. 7 shows a comparison of OGGA in two age groups. The OGGA distribution of the 123 individuals shown in Figure 4, was plotted separately for ages <50 (N=34) and ?50 (N=89)

FIGs. 8a-d show OGGA in apparently healthy individuals and in patients with breast cancer or chronic lymphocytic leukemia (CLL). Figure 8a - OGGA distribution of a control group of 70 healthy female individuals (see Figure 5), and of 31 breast cancer patients (Figure 8b). Figure 8c - OGGA distribution in the control group of 123 subjects, and 19 CLL patients (Figure 8d).

FIGs. 9a-b show OGGA in apparently healthy individuals and in patients with lung cancer (NSCLC). Figure 9a - OGGA distribution of the control group of 123 healthy individuals (see Figure 4), and of 102 lung cancer (NSCLC) patients (Figure 9b).

FIGs. 10a-b show OGGA in apparently healthy individuals and in lymphoma patients. Figure 10a - OGGA distribution of the control group of 123 healthy individuals (see Figure 4), and of 18 lymphoma patients (Figure 10b).

FIGs. 11a-b show OGGA in apparently healthy individuals and in patients with colorectal cancer. Figure 11a - OGGA distribution of the control group of 123 healthy individuals (see Figure 4), and of 16 colorectal cancer patients (Figure 11b).

FIGs. 12a-b are schematic representations of monomolecular (Figure 12a) and plurimolecular (Figure 12b) universal substrates in accordance with the teachings of the present invention.

## DESCRIPTION OF THE PREFERRED EMBODIMENTS

The present invention is of methods and kits which can be used for (i) determining a risk (e.g., odds ratio, reklative risk) of a subject to develop cancer; (ii) evaluating an effectiveness and dosage of cancer therapy administered to a cancer patient; and (iii) determining a presence of correlation or non-correlation between an activity of at least one DNA repair/damage preventing enzyme and at least one cancer.

The principles and operation of a method and kit according to the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

While conceiving the present invention it was hypothesized that inter-individual variations in DNA repair/damage preventing activity modulate susceptibility of developing cancer.

While reducing the present invention to practice an experimental system which is easily adaptable to clinical use was developed, such that a defined DNA repair activity can now be used in determining cancer risk, and be utilized as a tool in cancer prevention, early detection and prognosis. Since the repertoire of DNA lesions is very large, at present experimental focus was given to an abundant and mutagenic DNA lesion, 8-oxoguanine (also termed 7, 8-dihydro 8-oxoguanine or 8-hydroxyguanine; dubbed 8-oxoG). However, other mutagenic DNA lesions, such as, but not limited to, those listed in Table 3 below, can be similarly used to implement the methods of the invention, following suitable adaptation.

Thus, while reducing the present invention to practice, whether inter-individual variations in the activity of OGG, correlate with increased susceptibility to several types of cancers was studied. A lower repair activity might lead to an increased load of DNA lesions, and therefore to increased mutation rate, and earlier occurrence of cancer. Similarly, a lower repair activity renders cancer cells more susceptible to cancer therapy, which is genotoxic by nature. It should be noted that different types of DNA repair may be critical in different types of cancer. The present invention is exemplified, in a non-limiting fashion, with respect to the removal from DNA of a specific type of mutagenic lesion, 8-oxoG, by the activities of one or more DNA N-glycosylase repair enzymes, present in protein extracts from peripheral blood lymphocytes.

The present invention is herein exemplified with respect to the use of the level of the DNA repair enzymatic activity of DNA N-glycosylase(s) directed toward 7, 8 dihydroxy 8-oxoguanine (8-oxoguanine DNA N-glycosylase activity;

OGG), as a risk factor for lung cancer, lymphomas, and colorectal cancer. The enzymatic activity is measured in a protein extract extracted from peripheral blood lymphocytes and is referred to herein interchangeably as the OGGA nicking assay, OGGA assay or OGGA test.

Using the OGGA test, a case-control study was conducted on 309 individuals: 123 healthy individuals, and a total of 186 cancer patients as follows: 102 lung cancer (NSCLC) patients, 31 breast cancer patients, 18 lymphoma patients, 19 CLL patients, and 16 colorectal cancer patients. The following results were found.

The mean OGGA in healthy individuals of ages <50 (7.6±0.9; N=34) was slightly higher than in healthy individuals of ages ?50 (7.0±1.0; N=89). The difference is statistically significant (P=0.02).

The mean OGGA in healthy men (7.3±1.0; N=53) was similar to healthy women (7.1±1.0; N=70), the difference was not statistically significant (P=0.36).

The mean OGGA in smokers (7.3±1.00; N=35) was similar to that of non-smokers (7.1±1.0; N=88; P=0.46), indicating that the smoking status had a negligible effect on OGGA.

The mean OGGA in lung cancer patients (6.0±1.5; N=102) was significantly lower than in healthy individuals (7.2±1.0; N=123), with P=0.0001.

A strong association was found between low OGGA and lung cancer with odds ratio varying from 3.9 (95 % CI 1.7-8.6, P=0.0009), to 9.0 (95 % CI 3.2-25.0, P=0.0001), depending on the definition of the cutoff level (≤7.3 and ≤ 5.6, respectively). This indicates that a low OGGA value is a risk factor in lung cancer.

The mean OGGA in lymphoma patients (6.2±1.8; N=18) was significantly lower than in healthy individuals (7.2±1.0; N=123), with P=0.0001.

Low repair is defined as OGGA value ? 5.5 units/μg protein, representing <4% of the healthy individuals. Normal repair is defined as 5.5 units/μg protein. After adjustment for age, lymphoma patients were 15 times more likely than the healthy controls to have a low OGGA value (Odds Ratio 15.2; 95% confidence interval, 3.7-62.5). This provides evidence that a low OGGA value is a risk factor in lymphoma.

The data shows that OGGA was low in 2 out of 16 (12 %) colorectal cancer patients (compared to 5/123 i.e., 4.1 % among healthy individuals), indicating that low OGGA is a risk factor in colorectal cancer.

OGGA distribution was normal in breast cancer patients, indicating that OGGA is not a risk factor in this type of cancer.

It will be appreciated that the OGGA and similar tests for other DNA repair activities can be used for screening individuals for purposes of prevention, early diagnosis and prognosis of cancers. These uses will be described in more detail below.

The following provides examples:

(i) Screening for smokers who have low OGGA in order to prevent lung cancer.

Although 85 % of lung cancer patients are smokers, the great majority of smokers deal well with carcinogenic effects of smoking, and does not develop lung cancer. Even among heavy smokers, approximately 90 % do not develop the disease (Mattson et al, 1987; Minna et al, 2002). The results presented herein clarifies the fact that the combination of smoking and low OGGA causes a dramatic increase in susceptibility to lung cancer. For example, the estimated risk of 30-years old smokers, with an OGGA value of 3.0, is 221-fold higher than the reference (30-years old non-smokers with an OGGA value of 7.0). For comparison, the estimated risk of 30-years old non-smokers, with an OGGA value of 3.0 is only 12-fold higher than the reference. The simplest explanation for this finding is that smokers with Low OGGA in peripheral blood lymphocytes have a lower OGGA also in their lungs. Having a low repair to start with, smoking causes further overloading of DNA damage, therefore leading to a high cancer risk. This is a classical example in which the risk of developing cancer is a combination of genetic factors (level of DNA repair) and external factors (cigarette smoking). Such individuals may be persuaded to quit smoking. Such a screen will be effective as a preventive means against lung cancer, and will lead eventually to a decrease in the incidence of lung cancer.

(ii) Avoiding occupational hazard.

A considerable amount of people work in places which deal with radiation or with smoke. These include radiology departments in hospitals, nuclear industry, nuclear reactors, army personal dealing with nuclear weapons, etc. These people can be tested for OGGA, as a mandatory test, for their own safety. Individuals with Low OGGA might have an increased probability of developing cancer in such places, since ionizing radiation and smoke each produce 8-oxoG. Such individuals will be advised to seek an alternative working environment.

(iii) Using the OGGA value as a prognostic marker for cancer therapy.

Cancer therapy relies heavily on chemicals and radiation. These agents act, in most cases, by inflicting massive DNA damage, which leads to selective killing of the rapidly dividing cancer cells. The problem with such therapeutic agents is that they damage, or kill, also non-cancer cells. Knowing the level of OGGA in a cancer patient, may be used as a marker to estimate the prognosis of a particular therapeutic treatment.

(iv) Screening for susceptibility to lymphoma or colorectal cancers.

OGGA can be used to screen individuals for susceptibility to lymphomas or colorectal cancer.

(v) Early detection of cancer.

Individuals with low OGGA (e.g., smokers with low OGGA who would not quit smoking) can be advised to undergo periodical follow-ups, in order to enable early detection of lung cancer.

Thus, according to one aspect of the present invention there is provided a method of determining a risk of a subject to develop cancer. The method according to this aspect of the present invention is effected by determining a level of a factor indicative of a level of activity of a DNA repair/damage preventing enzyme in a tissue of the subject, and, according to the level, determining the risk of the subject to develop the cancer.

As used herein throughout the term "indicative of' includes correlating to.

According to another aspect of the present invention there is provided a method of determining a risk of a subject to develop cancer. The method according to this aspect of the present invention is effected by determining a presence or absence of exposure to environmental conditions, such as smoking and occupational exposure to smoke or ionizing radiation, associated with increased risk of developing cancer; and determining a level of a factor indicative of a level of activity of a DNA repair/damage preventing enzyme in a tissue of the subject; and according to the presence or absence and the level, determining the risk of the subject to develop the cancer.

Anyone of several approaches may be exploited according to the present invention in determining a level of a factor indicative of a level of activity of a DNA repair/damage preventing enzyme in a tissue of the subject.

According to one embodiment a protein level of the DNA repair/damage preventing enzyme is determined, which is indicative of the level of activity of the DNA repair/damage preventing enzyme. Several alternative quantitative assays are available for determining protein levels. Each of which is based on the specific interactions between proteins and antibodies specific thereto. Table 1 below lists known antibodies recognizing different DNA repair/damage preventing enzyme.

***Table 1***

| Enzyme | Antibody | Source/Reference |
|---|---|---|
| 1. Uracil DNA glycosylase (UNG) | PU101 | Slupphaug *et al.,* 1995(1) |
| 2. 8-oxoguanine glycosylase (OGG 1) | ABla33<br>Anti-human OGG1 | Monden *et al.,* 1999(2)<br>Alexis Biochemicals |
| 3. Adenine mismatch glycosylase (MYH) | Anti-hMYH α 344<br><br>Anti-hMYH α 516 | Parker *et al.,* 2000(3)<br>Parker *et al.,* 2000(3) |
| 4. 8-oxodGTPase (MTH1) | Anti-M78 | Kang *et al.,* 1995(4) |
| 5. dUTPase (DUT) | DUT415 | Ladner *et al.,* 1997(5) |
| 6. AP endonuclease I (HAP1, APE1, REF1, APEX) | Ref-1 (H-300), Ref-1 (C-20), Ref-1 (N-16), Ref-1 (E-17) | Santa Cruz Biotechnology |
| 7. Deoxyribose phosphate lyase (of | mAb-10S | Srivastava *et al.,* 1995(6) |

(continued)

| Enzyme | Antibody | Source/Reference |
|---|---|---|
| DNA polymerase β | 18S mAb | Srivastava *et al.,* 1999[7] |

[1] Slupphaug, G., Eftedal, I., Kavli, B., Bharati, S., Helle, N. M., Haug, T., Levine, D. W., Krokan, H. E. (1995) Properties of a recombinant human uracil-DNA glycosylase from the UNG gene and evidence that UNG encodes the major uracil-DNA glycosylase. Biochemistry 34, 128-138.
[2] Monden, Y., Arai, T., Asano, M., Ohtsuka, E., Aburatani, H., Nishimura, S. (1999) Human MMH (OGG1) type 1a protein is a major enzyme for repair of 8-hydroxyguanine lesions in human cells. Biochem. Biophys. Res. Comm. 258, 605-610.
[3] Parker, A., Gu, Y. and Lu, A. -L. (2000) Purification and characterization of a mammalian homolog of Escherichia coli MutY mismatch repair protein from calf liver mitochondria. Nucleic Acids Res. 28, 3206-3215.
[4] Kang, D., Nishida, J., Iyama, A., Nakabeppu, Y., Furuichi, M., Fujiwara, T., Sekiguchi, M. and Takeshige, K. (1995) Intracellular localization of 8-oxo-dGTPase in human cells, with special reference to the role of the enzyme in mitochondria. J. Biol. Chem. 270, 14659-14665.
[5]Ladner, R. D. and Caradonna, S. J. (1997) The human dUTPase gene encodes both nuclear and mitochondrial isoforms. J. Biol. Chem. 272 19072-19080.
[6] Srivastava, D. K., Rawson, T. Y., Showalter, S. D. and Wilson, S. H. (1995) Phorbol ester abrogates up-regulation of DNA polymerase β by DNA-alkylating agents in Chinese hamster ovary cells. J. Biol. Chem. 270, 16402-16408.
[7] Srivastava, D. K., Husain, I., Arteaga, C. L. and Wilson, S. H. (1999) DNA polymerase β expression differences in selected human tumor cell lines. Carcinogenesis 20, 1049-1054.

Antibodies recognizing any specific protein can be readily elicited using methods well known in the art in which cells of an immune system are exposed *in vivo* or *in vitro* to at least one epitope of the protein of interest, preferably a plurality of epitopes thereof. Such antibodies can be polyclonal or monoclonal. Commercial antibody developing services are available throughout the world. Examples include: Antibody Solutions, Palo Alto, California, Washington Biotechnology Inc., Baltimore, Maryland; TNB Laboratories Inc., St. John's, Newfoundland, Canada; and Genemed Synthesis Inc., South San Francisco, California.

Such antibodies can be used in a variety of well known antibody based detection assays, including, but not limited to, Western blot, ELISA, a protein chip assay and an antibody chip assay.

In Western blot, total protein preparation is electrophoresed typically under denaturing and optionally under reducing conditions through a gel, typically a polyacrylamide gel. Then, the proteins are blotted onto a membrane, which is thereafter blocked by a non-specific protein, such as milk proteins. An antibody specific to the protein of interest is then interacted with the blot. The antibody will quantitatively bind to the protein of interest. The binding between the antibody and the protein of interest can be monitored by either directly labeling the antibody, or, preferably using a labeled secondary antibody capable of recognizing the first.

In ELISA the antibody capable of binding the protein of interest is linked to an enzyme capable of catalyzing a colorimetric reaction, which serves for quantitative detection.

In a protein chip assay, the protein of interest, typically a plurality of different proteins of interest, are linked to a solid support in addressable positions, so as to form a matrix of proteins. An antibody or several antibodies specific to certain proteins, each being labeled by a distinguishable label, are interacted with the support in the presence of proteins derived from a biological sample. A protein recognized by an antibody and which is present in the biological sample will compete with its solid support bound counterpart, such that the level of binding of the antibody to the respective addressable location on the support, is determinable by such competition for binding.

In an antibody chip assay, antibodies are linked to a solid support in addressable positions, so as to form a matrix of antibodies each capable of binding a different protein. Proteins derived from a biological sample are labeled and the labeled proteins are interacted with the solid support. The level of binding to the solid support is determined, being indicative of the level of the protein in the sample.

These assays are well known and are well described in the art literature. Further details are available in, for example, "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Immobilized Cells and Enzymes" IRL Press, (1986); "Methods in Enzymology" Vol. 1-317, Academic Press; Marshak et al., "Strategies for Protein Purification and Characterization - A Laboratory Course Manual" CSHL Press (1996).

According to another embodiment of the present invention the level of a RNA, such as mRNA, encoding the DNA repair/damage preventing enzyme is determined, which is also indicative of the level of activity of the DNA repair/damage preventing enzyme. Several alternative quantitative assays are available for determining RNA levels. Each of which is based on the specific interactions between complementary nucleic acids. Table 2 below lists the human genes encoding DNA repair/damage preventing enzyme.

***Table 2***

| Enzyme | Gene (SEQ ID NO:) | Accession No. | GI No. (NCBI) |
|---|---|---|---|
| 1. Uracil DNA glycosylase | UNG (3) | NM_003362 | 6224978 |
| 2. SMUG1 | SMUG1 (4) | NM_014311 | 7657596 |
| 3. MBD4 | MBD4 (5) | NM_003925 | 4505120 |
| 4. Thymine glycosylase | TDG (6) | NM_003211 | 4507422 |
| 5. Methylpurine glycosylase | MPG (7) | NM_002434 | 4505232 |
| 6. Endonuclease III human homolog | NTH1 (8) | NM_002528 | 6224977 |
| 7. Adenine mismatch glycosylase | MYH (9) | NM_012222 | 6912519 |
| 8. 8-oxoguanine glycosylase | OGG1 (10) | NM_002542 | 7949101 |
| 9. 8-oxodGTPase | MTH1, NUDT1 (11) | NM_002452 | 4505274 |
| 10. dUTPase | DUT (12) | NM_001948 | 4503422 |
| 11. AP endonuclease | I APE1, HAP1. APEX, REF1 (13) | NM_001641 | 4502136 |
| 12. Deoxyribose phosphate lyase (of DNA polymerase β) | POLB (14) | NM_002690 | 4505930 |

Yet undescribed human genes of DNA repair/damage preventing enzyme can nowadays be readily isolated using in-silico searches, since the majority (nearly all) of the coding sequences of the human genome have been cloned and sequenced. Traditional methods of gene isolation can also be exploited as is further described in the list of references provided hereinbelow.

Based on gene sequences, Northern blot, quantitative RNA PCR (also known as quantitative RT-PCR), RNA dot blot and nucleic acid chip assays can be readily developed and used to determine the level of a specific RNA, such as mRNA, in a biological sample. Further details concerning these assays are available in, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "PCR Protocols: A Guide To Methods And Applications", Academic Press, San Diego, CA (1990).

In an alternative embodiment, and as is further described in detail below and exemplified by the Examples section that follows, the factor which is determined is the catalytic activity *per se* of the DNA repair/damage preventing enzyme.

The present invention is useful in determining a risk of a subject to develop cancer, whereby any type of cancer is subject to risk determination by way of implementing the method of the invention. It is well known that all cancers arise from DNA mutations and that the progress of a specific cancer from a primary tumor to a metastatic tumor, reflects clonal selection of cancer cells that accumulate mutations as they develop and turn more cancerous (e.g., proliferate more rapidly, escape proliferation control, acquire autosignalling behavior, induce angiogenesis, etc.) and more metastatic. This process is subject to variations depending on the specific genes involved in the development and progression of different cancers. It is therefore expected that different *in vivo* DNA repair/damage preventing activities are required to prevent the formation of different cancers. Also, the level of exposure of body tissues to genotoxic agents such as smoke and radiation, differs. Since different types of genotoxic agents cause different types of DNA lesions, it is again expected that different *in vivo* DNA repair/damage preventing activities are required to prevent the formation of different cancers.

The results obtained while reducing the present invention to practice are in agreement with the above, as low OGGA was found to be associated with some, but not all cancers tested. However, assays similar to the OGGA assay described herein can be readily developed for correlating other cancers with one or more DNA lesions, some of which are listed in Table 3 below.

In effect, all known cancers can be evaluated by finding correlation or non-correlation between the occurrence thereof and the occurrence of low DNA repair/damage preventing activity of certain types. When positive correlation is identified, a predictive risk determination assay can be readily implemented.

Thus, according to an aspect of the present invention there is provided a method of determining a presence of correlation or non-correlation between an activity of at least one DNA repair/damage preventing enzyme and at least one cancer. The method according to this aspect of the invention is effected by determining a level of activity of at least one DNA repair/damage preventing enzyme in tissue derived from a plurality of cancer patients and a plurality of apparently normal individuals, and, according to the level determining the correlation or non-correlation between the activity of the at least one DNA repair/damage preventing enzyme and the at least one cancer. This aspect of the invention is exemplified herein with respect to a single DNA repair enzyme activity (8-oxoguanine DNA glycosylase) using a suitable substrate having a single lesion therein, for a plurality of cancers, for some correlation was found, whereas for other, non-correlation was found.

Thus, the methods of determining a risk of a subject to develop cancer described herein can be implemented for a variety of cancers, including, but not limited to, lung cancers, e.g., small-cells lung cancer and non-small cells lung cancer, blood cancers, e.g., lymphomas and leukemias, including, for example, Hodgkin's lymphoma, non-Hodgkin's lymphoma, acute lymphocytic leukemia, acute myelocytic leukemia, chronic lymphocytic leukemia, chronic myelogenous leukemia and the like, colorectal cancer, breast cancer, prostate cancer, ovary cancer, malignant melanoma, stomach cancer, pancreas cancer, urinary cancer; uterus cancer, bone cancer, liver cancer, thyroid cancer, brain cancer; head and neck cancer, including, for example, salivary carcinoma and laryngeal carcinoma.

DNA repair/damage preventing activity can be measured in extracts of different body tissues or cells, which may be collected from a testee by known methods. Blood cells, scraped cells (e.g., mouth or skin scrapes) and biopsies are good examples as such tissues are routinely removed from subjects for diagnostics.

Several types of DNA repair/damage preventing activities can be assayed according to the present invention, e.g., DNA N-glycosylase, nucleotide pool sanitizing activity (dNTPase activity, e.g., 8-oxodGTPase), AP endonuclease and deoxyribose phosphate lyase (of DNA polymerase β).

An assay for determining the activity of a DNA N-glycosylase is described and exemplified herein with respect to 8-oxoguanine DNA glycosylase. In this respect it is convenient to monitor the nicking activity of DNA N-glycosylase towards DNA substrates including one or more lesion.

An assay for monitoring the activity of 8-oxodGTPase is, for example, as described by Mo *et al.* [Mo, J.-Y., Maki, H. and Sekiguchi, M. (1992) Proc. Natl. Acad. Sci. USA 89, 11021-11025]. Thus, 8-oxodGTPase activity can be assayed by measuring the hydrolysis of $\alpha$-$^{32}$P-labeled 8-oxodGTP to 8-oxodGMP. The reaction mixture (12.5 μl) contains 20 mM Tris-HC1 (pH 8.0), 4 mM $MgCl_2$, 40 mM NaCl, 20 μM $\alpha$-$^{32}$P-labeled 8-oxodGTP, 80 μg/ml bovine serum albumin, 8 mM dithiothreitol, 10 % glycerol, and a protein extract. The reaction is executed at 30 °C for 20 minutes. Thereafter, an aliquot (2 μl) from the reaction mixture is spotted onto a PEI-cellulose TLC plate, and the mixture is fractionated with a solution containing 1 M LiCl for 1 hour. The spots on the TLC plate are then visualized and quantified by phosphorimaging. The preparation of 8-oxodGTP is described in Mo *et al., ibid.*

An assay for monitoring the activity of AP endonuclease is, for example, as described by Wilson III, *et al.* [Wilson III, D. M., Takeshita, M., Grollman, A. P., Demple B. (1995) Incision activity of human apurinic endonuclease (Ape) at abasic site analogs in DNA. J. Biol. Chem. 270, 16002-16007]. The reaction mixture (10 μl) contains 50 mM Hepes-KOH pH 7.5, 50 mM KCl, 100 μg/ml bovine serum albumin, 10 mM $MgCl_2$, 0.05 % Triton X-100, 2 pmol of a the DNA substrate and a protein extract. Reactions are performed at 37 °C for 5-30 minutes, after which the reaction products are fractionated by urea-PAGE, to separate the intact and incised DNA strands. The activity is deduced from the extent of cleavage of the substrate. The preparation of the substrate is described in the same reference (Wilson III *et al., ibid.*).

An assay for monitoring the activity of deoxyribose phosphate lyase (dRPase) is, for example, as described by Prasad *et al.* [Prasad, R., Beard, W. A., Strauss, P. R. and Wilson, S. H. (1998) Human DNA polymerase β deoxyribose phosphate lyase. Substrate specificity and catalytic mechanism. J. Biol. Chem. 273, 15263-15270]. Deoxyribose phosphate lyase (dRPase) activity can be assayed by following the removal of deoxyribose phosphate from a $^{32}$P 3' end-labeled duplex oligonucleotide containing a site-specific 5'-incised abasic site. The reaction mixture (10 μl) contains 50 mM Hepes pH 7.4, 2 mM dithiothreitol, 5 mM $MgCl_2$, 20 nM $^{32}$P-labeled duplex oligonucleotide with a site specific abasic site (pre-incised at the 5' with AP endonuclease), and a protein extract. The reaction is carried out at 37 °C for 15 minutes.

After the reaction is terminated, the product is stabilized by the addition of $NaBH_4$ to a final concentration of 340 mM, and incubated for 30 minutes at 0 °C. The DNA is then ethanol precipitated and fractionated by urea-PAGE. The activity of the dRPase is deduced from the extent of formation of the shorter reaction product. The preparation of the DNA substrate is described in the same reference (Prasad *et al., ibid.*).

Table 3 below lists examples of DNA repair enzymes, the genes encoding same and the DNA lesion(s) they recognize:

***Table 3***

| Enzyme | Gene | Substrate |
|---|---|---|
| 1. Uracil DNA glycosylase | UNG1,2 | uracil, 5-fluorouracil, 5-hydroxyuracil isodialuric acid, alloxan |
| 2. hSMUG1 | hSMUG1 | uracil |
| 3. hMBD4 | hMBD4 | U or T in U/TpG:5meCpG |
| 4. Mismatch-specific thymine/uracil glycosylase | TDG | uracil (U:G), 3,$N^4$-ethenocytosine DNA (eC:G), T (T:G) |
| 5. Methylpurine DNA glycosylase | MPG (ANPG, Aag) | 3-methyladenine, 7-methyladenine, 3-methylguanine, 7-methylguanine 8-oxoguanine, hypoxanthine, 1, N6-ethenoadenine, 1,N2-ethenoguanine |
| 6. hNTH1 (human enodonuclease III ehomolog) | hNTH1 | thymine glycol, cytosine glycol, dihydrouracil, formamidopyrimidine urea |
| 7. Adenine-specific mismatch DNA glycosylase (human mutY homolog) | hMYH | A from A:G; A:8-oxoG; C:A 2-hydroxyadenine |
| 8. 8-oxoguanine DNA glycosylase | hOGG1 | 2,5-amino-5-formamidopyrimidine 7,8-dihydro-8-oxoguanine |
| 9. 8-oxo-GTPase/8-oxodGTPase (Human MutT homolog) | hMTH1 (NUDT1) | 8-oxo-GTP, 8-oxo-dGTP |
| 10. dUTPase | hDUT | dUTP |
| 11. AP endonuclease | APE1/APE2 | abasic site |
| 12. Deoxyribose phosphate lyase | POLB | Incised abasic site |

Enzymes 1-8 are DNA glycosylases;
Enzymes 9 and 10 hydrolyze damaged or unnatural dNTPs, thereby preventing their incorporation into DNA during DNA synthesis.
Further details concerning mammalian DNA repair genes and activity can be found in Krokan et al. (2000) FEBS Letters 476:73-77; and Wood et al. (2001) Science 291:1284-1289, both are incorporated herein by reference.

The risk according to the present invention can be expressed in one of a plurality of ways. In one example the risk is expressed as a fold risk increase in developing cancer as is compared to a normal, apparently healthy, population, or a reference control group. In another example, the risk is expressed in enzyme specific activity units. In another example, a linear or logarithmic risk scale is generated for either the "fold risk increase" or the "activity units" and the risk is expressed as a magnitude of the scale.

According to still further features in the described preferred embodiments determining the level of activity of the DNA repair/damage preventing enzyme is effected using a DNA substrate having at least one lesion therein.

As is schematically exemplified by Figures 12a-b, a monomolecular (MMS, Figure 12a) or plurimolecular (PMS, Figure 12b) universal substrate can also be generated and used while implementing the methods and kits of the present invention. Such a universal substrate is used according to the present invention to simultaneously determine the activity of more than a single DNA repair/damage preventing enzyme. Thus, a universal substrate of the invention includes at least two (four are shown in Figures 12a-b identified by L1-L4) different DNA lesions specifically recognized by at least two different DNA repair enzymes. Careful selection of the positions of the different DNA lesions along the universal substrate, can be used to ensure the generation of distinguishable (e.g., size distinguishable) reaction products (P1-P10 in Figure 12a, P11-14 in Figure 12b), being indicative of the activity of the different DNA repair enzymes. In order to ensure accuracy, the lesions are selected to be unique to the activities tested. The length of the universal substrate, especially for a monomolecular substrate, which preferably includes labels along its length, is selected such that reciprocal reaction products are substantially longer than all of the reaction products to be analyzed (P1-P10 in Figure 12a). End labeling can be used in the case of a plurimolecular substrate to circumvent this problem altogether. Thus, the length of a substrate according to the present invention, without limitation, can range between 10 base pairs and several hundreds base pairs.

A substrate of the invention can thus have at least one lesion of at least one type or at least one lesion of at least two types (universal substrate), the lesions preferably being positioned at predetermined site(s) in the DNA substrate. The lesion(s) can be of any type, including, but not limited to, uracil, 5-fluorouracil, 5-hydroxyuracil, isodialuric acid, alloxan, uracil or thymine in U/TpG:5meCpG, uracil (U:G), 3,$N^4$-ethenocytosine, (eC:G), T (T:G), 3-methyladenine, 7-methyladenine, 3-methylguanine, 7-methylguanine, hypoxanthine, 1, N6-ethenoadenine, 1,N2-ethenoguanine, thymine glycol, cytosine glycol, dihydrouracil, formamidopyrimidine urea, adenine from A:G; A:8-oxoG; C:A, 2-hydroxyadenine, 2,5-amino-5-formamidopyrimidine, 7,8-dihydro-8-oxoguanine and abasic site.

A lesion can be introduced at a unique and defined location (site) in a DNA molecule using solid phase DNA synthesis, using in sequence the four conventional phosphoramidite building blocks used in the synthesis of oligodeoxynucleotides and additional at least one modified phosphoramidite building block, which when introduced into the DNA introduces a lesion therein, which lesion is recognizable by a DNA repair enzyme. In the alternative, a DNA molecule is exposed to a mutagenic agent (e.g., an oxidative agent or UV radiation) which forms one or more lesion of one or more types therein. Even when using this method, one can select a presubstrate which will result in a product (substrate of the invention) in which the lesions are non-randomly distributed, since the extent by which a specific lesion is formed in DNA is often dependent on the DNA sequence.

Other alternatives also exist. For example, one can oxidize a plasmid DNA with an oxidizing agent. This will form several lesions in the plasmid DNA. One can now use this plasmid DNA to assay a repair enzyme that acts on this DNA, without knowing precisely where the lesions are. The enzyme will produce a nick in the DNA, and this will convert the plasmid from the supercoiled closed form to the nicked (open circular) form. These two can be easily distinguished by gel electrophoresis or gradient centrifugation. In another example a piece of DNA is enzymatically synthesized in the presence of lesioned building blocks. Other alternatives are also known, such as chemical deamination, etc.

Thus, the substrate of the present invention can include at least two different lesions of at least two types, a single lesion, or at least two different lesions of a single type.

A cancer risk determination test according to the present invention is specifically advantageous for a subject which is known to be, or is about to be, exposed to environmental conditions associated with increased risk of developing cancer, such as smoking and occupational exposure to smoke, ionizing radiation and other carcinogens.

As is discussed hereinabove, the effectiveness of cancer therapy is due to its genotoxic effect affecting cancer cells more than normal cells. Thus, according to another aspect of the present invention there is provided a method of predicting the efficacy of a mutagenic anti-cancer treatment, such as chemotherapy and/or radiotherapy, in a subject. The method according to this aspect of the invention is effected by determining a level of activity of a DNA repair enzyme in a tissue of the subject, and, according to the level, predicting the efficacy of the mutagenic anti-cancer treatment in the subject.

Anti cancer therapy dosage can also be individually optimized in view of the teachings of the present invention. Thus, according to still another aspect of the present invention there is provided a method of selecting dosage of a mutagenic anti-cancer treatment, such as chemotherapy and/or radiotherapy, for treating a subject. The method according to this aspect of the invention is effected by determining a level of activity of a DNA repair/damage preventing enzyme in a tissue of the subject, and, according to the level, selecting dosage of the mutagenic anti-cancer treatment for treating the subject. In this case, the tissue is preferably a biopsy derived from the cancer itself.

According to an additional aspect of the present invention there is provided a kit for determining a level of activity of a DNA repair/damage preventing enzyme in a tissue of a subject. In its minimal configuration, the kit includes, a package including, contained in sealable containers, a DNA substrate having at least one lesion therein and a reaction buffer selected suitable for supporting DNA repair activity. Preferably, the kit also includes test tubes for separating lymphocytes. Preferably, the test tubes are prepackaged with an anti-coagulant, such as, but not limited to, heparin. Still preferably, the kit further includes a liquid having a specific gravity selected effective in separating lymphocytes from red blood cells via centrifugation, e.g., Ficol1 contained in lymphocytes isolation tubes. Advantageously, the kit includes a solution having osmolarity selected effective in lysing red blood cells. In a preferred embodiment of the invention a protein extraction buffer is also included in the kit. Preferably, the kit further includes reagents for conducting protein determinations, e.g., reagents included in the BCA kit by Pierce. Still preferably, the kit includes a purified DNA repair enzyme, which serves as a control for such activity.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

## EXAMPLES

Reference is now made to the following examples, which together with the above descriptions, illustrate the invention in a non limiting fashion.

### *Materials and Experimental Methods*

***DNA substrates****:* The DNA substrate was prepared by annealing two complementary synthetic oligonucleotides, 32-bases long each. They were synthesized by the Synthesis Unit of the Biological Services Department at the Weizmann Institute of Science. The oligonucleotide containing 8-oxoG had the sequence 5'-CCGGTGCATGACACTGTOACC TATCCTCAGCG-3' (SEQ ID NO:1) (O = 8-oxoG). The 8-oxoG phosphoramidite building block was purchased from Glen Research. The oligonucleotide was $^{32}$P-labeled using T4 polynucleotide kinase, and annealed to the oligonucleotide 5'-CGCTGAGGATAGGTCACAGTGTCATGCA CCGG-3' (SEQ ID NO:2).

The radiolabeled duplex was purified by PAGE on a native 10 % gel. Its concentration was determined by the PicoGreen dsDNA quantitation assay (Molecular Probes).

***Blood samples****:* Large blood samples were obtained from the blood bank in the Sheba Medical Center. Samples of 10 ml peripheral blood were obtained from healthy donors or from cancer patients. Those were collected after obtaining permission from the Institutional Helsinki Committee.

***Isolation of peripheral lymphocytes:*** The blood samples were processed 18-24 hours after collection. A 100 μl aliquot from each sample of whole blood was analyzed using a Cobas Micros (Roche Diagnostic System) blood counter. Ten ml PBS (Dulbecco's phosphate buffered saline, Sigma) were added to the remaining blood portion, and peripheral blood lymphocytes were isolated by density gradient centrifugation of the diluted whole blood on a polysucrose-sodium metrizoate medium in UNI-SEP tube (NOVA*med*, Jerusalem, Israel). Centrifugation was performed at 1,000 x g for 30 minutes at 20 °C.

Following centrifugation the lymphocyte band was removed and washed with PBS buffer. Elimination of red blood cells was done by lysis in 5 ml of 155 mM $NH_4Cl$; 0.01 M $KHCO_3$; 0.1 mM EDTA for 4 minutes at room-temperature. The lymphocytes were washed with PBS, and suspended in 1 ml PBS. The number of white blood cells in this suspension was determined using a Cobas Micros (Roche Diagnostic System) blood counter.

Samples containing 1-4 x $10^6$ cells were precipitated by centrifugation at 5,000 rpm, for 4 minutes at room temperature. The cells pellet was then resuspended to a concentration of 20,000 cells/μl in 50 mM Tris·HCl (pH 7.1), 1 mM EDTA, 0.5 mM DTT, 0.5 mM spermidine, 0.5 mM spermine, and a protease inhibitor cocktail (Sigma). The cells were incubated on ice for 30 minutes, after which they were frozen in liquid nitrogen. The frozen lymphocytes were stored at -80 °C.

***Preparation of a protein extract****:* The frozen lymphocytes were thawed at 30 °C, after which their protein content was extracted with 220 mM KCl, for 30 minutes on ice. Cell debris was removed by centrifugation at 13,200 rpm for 15 minutes at 4 °C. Glycerol was added to the protein extract to a final concentration of 10 %, and the extract was frozen in liquid nitrogen. Protein concentration was determined by the BCA assay kit (Pierce) using bovine γ -globulin as a standard.

***Standard analysis of OGG activity:*** The reaction mixture (20 μl) contained 50 mM Tris·HCl (pH 7.1), 1 mM EDTA, 115 mM KCl, 20 μg bovine γ-globulin, 2 pmol PolydA·polydT, 0.5 pmol substrate and 8-12 μg protein extract. The reaction was carried out at 37 °C for 30 minutes, after which it was stopped by the addition of 15 mM EDTA, 0.2 % SDS. The proteins were degraded by incubation with proteinase K (20 μg) for one hour at 37 °C, after which they were treated with 80 mM NaOH for 30 minutes at 37 °C. The denatured DNA products were analyzed by electrophoresis on a 15 % polyacrylamide gel containing 8 M urea, in 89 mM Tris·borate, 2.5 mM EDTA pH 8.0, at 1,500 V for 2 hours at 45-50 °C. The distribution of radiolabeled DNA products was visualized and quantified using a Fuji BAS 2500 phosphorimager. One unit of OGG activity is defined herein to cleave 1 fmol of DNA substrate in 1 hour at 37 °C, under the standard reaction conditions described herein. In the following, OGGA is presented as specific activity, i.e., activity units/ 1 μg of total protein extract.

***Statistical analysis:*** A 3-way ANOVA was employed for healthy subject to compare mean OGGA values, with gender, age (?50, <50), and smoking status as fixed effects.

Student's *t*-test was used to compare the mean OGGA values, analyzed as a continuous variable, between adenocarcinoma and squamous cell carcinoma patients.

To neutralize possible effects on OGGA means originating from the difference in mean age between the cases and controls, OGGA means were compared using ANCOVA, with age (treated as a continuous variable) as a covariate. This analysis was possible since no significant interaction was found between age and health conditions.

Associations were calculated using Fisher's exact test, and Odds ratios (OR) were calculated from a 2 X 2 table. Adjusted ORs and CI values were calculated by fitting logistic regression models with adjustment for age, sex and smoking status for lung cancer; and adjustment for age only, for lymphoma. OGGA values were analyzed as a continuous variable or as a dichotomized variable at values corresponding to 4 % (OGGA cutoff at 5.5), 5 % (OGGA cutoff at 5.6), 10 % (OGGA cutoff at 5.9), 15 % (OGGA cutoff at 6.2), 25 % (OGGA cutoff at 6.4) or 50 % (OGGA cutoff at 7.3) of the control group. Age was analyzed as a continuous variable, whereas gender and smoking status were analyzed as dichotomic variables.

Odds Ratio (OR) were calculated by the formula (Kleinbaum, 1994)

$$OR_{X1\text{-}X0} = e^{\sum_{i=1}^{k} b_i(X_{1i}-X_{0i})}$$

using the $b_i$ estimates from the logistic regression model where OGGA values were analyzed as a continuous variable ($b_{OGGA}$ = -0.624; $b_{age}$ = 0.1; $b_{smoker}$ = 2.9). For example, $X_0$, the reference, was used to represent non-smoking, 30 years-old individuals with an OGGA value of 7.0, and $X_i$ was used to represent the tested subject group. Thus the formula for the current model is:

$$ORi = e^{-0.624(OGGA_i-7.0) + 0.1 (Age_i-30) + 2.9S_i}$$

where $OGGA_i$ and $Age_i$ are the OGGA value and age of individual i, and $S_i$ is either 1 or zero, for a smoker or a non smoker, respectively.

All the statistical analyses were performed using SAS software (version 6.12; SAS Institute Inc., Cary, NC).

***Experimental Results***

***The OGG activity (OGGA) DNA repair test:*** Base excision repair (BER) is initiated by a DNA N-glycosylase, that releases the damaged or unusual base from DNA, generating an abasic site. The latter is then repaired by an AP endonuclease (APE/HAP1) and/or the lyase activity of the glycosylase, as well as the deoxyribose phosphate lyase (dRPase) activity of DNA polymerase β. The resulting gap is filled-in by DNA polymerase β, forming a patch of 1-3 nucleotides, followed by ligation (Dianov, *et al.,* 1992, Singhal, *et al.,* 1995). A long patch pathway of BER was identified which requires also PCNA, and the FEN-1 flap endonuclease (Fortini, *et al.,* 1998, Kim, *et al.,* 1998). It was reported that 8-oxoG can be repaired in cell extracts also by nucleotide excision repair (Reardon, *et al.,* 1997), however, the *in vivo* significance of this finding is not clear (Runger, *et al.,* 1995). In addition, it was reported that there is transcription-coupled repair of 8-oxoG, and that it required the XPG, TFIIH and CSB (Le Page, *et al.,* 2000), and the BRCA1 and BRCA2 proteins (Le Page, *et al.* , 2000).

While reducing the present invention to practice, an assay was developed for OGG activity (the OGGA test), using as substrate a $^{32}$P end-labeled synthetic oligonucleotide, 32-base pairs long, carrying a site-specific 8-oxoG. The source of the OGG activity was a protein extract prepared from human peripheral blood lymphocytes (PBL), obtained by Ficoll fractionation from 10 ml blood samples. A protein extract was prepared from the lymphocytes by freeze-thaw, followed by salt extraction. The removal of 8-oxoG from the oligonucleotide, by the OGG activity in the extract, generates an abasic site, which was rapidly incised either by the AP lyase activity of the enzyme, or by AP endonucleases present in the extract. Alkali treatment, which breaks abasic sites, was performed after the incubation with the extract in order to ensure complete cleavage of the abasic site, such that only OGG activity is measured in the test. Analysis by urea-PAGE followed by phosphorimaging was used to quantify the extent of nicking, indicated by the formation of a shorter radiolabeled DNA fragment, 17 nucleotides long (Figure 1a). The OGG activity level (OGGA value) is measured as specific activity, i.e., units of OGG activity/1 μg of protein extract. One unit of OGG activity cleaves 1 fmol of DNA substrate in 1 hour at 37 °C, under standard reaction conditions.

Figures 1b-c and 2a-b show a time course and a titration of OGG activity, respectively, in lymphocyte extracts. The activity was dependent on the presence of 8-oxoG in the DNA substrate. No activity was observed when the DNA contained a G instead of the 8-oxoG. This observed activity is mostly due to the OGG1 enzyme, which was shown to be responsible for most of OGG activity in extracts prepared from human cells (Monden, *et al.,* 1999). The existence of OGG2, a second OGG enzyme was reported. However, its activity was much lower than OGG1 in whole cell extracts (Hazra, *et al.,* 1998). In addition to OGG, APNG (alkylpurine DNA N-glycosylase), also termed Aag (alkyladenine DNA glycosylase), or MPG (N-methylpurine glycosylase), was reported to act on 8-oxoG (Bessho, *et al.,* 1993) but this finding was challenged in Hang, *et al.* (1997). *In vivo* this protein has no significant role in removing 8-oxoG from DNA, at least in mice (Engelward, *et al.,* 1997, Hang, *et al.,* 1997). In order to establish whether MPG is involved in the removal of 8-oxoG from DNA by lymphocyte extracts, a competition experiment was performed with an unlabeled duplex oligonucleotide containing a site-specific hypoxanthine (a substrate of MPG but not for OGG1; (see, Engelward, *et al.,* 1997, Hang, *et al.,* 1997)). As can be seen in Figure 3, this duplex oligonucleotide did not inhibit the incision of the 8-oxoG-containing DNA by the extract, suggesting that APNG is not involved in the incision reaction. A control experiment with an excess of unlabeled duplex oligonucleotide containing a G instead of 8-oxoG showed no inhibition, whereas a duplex oligonucleotide containing 8-oxoG-DNA did cause inhibition, as expected (Figures 3a-b). These competition experiments are an indication of the specificity of the OGGA test to 8-oxoG.

Reproducibility experiments showed that the assay is accurate and highly reproducible, with a coefficient of variation ?10 %. An example of a reproducibility experiment is shown in Table 4.

***Table 4***
***Reproducibility of the OGGA test***

**A**

| **Blood sample:** | 1 | 2 | 3 | 4 | 5 | 6 | 7 | 8 | 9 | 10 | 11 | 12 |
|---|---|---|---|---|---|---|---|---|---|---|---|---|
| **OGGA (units/μg protein):** | 6.8 | 6.9 | 6.4 | 6.5 | 6.7 | 7.4 | 6.9 | 6.5 | 6.6 | 6.7 | 5.9 | 6.9 |

| | |
|---|---|
| Average OGGA: | 6.7 |
| Standard variation: | 0.4 |
| Coefficient of variance: | 6 % |

Twelve blood samples from a healthy donor (donor No. 54), 10 ml each, were processed and assayed for OGGA. One unit of OGG activity incises 1 fmol GO-containing substrate in 60 minutes at 37 °C under standard assay conditions.

**B**

| **Blood Sample:** | 1 | 2 | 3 | 4 | 5 | 6 | Ave | SD | CV |
|---|---|---|---|---|---|---|---|---|---|
| **OGGA** (units/μg protein) | | | | | | | | | |
| Experiment 1: | 6.7 | 7.2 | 6.7 | 6.8 | 7.1 | 6.7 | 6.9 | 0.2 | 3% |
| Experiment 2: | 7.9 | 6.9 | 7.8 | 8.2 | 8.1 | 7.8 | 7.8 | 0.5 | 6% |
| Experiment 3: | 6.9 | 7.0 | 7.1 | 7.3 | 7.9 | 7.2 | 7.2 | 0.4 | 5% |

| | |
|---|---|
| Overall average OGGA: | 7.1 |
| Standard deviation (SD): | 0.5 |
| Coefficient of variance (CV): | 7 % |

Six blood samples from a healthy donor (donor No. 50), 10 ml each, were processed to prepare protein extracts. The table shows the results of three independent assays performed with these assays on three different days.

***OGGA value in healthy individuals (control subjects):*** The OGGA test was performed on blood samples from 123 healthy individuals, and the distribution is shown in Figure 4. The mean OGGA value was 7.2±1.0 units/μg protein (this will be also dubbed OGGA value of 7.2±1.0; Table 5).

***Table 5***
***OGGA values in healthy individuals***

| Factor | No. | Mean OGGA±SD* | P** |
|---|---|---|---|
| All | 123 | 7.2±1.0 | |
| Age, years | | | |
| <50 | 34 | 7.6±0.9 | |
| ≥50 | 89 | 7.0±1.0 | 0.02 |
| Gender | 53 | 7.3±1.0 | |
| Male | 70 | 7.1±1.0 | 0.36 |
| Female | | | |
| Smoking status | | | |
| Never | 88 | 7,1±1.0 | |
| Current | 35 | 7.3±1.0 | 0.46 |

*SD, standard deviation.
**P values are results of 3-way ANOVA.

The range of OGGA was 3.6-10.1 units/μg protein, representing a 2.8-fold range of OGG activity. This is a rather narrow distribution of activity, significantly narrower than previously reported (Asami, *et al.,* 1996).

A 3-way ANOVA with gender, age (<50, ?50), and smoking status revealed that there was no significant difference in mean OGGA value between men (53 individuals; 7.3±1.0 units/μg protein) and women (70 individuals; 7.1±1.0 units/μg protein; P=0.36 Figure 5; Table 5), or between smokers (N=35; 7.3±1.0 units/μg protein) and non-smokers (N=88; 7.1±1.0 units/μg protein; P=0.46). This indicates that smoking does not affect the OGGA value in peripheral blood lymphocytes (Figure 6; Table 5). This result differs from the result obtained by Asami *et al.* (1996), who reported that 8-oxoG repair activity was increased 1.6-fold in smokers. In contrast, there was a small (6.6 %), but statistically significant decrease in mean OGGA values between the two age groups: Individuals under the age of 50 had a mean OGGA value of (7.6±0.9; N=34), whereas those 50 years or older had a mean OGGA value of (7.0±1.0; N=89; P=0.02; Figure 7; Table 5). Taken together these results indicate little or no variation of the OGGA value with age, smoking status and gender.

***OGGA is not reduced in patients with breast cancer, and is altered in chronic lymphocytic leukemia (CLL):*** The OGGA test was performed on blood samples from 31 breast cancer patients and 19 CLL patients. As can be seen in Table 6, the mean OGGA value was 7.3±1.4 units/μg protein in breast cancer patients, similar to that of control female subjects (7.1±1.0, P=0.29; Tables 5 and 6). Also, the distribution of OGGA values was similar (Figures 8a-d). These results indicate that OGGA is not a risk factor in breast cancer. The mean OGGA value of CLL patients was 7.9±1.5, higher than the control subjects (7.2±1.0, P=0.0007; Table 6). However, the distribution of OGGA values among CLL patients was similar to the control group (Figures 8c-d).

***Table 6***
***Mean OGGA values in cancer patients***

| Healthy/Disease | No. | Mean OGGA±SD* | P** |
|---|---|---|---|
| Healthy | 123 | 7.2±1.0 | |
| Lung cancer (NSCLC) | 102 | 6.0±1.5 | 0.0001 |
| Breast cancer | 31 | 7.3±1.4 | 0.29 |
| Lymphoma | 18 | 6.2±1.8 | 0.0001 |
| CLL | 19 | 7.9±1.5 | 0.0007 |

(continued)

***Mean OGGA values in cancer patients***

| Healthy/Disease | No. | Mean OGGA±SD* | P** |
|---|---|---|---|
| Colorectal cancer | 16 | 7.5±1.8 | 0.047 |

*SD, standard deviation.
** OGGA means were compared using ANCOVA, with age (treated as a continuous variable) as a covariate. For breast cancer patients, the control group consisted of female subjects.

***OGGA is a risk factor in lung cancer:*** The OGGA test was performed with blood samples from 102 patients who suffered from operable non-small cell lung cancer (NSCLC), and had not been subjected to either chemo- or radiotherapy at the time when the blood samples were taken. As can be seen in Figures 9a-b and Table 6, the mean OGGA value was 6.0±1.5 units/μg protein, significantly lower than the mean value of controls (7.2±1.0, P=0.0001). Analyzing separately cases with adenocarcinoma or squamous cell carcinoma revealed a similar OGGA level in these two main sub-types of NSCLC: adenocarcinoma: 6.1±1.5, N=37; squamous cell carcinoma: 5.8±1.6 N=35; P=0.44 (the other 30 cases were either other sub-types or unclassified NSCLC). The comparison of the distributions of OGGA in controls and in cases highlights the difference between the two groups. As can be clearly seen in Figures 9a-b, there is a shift to lower OGGA values in cases as compared to controls. For example, only 4 % of controls have OGGA values of ?5.5, whereas 38 % of cases have OGGA values in this range. This includes values 2-3 fold lower than the mean OGGA values of the controls. The mean age of the control group (57±14) was significantly different from the cases group (68±10; P<0.0001). Therefore, logistic regression, adjusted for age, was used to analyze associations, and analysis of covariance was used to compare age-adjusted mean OGGA values.

To analyze the association between levels of OGGA and presence of lung cancer logistic regression was used, where the binary dependent variable was presence/absence of lung cancer, and with age as a continuous variable, and gender, smoking status and OGGA as dichotomic variables. The latter was dichotomized at values corresponding to 5 % (OGGA cutoff at 5.6), 10 % (OGGA cutoff at 5.9), 15 % (OGGA cutoff at 6.2), 25 % (OGGA cutoff at 6.4) or 50 % (OGGA cutoff at 7.3) of the control group. As can be seen in Table 7, smoking is strongly associated with lung cancer, in agreement with its established role as a major risk factor in the disease. For example, in a model where OGGA was dichotomized at ?5.9 (corresponding to 10 % of the controls), the Odds Ratio (OR) for smokers was 20.8 (95 % CI 7.8-55.4, P=0.0001). In a model where the OGGA cutoff was defined as ?7.3 (corresponding to 50 % of controls), the OR for smokers was 23.0 (95 % CI 8.9-59.2, P=0.0001). The gender had no significant effect in any of the models, whereas increased age was associated with the presence of lung cancer. Notice that although the OR for age was relatively small in all models (1.1 95 % CI 1.1-1.2, P=0.0001) it is statistically significant. The age was analyzed as a continuous variable, and the relatively small OR is given per one-year change. Therefore, its final effect when applied to a particular change of age might be much larger (see Table 8 below).

As can be seen from Table 7, a clear association was found between the level of OGGA and presence of lung cancer. Moreover, there is a dose-dependent effect, with higher OR obtained for lower OGGA. For example, OR values of 3.9, 5.2, 7.0 and 9.0 were obtained for cutoff OGGA values of 7.3, 6.4, 5.9 and 5.6, respectively (Table 7). These high OR values indicate a strong association between low OGGA and lung cancer. Moreover, the increase in OR with decreasing OGGA further strengthens the significance of the association. In addition, the high OR values argue against the possibility of a selection bias in the control group.

***Table 7***

***Association of low OGGA and lung cancer***

| OGGA cutoff† | Controls | Cases | Adjusted Odds Ratio (95 % CI)* | | | |
|---|---|---|---|---|---|---|
| | | | DNA repair OGGA | Smoking | Age | Sex |
| ≤5.6 (5%) | 7 | 42 | 9.0 (3.2-25.0) | 18.6 (7.1-48.7) | 1.1 (1.1-1.2) | 1.0 (0.4-2.1) |
| 5.6 | 116 | 60 | | | | |
| | | | P=0.0001 | P=0.0001 | P=0.0001 | P=0.79 |
| ≤5.9 (10%) | 14 | 52 | 7.0 (3.0-16.7) | 20.8 (7.8-55.4) | 1.1 (1.1-1.2) | 1.0 (0.4-2.2) |
| 5.9 | 109 | 50 | | | | |
| | | | P=0.0001 | P=0.0001 | P=0.0001 | P=0.96 |

(continued)

***Association of low OGGA and lung cancer***

| OGGA cutoff† | Controls | Cases | Adjusted Odds Ratio (95 % CI)* | | | |
|---|---|---|---|---|---|---|
| | | | DNA repair OGGA | Smoking | Age | Sex |
| ≤6.2 (15%) | 18 | 60 | 6.5 (2.9-14.5) | 21.5 (8.0-58.0) | 1.1 (1.1-1.2) | 0.9 (0.4-2.0) |
| 6.2 | 105 | 42 | | | | |
| | | | P=0.0001 | P=0.0001 | P=0.0001 | P=0.77 |
| ≤6.4 (20%) | 25 | 63 | 5.2 (2.4-11.2) | 20.6 (7.9-53.6) | 1.1 (1.1-1.2) | 0.9 (0.4-2.0) |
| 6.4 | 98 | 39 | | | | |
| | | | P=0.0001 | P=0.0001 | P=0.0001 | P=0.77 |
| ≤6.6 (25%) | 34 | 68 | 4.3 (2.0-9.0) | 21.4 (8.2-55.5) | 1.1 (1.1-1.2) | 0.9 (0.4-2.0) |
| 6.6 | 89 | 34 | | | | |
| | | | P=0.0002 | P=0.0001 | P=0.0001 | P=0.85 |
| ≤7.3 (50%) | 63 | 83 | 3.9 (1.7-8.6) | 23.0 (8.9-59.2) | 1.1 (1.1-1.2) | 0.9 (0.4-1.8) |
| 7.3 | 60 | 19 | | | | |
| | | | P=0.0009 | P=0.0001 | P=0.0001 | P=0.67 |

The logistic regression model is based on age as a continuous variable, and the dichotomic variables were smoking status (smoker, non-smoker), gender (female, male) and DNA repair activity value (low or normal, with various cutoff values, as indicated). The goodness of fit of the model, as described by $R^2$, is in the range of 58-61%.

*95% CI, 95 % confidence interval.

†Cutoff values defined for the dichotomic variable of OGGA. The numbers in parentheses show the corresponding percentage of control subjects with OGGA values lower than or equal to the cutoff value.

In case-control studies there is a possibility that the examined variable is a consequence of the disease, rather than being a risk factor. In the present case, the possibility that the lung tumor causes a decrease of OGGA in peripheral blood lymphocytes (PBL) was considered. The OGGA value may be affected, for example, by factors that the tumor secretes into the blood stream. The main treatment of NSCLC is surgical removal of the tumor. This offers a way to distinguish between a causative and a resultive model for the association of PBL OGGA and lung cancer. Once eliminated from the lung, the effect the tumor have (if any) on OGGA in lymphocytes should decay with time. No correlation between OGGA and the time period that passed between surgery and taking the blood sample (ranging from 4 months before surgery to over a year after surgery) was found, indicating that whether the samples were taken before or after surgery had no effect on the level of OGGA in PBL. In the current group of case subjects, most (67/102) samples were taken after surgery. These results, clearly indicate that reduced OGGA is indeed a risk factor in lung cancer.

The simplest biological explanation for the present finding is the following: Low OGGA in PBL reflects low OGGA in the lungs. Correlations between DNA repair activities in PBL and lung cells (Auckley *et al.,* 2001) or gastric mucosa (Kyrtopoulos *et al.,* 1990) were previously reported. The lower DNA repair capacity leads to a reduced ability to repair oxidative DNA damage, and as a result 8-oxoguanine accumulates and leads to an increased mutation rate, which causes a higher cancer risk. In smokers there is an overload of DNA damage in the lungs, and therefore a higher risk is expected. No interaction was found between OGGA values and smoking status, implying that each of the two is an independent risk factor for lung cancer. This means that low OGGA is a risk factor also in non-smokers. This is not surprising, since oxidative DNA damage is a common intracellular damage that occurs even without exposure to external agents (Lindahl, 1993).

As discussed before, OGGA is not reduced in patients with breast cancer. This suggests that the repair of 8-oxoG is a bottleneck in the case of lung cancer, and in some additional cancers, but not in others (e.g., breast cancer). This is consistent with the finding that hereditary defects in particular DNA repair genes cause predisposition to specific types of cancer. For example, defects in nucleotide excision repair were shown to cause skin cancer (Weeda *et al.,* 1993) whereas defects in mismatch repair cause hereditary non-polyposis colon cancer (Modrich, 1994). To our knowledge the results presented herein, are the first demonstration that decreased activity of a specific base excision repair enzyme is associated with cancer.

A useful application of the results of this study would be a quantitative model, which will provide an estimation of the risk of lung cancer associated with a particular OGGA value, age and smoking status. For diseases that do not occur frequently, such as lung cancer, and assuming that the cases and the controls are reasonably representative of the population, the odds ratio can be used as estimated relative risk (Gordis, 1996). Thus, a model was formulated using logistic regression, with age and OGGA as continuous variables, and smoking status as a dichotomic variable (smokers or non-smokers). This yielded OR values for lung cancer and these were taken as an estimation of risk. The OR values were calculated by dividing the odds of each particular group (having a particular age, OGGA value and smoking status) by the odds of 30 years-old non-smokers with a normal OGGA value of 7.0 (the reference group; OR of 1.0). The OR values for a specific age, OGGA value and smoking status are listed in Table 8. For example, according to Table 8, the estimated risk for 30 years-old smokers with a low OGGA value of 4.0, is 118-fold higher than the reference. At the age of 40, the estimated risk will increase to 321-fold higher than the reference. This high estimated risk is primarily the combined result of smoking and low OGGA. Having a low repair activity to start with, smoking causes further overloading of DNA damage, therefore leading to a high cancer risk. This model is instrumental in clarifying the fact that the combination of smoking and low OGGA causes a dramatic increase in susceptibility to lung cancer. For example, 40 years-old non-smokers with an OGGA value of 4.0 have an estimated risk 18-fold higher than the reference, compared to an estimated risk of 321-fold higher than the reference of smokers with the same age and OGGA (Table 8).

The OGGA test can be used to screen smokers for reduced DNA repair capacity. These individuals can be persuaded to quit smoking based on their personal reduced ability to cope with DNA damage. Since smoking is the main contributor to the high relative estimated risk for lung cancer (Tables 7 and 8), quitting smoking is expected to significantly improve the chances of preventing lung cancer. Such an approach of personalized smoking cessation, based on personal susceptibility, may provide a successful and cost-effective strategy to prevent lung cancer, and may be extended to include additional DNA repair assays.

***Table 8***

***An odds ratio model for estimating the risk of lung cancer for specific DNA repair OGGA values, age and smoking status***

| | | Estimated Risk (Odds Ratio*) | |
|---|---|---|---|
| Age, y | OGGA value | Non-smokers | Smokers |
| 30 | 7 | 1 | 18 |
| 30 | 6 | 2 | 34 |
| 30 | 5 | 3 | 63 |
| 30 | 4 | 7 | 118 |
| 30 | 3 | 12 | 221 |
| 40 | 7 | 3 | 49 |
| 40 | 6 | 5 | 92 |
| 40 | 5 | 9 | 172 |
| 40 | 4 | 18 | 321 |
| 40 | 3 | 33 | 599 |
| 50 | 7 | 7 | 134 |
| 50 | 6 | 14 | 251 |
| 50 | 5 | 26 | 468 |
| 50 | 4 | 48 | 873 |
| 50 | 3 | 90 | 1629 |
| 60 | 7 | 20 | 365 |
| 60 | 6 | 37 | 681 |
| 60 | 5 | 70 | 1272 |
| 60 | 4 | 131 | 2373 |
| 60 | 3 | 244 | 4429 |

(continued)

***An odds ratio model for estimating the risk of lung cancer for specific DNA repair OGGA values, age and smoking status***

| | | Estimated Risk (Odds Ratio*) | |
|---|---|---|---|
| Age, y | OGGA value | Non-smokers | Smokers |
| 70 | 7 | 55 | 992 |
| 70 | 6 | 102 | 1852 |
| 70 | 5 | 190 | 3456 |
| 70 | 4 | 355 | 6451 |
| 70 | 3 | 662 | 12040 |

* The Table is based on logistic regression analysis of the case-control study, and therefore the numbers represent only estimated values of risk. The odds ratio is calculated relative to the odds ratio of 30 years-old non-smokers with an OGGA value of 7.0.

The data presented herein indicates that low OGGA is a risk factor for lung cancer also among non-smokers (Tables 7 and 8). What can non-smokers with low OGGA do to protect themselves? One possibility is to make sure that they are not exposed to external sources of oxidative DNA damage such as secondary smoking or ionizing radiation. The latter includes radiology departments in hospitals, nuclear industry, and nuclear reactors. However, oxidative DNA damage is caused also by internal agents; therefore, dietary anti-oxidants might have a protective effect. Large population studies found that antioxidants had no protective effect against cancer (reviewed in Collins, 1999; Lippman and Spitz, 2001). However, these food additives might have a protective effect when taken by individuals with low capacity to repair oxidative DNA damage.

***Low OGG activity is a risk factor in lymphoma:*** Analysis of 18 lymphoma patients showed a clear shift to lower OGG DNA repair values (Figures 10a-b; Table 6): The mean OGGA value was 6.2$\pm$1.8 units/$\mu$g protein, significantly lower than in healthy individuals (P=0.0001). Analysis of Normal and Low repair in healthy individuals and in lymphoma patients using logistic regression yielded an adjusted Odds Ratio of 15.2 (95 % CI, 3.7-62.5). This means that after adjustment for age, lymphoma patients were 15 times more likely than the healthy controls to have a Low OGGA. This indicates that Low OGGA is a risk factor in lymphoma (Table 9).

***Table 9***

***Association of Low OGGA and lymphoma***

| Factor | Cases | Controls | Crude OR (95% CI*) | Adjusted** OR (95% CI) |
|---|---|---|---|---|
| OGGA | | | | |
| Normal>5.5 | 12 | 118 | | |
| Low≤5.5 | 6 | 5 | 11.8 (3.1-44.5) P=0.0006 | 15.2(3.7-62.5) P=0.0002 |

* CI, 95% confidence interval.
** Adjusted for age.

***OGG activity seems to be reduced in colorectal cancer patients:*** An analysis was performed with 16 colorectal cancer patients (Figures 11a-b). Two of the patients exhibited low OGG (12 %). This data indicates that low OGG is a risk factor in colorectal cancer.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

CITED REFERENCES

(Additional references are cited in the text)

Aburatani, H., Y. Hippo, T. Ishida, R. Takashima, C. Matsuba, T. Kodama, M. Takao, A. Yasui, K. Yamamoto and M. Asano. (1997) Cloning and characterization of mammalian 8-hydroxyguanine-specific DNA glycosylase/apurinic, apyrimidinic lyase, a functional mutM homologue, Cancer Res., 57, 2151-2156.

Arai, K., K. Morishita, K. Shinmura, T. Kohno, S. R. Kim, T. Nohmi, M. Taniwaki, S. Ohwada and J. Yokota. (1997) Cloning of a human homolog of the yeast OGG1 gene that is involved in the repair of oxidative damage, Oncogene, 14, 2857-2861.

Asami, S., T. Hirano, R. Yamaguchi, Y. Tomioka, H. Itoh and H. Kasai (1996) Increase of a type of oxidative DNA damage, 8-hydroxyguanine, and its repair activity in human leukocytes by cigarette smoking, Cancer Res., 56, 2546-2549.

Asami, S., H. Manabe, J. Miyake, Y. Tsurudome, T. Hirano, R. Yamaguchi, H. Itoh and H. Kasai. (1997) Cigarette smoking induces an increase in oxidative DNA damage, 8-hydroxydeoxyguanosine, in a central site of the human lung, Carcinogenesis, 18, 1763-1766.

Athas, W. F., M. A. Hedayati, G. M. Matanoski, E. R. Farmer and L. Grossman. (1991) Development and field-test validation of an assay for DNA repair in circulating human lymphocytes, Cancer Res., 51, 5786-5793.

Aucklley, D.H., Crowell, R.E., Heaphy, E.R., Stidley, C.A., Lechner, J.F., Gilliland, F.D. and A., B.S. (2001) Reduced DNA-dependent prorein kinase is associated with lung cancer. Carcinogenesis, 22, 723-727.

Audebert, M., S. Chevillard, C. Levalois, G. Gyapay, A. Vieillefond, J. Klijanienko, P. Vielh, A. K. El Naggar, S. Oudard, S. Boiteux and J. P. Radicella. (2000) Alterations of the DNA repair gene OGG1 in human clear cell carcinomas of the kidney, Cancer Res., 60, 4740-4744.

Bessho, T., R. Roy, K. Yamamoto, H. Kasai, S. Nishimura, K. Tano and S. Mitra. (1993) Repair of 8-hydroxyguanine in DNA by mammalian N-methylpurine-DNA glycosylase, Proc. Natl. Acad. Sci. USA, 90, 8901-8904.

Bishop, J. M. (1995) Cancer: The rise of the genetic paradigm, Genes & Dev., 9, 1309-1315.

Bjoras, M., L. Luna, B. Johnsen, E. Hoff, T. Haug, T. Rognes and E. Seeberg. (1997) Opposite base-dependent reactions of a human base excision repair enzyme on DNA containing 7,8-dihydro-8-oxoguanine and abasic sites, EMBO J., 16, 6314-6322.

Chevillard, S., J. P. Radicella, C. Levalois, J. Lebeau, M. F. Poupon, S. Oudard, B. Dutrillaux and S. Boiteux. (1998) Mutations in OGG1, a gene involved in the repair of oxidative DNA damage, are found in human lung and kidney tumours, Oncogene, 16, 3083-3086.

Collins, A.R. (1999) Oxidative DNA damage, antioxidants, and cancer. BioEssays, 21, 238-246. Connor, F., D. Bertwistle, P. J. Mee, G. M. Ross, S. Swift, E. Grigorieva, V. L. J. Tybulewicz and A. Ashworth. (1997) Tumorigenesis and a DNA repair defect in mice with a truncating Brca2 mutation, Nature Genet, 17, 423-430.

Dianov, G., A. Price and T. Lindahl. (1992) Generation of single-nucleotide repair patches following excision of uracil residues from DNA, Mol. Cell Biol., 12, 1605-1612.

Echols, H. and M. F. Goodman. (1991) Fidelity mechanisms in DNA replication, Annu. Rev. Biochem., 60, 477-511.

Engelward, B. P., G. Weeda, M. D. Wyatt, J. L. Broekhof, J. de Wit, I. Donker, J. M. Allan, B. Gold, J. H. Hoeijmakers and L. D. Samson. (1997) Base excision repair deficient mice lacking the Aag alkyladenine DNA glycosylase, Proc. Natl. Acad. Sci. USA, 94, 13087-13092.

Fishel, R., M. K. Lescoe, M. R. Rao, N. G. Copeland, N. A. Jenkins, J. Garber, M. Kane and R. Kolodner. (1993) The human mutator gene homolog MSH2 and its association with hereditary nonpolyposis colon cancer, Cell, 75, 1027-38.

Fortini, P., B. Pascucci, E. Parlanti, R. W. Sobol, S. H. Wilson and E. Dogliotti. (1998) Different DNA polymerases are involved in the short- and long-patch base excision repair in mammalian cells, Biochemistry, 37, 3575-3580.

Friedberg, E. C., G. C. Walker and W. Siede (1995) DNA repair and mutagenesis, ASM Press, Washington, D. C.

Gajewski, E., G. Rao, Z. Nackerdien and M. Dizdaroglu. (1990) Modification of DNA bases in mammalian chromatin by radiation-generated free radicals, Biochemistry, 29, 7876-7882.

Gordis, L. (1996) Epidemiology, W. B. Saunders Co., Philadelphia.

Gowen, L. C., A. V. Avrutskaya, A. M. Latour, B. H. Koller and S. A. Leadon. (1998) BRCA1 required for transcription-coupled repair of oxidative DNA damage, Science, 281, 1009-1012.
Hanawalt, P. C. (1994) Transcription-coupled repair and human disease, Science, 266, 1957-1958.
Hang, B., B. Singer, G. P. Margison and R. H. Elder. (1997) Targeted deletion of alkylpurine-DNA -N-glycosylase in mice eliminates repair of 1, N6-ethenoadenine and hypoxanthine but not 3, N4-ethenocytosine or 8-oxoguanine, Proc. Natl. Acad. Sci. USA, 94, 12869-12874.
Hazra, T. K., T. Izumi, L. Maidt, R. A. Floyd and S. Mitra. (1998) The presence of two distinct 8-oxoguanine repair enzymes in human cells: their potential complementary roles in preventing mutation., Nucleic Acids Res., 26, 5116-5122.
Helzlsouer, K. J., E. L. Harris, R. Parshad, H. R. Perry, F. M. Proce and K. K. Sanford. (1996) DNA repair proficiency: Potential susceptibility factor for breast cancer, J. Natl. Cancer. Inst., 88, 754-755.
Hernandez-Boussard, T., P. Rodriguez-Tome, R. Montesano and P. Hainaut, P. (1999) IARC p53 mutation database: a relational database to compile and analyze p53 mutations in human tumors and cell lines. International Agency for Research on Cancer, Hum. Mutat., 14, 1-8.
Hollstein, M., Shomer, B., Greenblatt, M., Soussi, T., Hovig, E., Montesano, R. and Harris, C.C. (1996) Somatic point mutations in the p53 gene of human tumors and cell lines: updated compilation. Nucleic Acids Res., 24, 141-146.
Hutchinson, F. (1985) Chemical changes induced in DNA by ionizing radiation, Prog. Nucleic Acid Res. Mol. Biol., 32, 115-154.
Hyun, J. W., J. Y. Choi, H. H. Zeng, Y. S. Lee, H. S. Kim, S. H. Yoon and M. H. Chung. (2000) Leukemic cell line, KG-1 has a functional loss of hOGG1 enzyme due to a point mutation and 8-hydroxydeoxyguanosine can kill KG-1, Oncogene, 19, 4476-4479.
Ishida, T., Y. Hippo, Y. Nakahori, I. Matsushita, T. Kodama, S. Nishimura and H. Aburatani. (1999) Structure and chromosome location of human OGG1, Cytogenet. Cell Genet., 85, 232-236.
Jyothish, B., R. Ankathil, B. Chandini, B. Vinodkumar, G. Sunil Nayar, D. Dinesh Roy, J. Madhavan and M. Krishnan Nair. (1998) DNA repair proficiency: a potential marker for identification of high risk members in breast cancer families, Cancer Lett., 124, 9-13.
Kim, K., S. Biade and Y. Matsumoto. (1998) Involvement of flap endonuclease 1 in base excision DNA repair, J. Biol. Chem., 273, 8842-8848.
Kleinbaum, D. G. (1994) Logistic Regression, Springer-Verlag, New York.
Klungland, A., I. Rosewell, S. Hollenbach, E. Larsen, G. Daly, B. Epe, E. Seeberg, T. Lindahl and D. E. Barnes. (1999) Accumulation of premutagenic DNA lesions in mice defective in removal of oxidative base damage, Proc. Natl. Acad. Sci. USA, 96, 13300-13305.
Kyrtopoulos, S.A., Ampatzi, P., Davaris, P., Haritopoulos, N. and Golematis, B. (1990) Studies in gastric carcinogenesis. IV. O6-methyguanine and its repair in normal and atrophic biopsy specimen of human gastric mucosa. Correlation of O6-methylguanine-DNA alkyltransferase activities in gastric mucosa and circulating lymphocytes. Carcinogenesis, 11, 431-436.
Laval, F. (1994) Expression of the E. coli fpg gene in mammalian cells reduces mutagenicity of g rays, Nucleic Acids Res., 22, 4943-4946.
Le Page, F., E. E. Kwoh, A. Avrutskaya, A. Gentil, S. A. Leadon, A. Sarasin and P. K. Cooper. (2000) Transcription-coupled repair of 8-oxoguanine: requirement for XPG, TFIIH, and CSB and implications for Cockayne syndrome, Cell, 101, 159-171.
Le Page, F., V. Randrianarison, D. Marot, J. Cabannes, M. Perricaudet, J. Feunteun and A. Sarasin. (2000) BRCA1 and BRCA2 are necessary for the transcription-coupled repair of the oxidative 8-oxoguanine lesion in human cells, Cancer Res., 60, 5548-5552.
Leach, F. S., N. C. Nicolaides, N. Papadopoulos, B. Liu, J. Jen, R. Parsons, P. Peltomaki, P. Sistonen, L. A. Aaltonen, M. Nystromlahti, X. Y. Guan, J. Zhang, P. S. Meltzer, J. W. Yu, F. T. Kao, D. J. Chen, K. M. Cerosaletti, R. E. K. Fournier, S. Todd, T. Lewis, R. J. Leach, S. L. Naylor, J. Weissenbach, J. P. Mecklin, H. Jarvinen, G. M. Petersen, S. R. Hamilton, J. Green, J. Jass, P. Watson, H. T. Lynch, J. M. Trent, J. M. Trent, A. de la Chapelle, K. W. Kinzler and B. Vogelstein. (1993) Mutations of a mutS homolog in hereditary nonpolyposis colorectal cancer, Cell, 75, 1215-1225.
Leanderson, P. and C. Tagesson. (1992) Cigarette smoke-induced DNA damage in cultured human lung cells: role of hydroxyl radicals and endonuclease activation, Che. Biol. Interact., 81, 197-208.
Lindahl, T. (1993) Instability and decay of the primary structure of DNA. Nature, 362, 709-715.
Lippman, S.M. and Spitz, M.R. (2001) Lung cancer chemoprevention: An integrated approach. J. Clin. Oncol., 19, 74s-82s.
Livneh, Z., O. Cohen-Fix, R. Skaliter and T. Elizur. (1993) Replication of damaged DNA and the molecular mechanism of ultraviolet light mutagenesis, CRC Crit. Rev. Biochem. Mol. Biol., 28, 465-513.
Lu, R., H. M. Nash and G. L. Verdine. (1997) A mammalian DNA repair enzyme that excises oxidatively damaged guanines maps to a locus frequently lost in lung cancer., Curr. Biol., 7, 397-407.

Maki, H. and M. Sekiguchi. (1992) MutT protein specifically hydrolyses a potent mutagenic substrate for DNA synthesis, Nature (London), 355, 273-275.
Mattson, M. E., Pollack, E.S. & Cullen, J.W. (1987) What are the odds that smoking will kill you? Am. J. Public Health 77, 425-431.
Minna, J.D., Roth, J.A. and Gazdar, A.F. (2002) Focus on lung cancer. Cancer Cell, 1, 49-52.
Minowa, O., T. Arai, M. Hirano, Y. Monden, S. Nakai, M. Fukuda, M. Itoh, H. Takano, Y. Hippou, H. Aburatani, K. Masumura, T. Nohmi, S. Nishimura and T. Noda. (2000) Mmh/Ogg1 gene inactivation results in accumulation of 8-hydroxyguanine in mice, Proc. Natl. Acad. Sci. USA, 97, 4156-4161.
Modrich, P. (1994) Mismatch repair, genetic stability, and cancer, Science, 266, 1959-1960.
Monden, Y., T. Arai, M. Asano, E. Ohtsuka, H. Aburatani and S. Nishimura. (1999) Human MMH (OGG1) Type 1a Protein Is a Major Enzyme for Repair of 8-Hydroxyguanine Lesions in Human Cells, Biochem. Biophys. Res. Commun., 258, 605-610.
Parshad, R., F. M. Price, V. A. Bohr, K. H. Cowans, J. A. Zujewski and K. K. Sanford. (1996) Deficient DNA repair capacity, a predisposing factor in breast cancer, Brit. J. Cancer, 74, 1-5.
Parsons, R., G. M. Li, M. J. Longley, W. H. Fang, N. Papadopoulos, J. Jen, 1. C. A. de, K. W. Kinzler, B. Vogelstein and P. Modrich. (1993) Hypermutability and mismatch repair deficiency in RER+ tumor cells, Cell, 75, 1227-36.
Patel, K. J., V. P. C. C. Yu, H. Lee, A. Corcoran, F. C. Thistlethwaite, M. J. Evans, W. H. Colledge, L. S. Friedman, B. A. J. Ponder and A. R. Venkitaraman. (1998) Involvement of Brca2 in DNA Repair, Molecular Cell, 1, 347-357.
Patel, R. K., A. H. Trivedi, D. C. Arora, J. M. Bhatavdekar and D. D. Patel. (1997) DNA repair proficiency in breast cancer patients and their first-degree relatives, Int. J. Cancer, 73, 20-24.
Pavlov, Y. I., D. T. Minnick, S. Izuta and T. A. Kunkel. (1994) DNA replication fidelity with 8-oxodeoxyguanosine triphosphate, Biochemistry, 33, 4695-4701.
Radicella, J. P., C. Dherin, C. Desmaze, M. S. Fox and S. Boiteux. (1997) Cloning and characterization of hOGG1, a human homolog of the OGG1 gene of Saccharomyces cerevisiae, Proc. Natl. Acad. Sci. USA, 94, 8010-8015.
Reardon, J. T., T. Bessho, H. C. Kung, P. H. Bolton and A. Sancar. (1997) In vitro repair of oxidative DNA damage by human nucleotide excision repair system: possible explanation for neurodegeneration in xeroderma pigmentosum patients, Proc. Natl. Acad. Sci. USA, 94, 9463-9468.
Roldan-Arjona, T., Y. F. Wei, K. C. Carter, A. Klungland, C. Anselmino, R. P. Wang, M. Augustus and T. Lindahl. (1997) Molecular cloning and functional expression of a human cDNA encoding the antimutator enzyme 8-hydroxyguanine DNA glycosylase, Proc. Natl. Acad. Sci. USA, 94, 8016-8020.
Rosenquist, T. A., D. O. Zharkov and A. P. Grollman. (1997) Cloning and characterization of a mammalian 8-oxoguanine DNA glycosylase, Proc. Natl. Acad. Sci. USA, 94, 7429-7434.
Runger, T. M., B. Epe and K. Moller. (1995) Repair of ultraviolet B and singlet oxygen-induced DNA damage in xeroderma pigmentosum cells, J. Invest. Dermatol., 104, 68-73.
Sagher, D., T. Karrison, J. L. Schwartz, R. Larson, P. Meier and B. Strauss. (1988) Low O6-alkylguanine DNA alkyltransferase activity in the peripheral blood lymphocytes of patients with therapy-related acute nonlymphocytic leukemia, Cancer Res., 48, 3084-3089.
Sancar, A. (1994) Mechanisms of DNA repair, Science, 266, 1954-1956.
Savitsky, K., A. Bar-Shira, S. Gilad, G. Rotman, Y. Ziv, L. Vanagaite, D. A. Tagle, S. Smith, T. Uziel, S. Sfez, M. Ashkenazi, I. Pecker, M. Frydman, R. Harnik, S. R. Patanjali, A. Simmons, G. A. Clines, A. Sartiel, R. A. Gatti, L. Chessa, O. Sanal, M. Lavin, N. G. J. Jaspers, A. M. R. Taylor, C. F. Arlett, T. Miki, S. M. Weissman, M. Lovett, F. S. Collins and Y. Shiloh. (1995) A single Ataxia Telangiectasia gene with a product similar to PI-3 kinase, Science, 268, 1749-1753.
Scully, R., J. Chen, A. Plug, Y. Xiao, D. Weaver, J. Feunteun, T. Ashley and D. M. Livingston. (1997) Association of BRCA1 with Rad51 in Mitotic and Meiotic cells, Cell, 88, 265-275.
Sharan, S. K., M. Morimatsu, U. Albrecht, D. S. Lim, E. Regel, C. Dinh, A. Sands, G. Eichele, P. Hasty and A. Bradley. (1997) Embryonic lethality and radiation hypersensitivity mediated by Rad51 in mice lacking Brca2, Nature, 386, 804-810.
Shibutani, S., M. Takeshita and A. P. Grollman. (1991) Insertion of specific bases during DNA synthesis past the oxidation-damaged base 8-oxodG, Nature, 349, 431-434.
Shinmura, K., T. Kohno, H. Kasai, K. Koda, H. Sugimura and J. Yokota. (1998) Infrequent mutations of the hOGG1 gene, that is involved in the excision of 8-hydroxyguanine in damaged DNA, in human gastric cancer, Jpn. J. Cancer Res., 89, 825-828.
Singhal, R. K., R. Prasad and S. H. Wilson. (1995) DNA polymerase beta conducts the gap-filling step in uracil-initiated base excision repair in a bovine testis nuclear extract, J Biol Chem, 270, 949-957.
Srivastava, S., Z. Zou, K. Pirollo, W. Blattner and E. H. Chang. (1990) Germ-line transmission of a mutated p53 in a cancer-prone family with Li-Fraumeni syndrome, Nature (London), 348, 747-749.
Strauss, B. S. (1985) Translesion DNA synthesis: polymerase response to altered nucleotide, Cancer Surv., 4, 493-516.
Vandenbroucke, J. P., T. Koster, E. Briet, P. H. Reitsma, R. M. Bertina and F. R. Rosendaal. (1994) Increased risk of venous thrombosis in oral-contraceptive users who are carriers of factor V Leiden mutation, Lancet, 344, 1453-1457.
Vogelstein, B. and K. W. Kinzler. (1993) The multistep nature of cancer, Trends Genet., 9, 138-141.
Weeda, G., J. H. J. Hoeijmakers and D. Bootsma. (1993) Genes controlling nucleotide excision repair in eukaryotic cells, BioEssays, 15, 249-258.
Wei, Q., L. Cheng, W. K. Hong and M. R. Spitz. (1996) Reduced DNA repair capacity in lung cancer patients, Cancer Res., 56, 4103-4107.
Wei, Q., G. M. Matanoski, E. R. Farmer, M. A. Hedayati and L. Grossman. (1993) DNA repair and aging in basal cell carcinoma: a molecular epidemiology study, Proc. Natl. Acad. Sci. U S A, 90, 1614-1618.
Wei, Q., G. M. Matanoski, E. R. Farmer, M. A. Hedayati and L. Grossman. (1994) DNA repair and susceptibility to basal cell carcinoma: a case-control study, Am. J. Epidemiol., 140, 598-607.
Weinberg, R. A. (1989) Oncogenes, antioncogenes, and the molecular bases of multistep carcinogenesis, Cancer Res., 49, 3713-3721.
Wikman, H., A. Risch, F. Klimek, P. Schmezer, B. Spiegelhalder, H. Dienemann, K. Kayser, V. Schulz, P. Drings and H. Bartsch. (2000) OGG1 polymorphism and loss of heterozygosity (LOH): significance for lung cancer susceptibility in a caucasian population, Int. J. Cancer, 88, 932-937.
Wood, M. L., M. Dizdaroglu, E. Gajewski and J. M. Essigmann. (1990) Mechanistic studies of ionizing radiation and oxidative mutagenesis: Genetic effects of a single 8-hydroxyguanine (7-hydro-8-oxo-guanine) residue inserted at a unique site in a viral genome, Biochemistry, 29, 7024-7032.

## Claims

1. A method of determining a risk of a subject to develop cancer, the method comprising determining a level of catalytic activity of a DNA repair enzyme in a biological sample of the subject, wherein said DNA repair enzyme is selected from the group consisting of 8-oxoguanine DNA glycosylase (OGG1), AP endonuclease1 (APE1), methylpurine DNA glycosylase (MPG), uracil DNA glycosylase1 (UNG1), uracil DNA glycosylase2 (UNG2), SMUG1, MBD4, mismatch-specific thymine/uracil glycosylase (TDG), enodonuclease III (NTH1), adenine-specific mismatch DNA glycosylase (MYH), 8-oxo-GTPase/8-oxodGTPase (MTH1), dUTPase (DUT), AP endonuclease2 (APE2), deoxyribose phosphate lyase (POLB) and wherein a level of said activity below a predetermined value is indicative of an increased risk of the subject to develop said cancer.

2. A method of determining a risk of a subject to develop colorectal cancer, the method comprising determining a level of catalytic activity of 8-oxoguanine DNA glycosylase in a sample of peripheral blood lymphocytes of the subject and, according to said level, determining the risk of the subject to develop colorectal cancer, wherein a level of said activity below a predetermined value is indicative of an increased risk of said subject to develop colorectal cancer.

3. A method of determining a risk of a subject to develop head and neck cancer, the method comprising determining a level of catalytic activity of 8-oxoguanine DNA glycosylase in a sample of peripheral blood lymphocytes of the subject and, according to said level, determining the risk of the subject to develop head and neck cancer, wherein a level of said activity below a predetermined value is indicative of an increased risk of said subject to develop head and neck cancer.

4. The method of claims 2 or 3, wherein said level of catalytic activity is determined using a double-stranded DNA substrate comprising the complementary oligonucleotides having a polynucleotide sequence as set forth in SEQ ID NOs: 1 and 2.

5. A method of determining a risk of a subject to develop lung cancer or colorectal cancer, the method comprising determining a level of catalytic activity of APE1 in a biological sample of the subject and, according to said level, determining the risk of the subject to develop lung cancer or colorectal cancer, wherein a level of said activity below a predetermined value is indicative of an increased risk of said subject to develop said cancer.

6. A method of determining a risk of a subject to develop lung cancer or colorectal cancer, the method comprising determining a level of catalytic activity of MPG in a biological sample of the subject and, according to said level, determining the risk of the subject to develop lung cancer or colorectal cancer, wherein a level of said activity below a predetermined value is indicative of an increased risk of said subject to develop said cancer.

7. The method of claim 1, wherein said cancer is selected from the group consisting of lung cancer, colorectal cancer and head and neck cancer.

8. The method of any of claims 1 to 7, wherein the risk is expressed as a fold risk increase as is compared to a normal, apparently healthy, population, or a reference control group.

9. The method of claim 8, wherein the risk is expressed in enzyme specific activity units.

10. The method of claim 8, wherein the risk is expressed as a magnitude of a scale.

11. The method of any of claims 1 to 10, wherein determining the level of catalytic activity of the DNA repair enzyme is effected using a DNA substrate having at least one lesion therein.

12. The method of any of claims 1 to 11, wherein the subject is known to be, or is about to be, exposed to environmental conditions associated with increased risk of developing said cancer.

**13.** The method of claim 12, wherein said environmental conditions are selected from the group consisting of smoking and occupational exposure to smoke or ionizing radiation.

**14.** A method of predicting the efficacy of a mutagenic cancer treatment in a subject, the method comprising determining a level of catalytic activity of a DNA repair enzyme in a biological sample of the subject, and, according to said level, predicting the efficacy of the mutagenic anti-cancer treatment in the subject, wherein a level of said activity below a predetermined value is indicative of an increased efficacy of said mutagenic cancer treatment, wherein said DNA repair enzyme is selected from the group consisting of 8-oxoguanine DNA glycosylase (OGG1), AP endonuclease1 (APE1), methylpurine DNA glycosylase (MPG), uracil DNA glycosylasel (UNG1), uracil DNA glycosylase2 (UNG2), SMUG1, MBD4, mismatch-specific thymine/uracil glycosylase (TDG), enodonuclease III (NTH1), adenine-specific mismatch DNA glycosylase (MYH), 8-oxo-GTPase/8-oxodGTPase (MTH1), dUTPase (DUT), AP endonuclease2 (APE2), deoxyribose phosphate lyase (POLB).

**15.** The method of claim 14, wherein said mutagenic anti-cancer treatment is selected from the group of chemotherapy and radiotherapy.

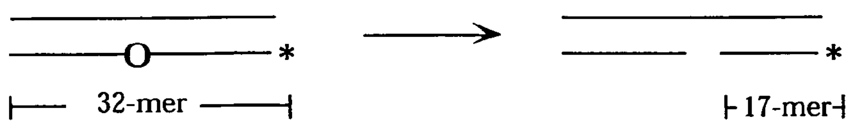


Fig. 1a

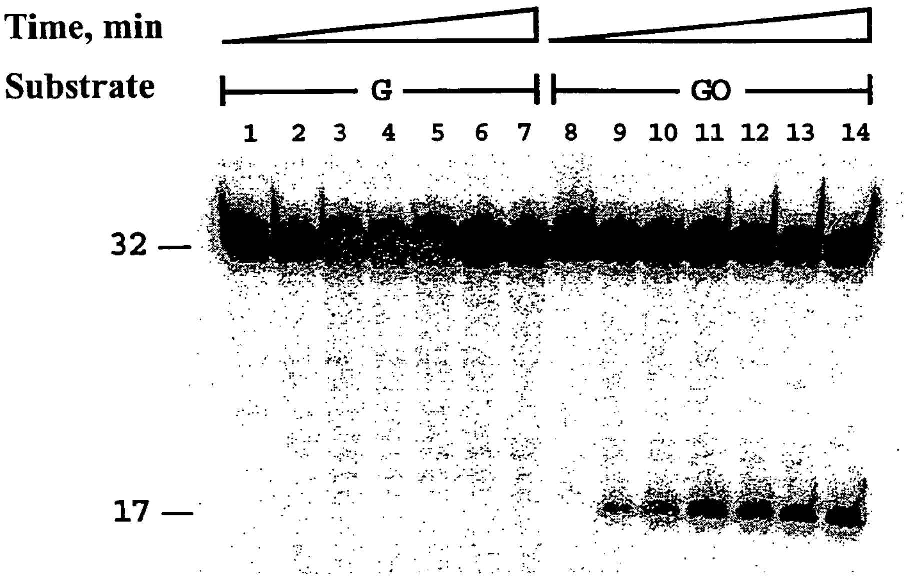


Fig. 1b

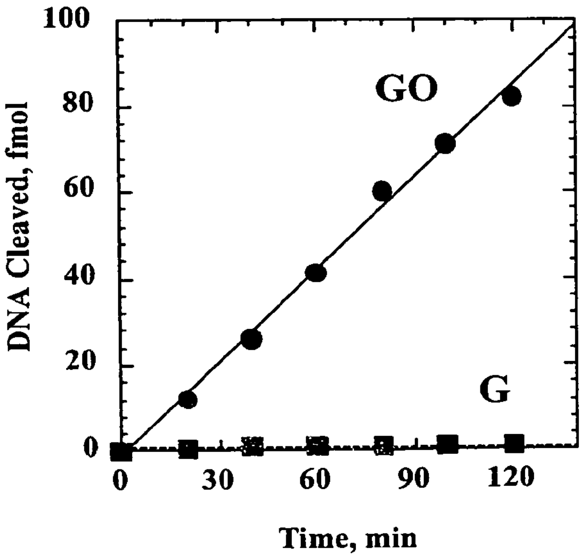


Fig. 1c

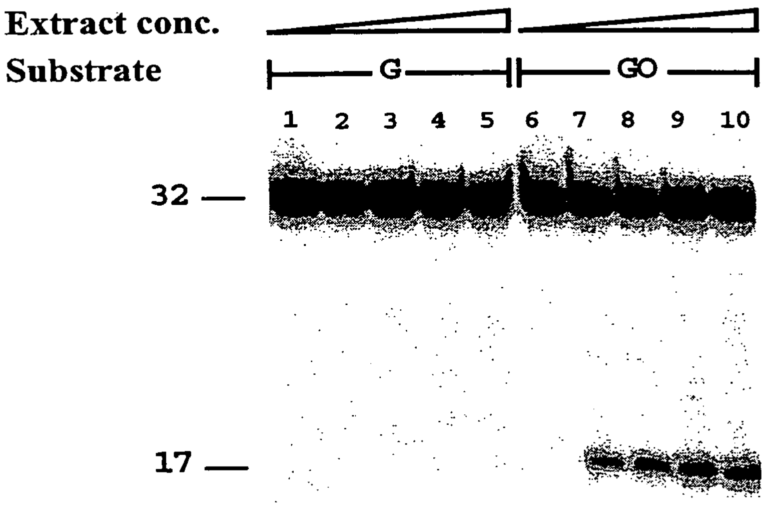
Extract conc.
Substrate
G
GO
1 2 3 4 5 6 7 8 9 10
32
17

Fig. 2a

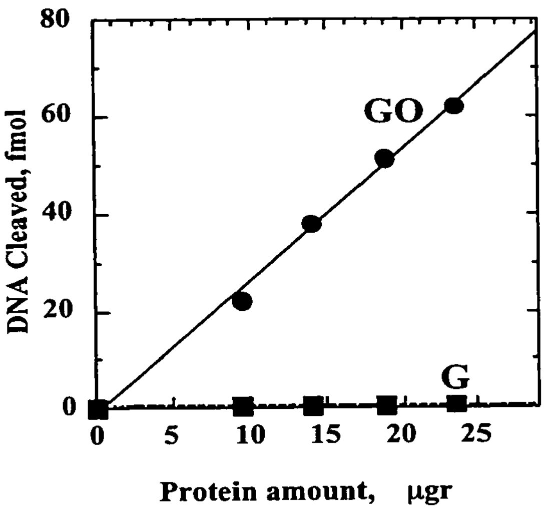
80
60
40
20
0
DNA Cleaved, fmol
GO
G
0 5 10 15 20 25
Protein amount, μgr

Fig. 2b

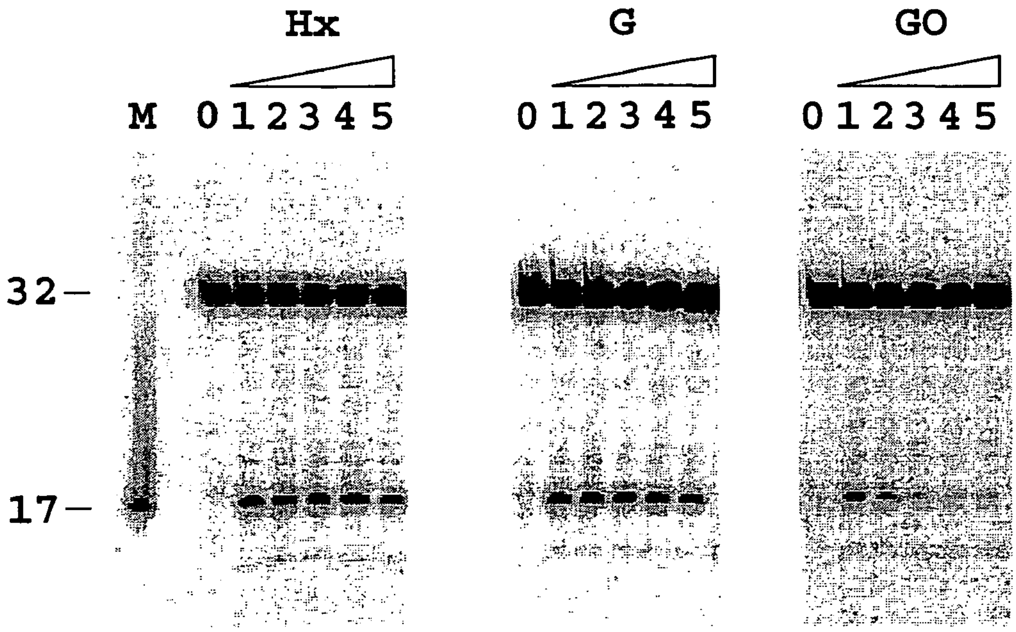
Hx
G
GO
M
0 1 2 3 4 5
0 1 2 3 4 5
0 1 2 3 4 5
32—
17—

Fig. 3a

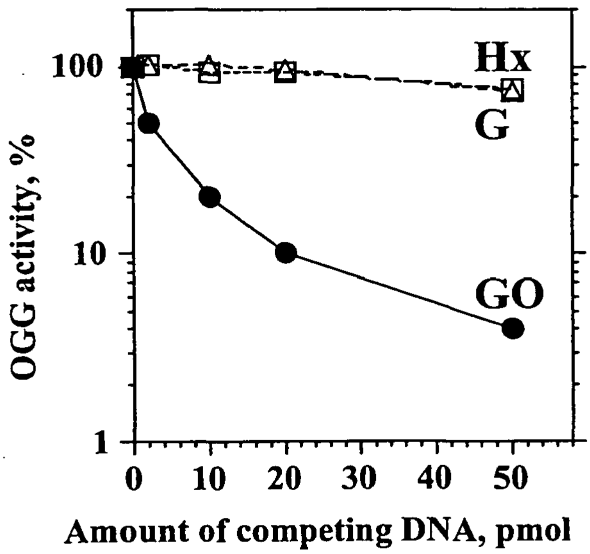
100
10
1
Hx
G
GO
OGG activity, %
0
10
20
30
40
50
Amount of competing DNA, pmol

Fig. 3b

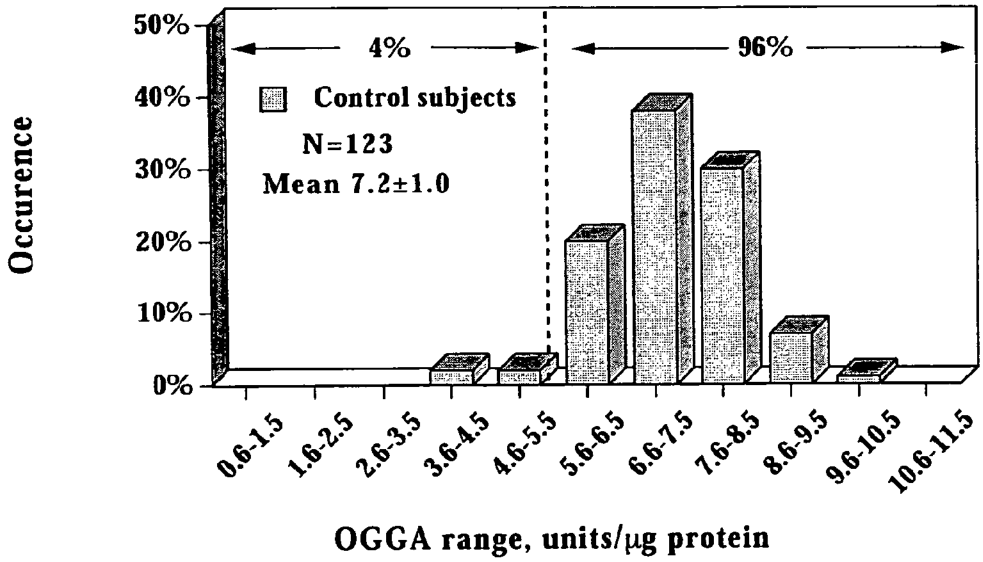
Occurence
50%
40%
30%
20%
10%
0%
4%
96%
Control subjects
N=123
Mean 7.2±1.0
0.6-1.5
1.6-2.5
2.6-3.5
3.6-4.5
4.6-5.5
5.6-6.5
6.6-7.5
7.6-8.5
8.6-9.5
9.6-10.5
10.6-11.5
OGGA range, units/μg protein

Fig. 4

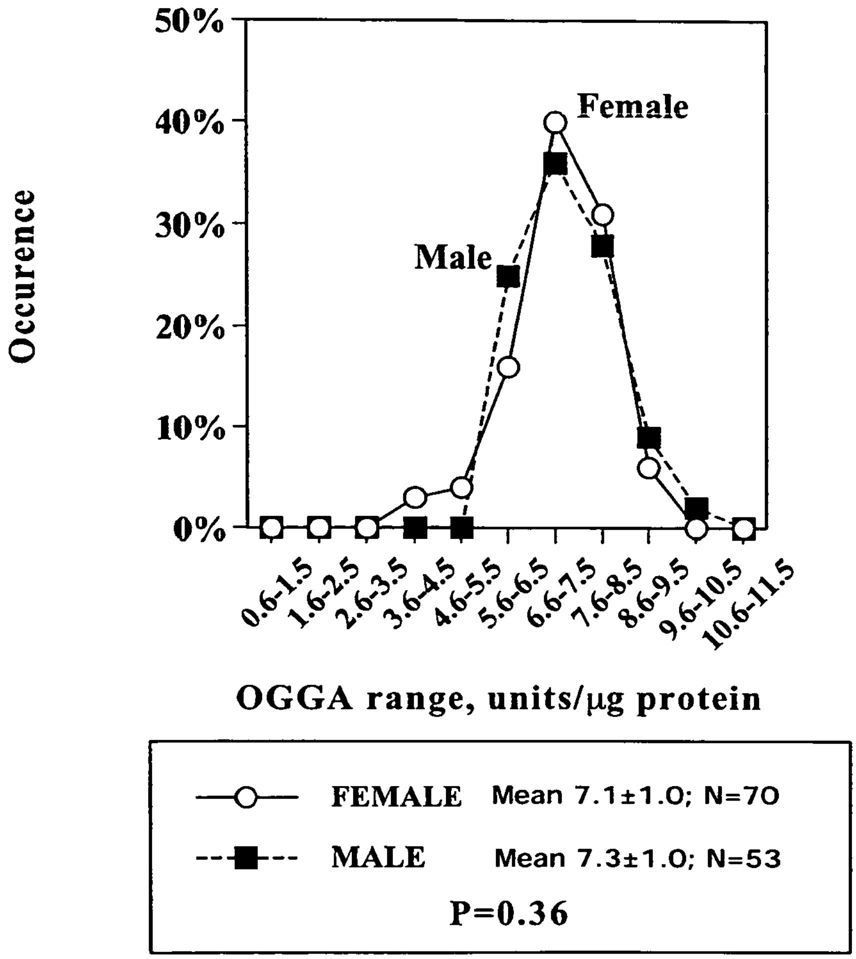
50%
40%
30%
20%
10%
0%
Occurence
Female
Male
0.6-1.5
1.6-2.5
2.6-3.5
3.6-4.5
4.6-5.5
5.6-6.5
6.6-7.5
7.6-8.5
8.6-9.5
9.6-10.5
10.6-11.5
OGGA range, units/μg protein
FEMALE Mean 7.1±1.0; N=70
MALE Mean 7.3±1.0; N=53
P=0.36

Fig. 5

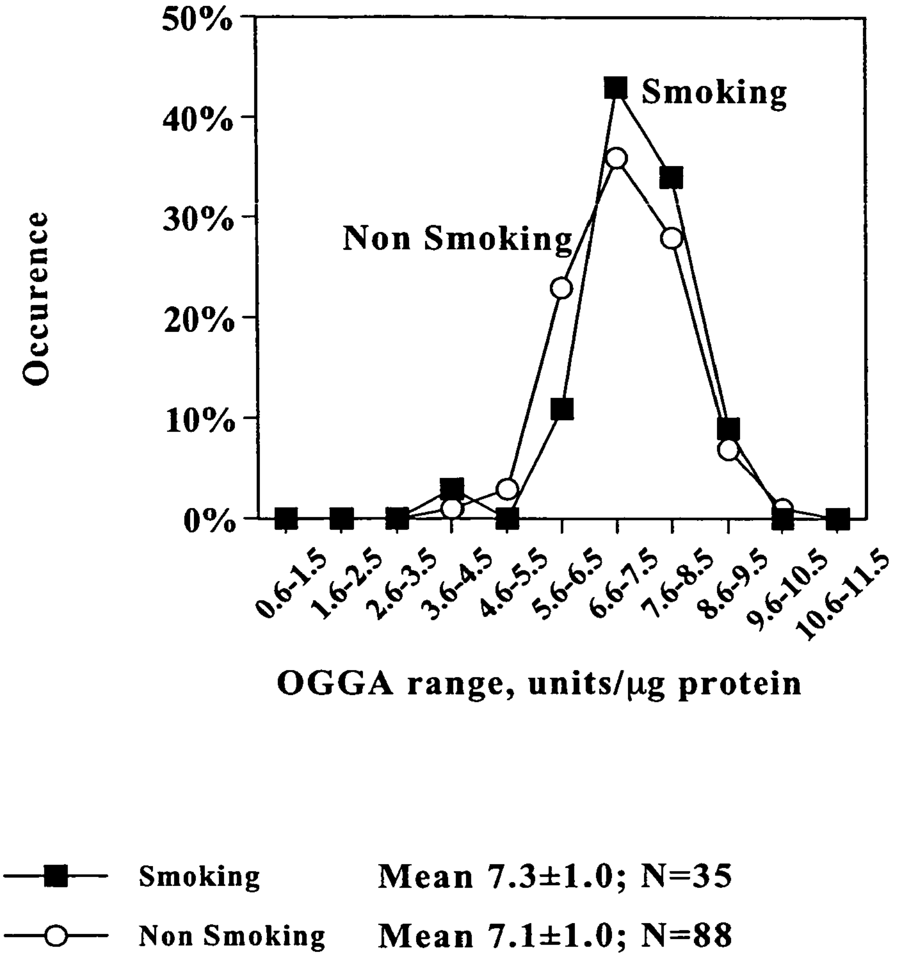


Mean 7.3±1.0; N=35

Mean 7.1±1.0; N=88

P=0.46

Fig. 6

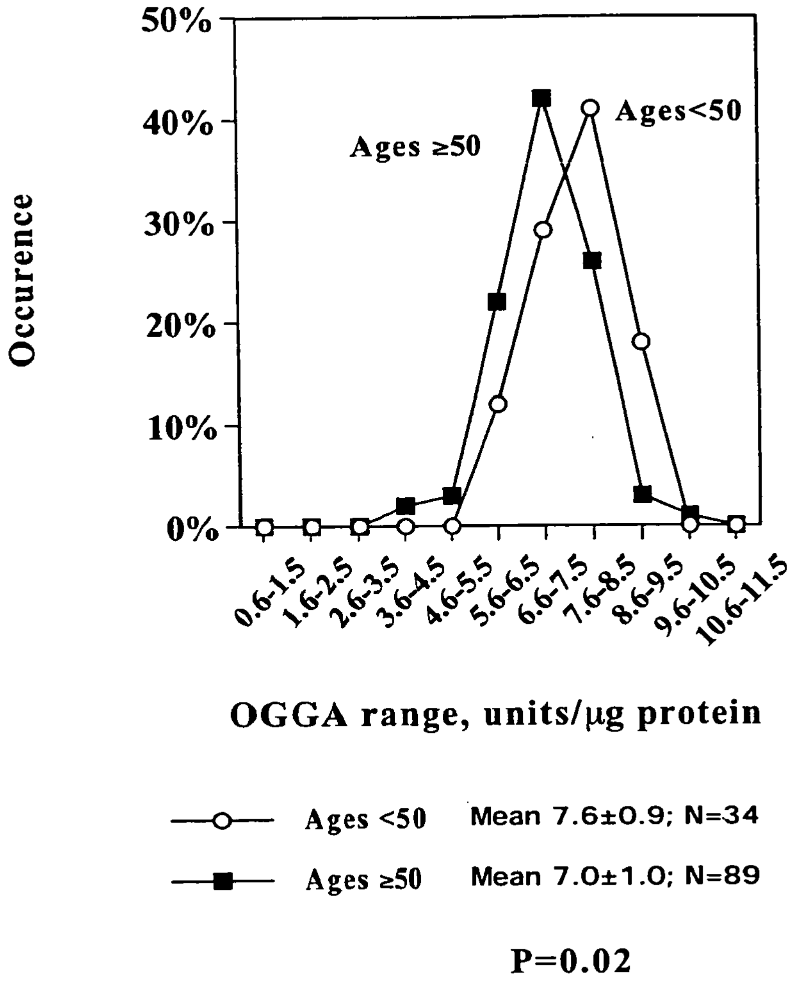
50%
40%
30%
20%
10%
0%
Occurence
Ages ≥50
Ages<50
0.6-1.5
1.6-2.5
2.6-3.5
3.6-4.5
4.6-5.5
5.6-6.5
6.6-7.5
7.6-8.5
8.6-9.5
9.6-10.5
10.6-11.5
OGGA range, units/μg protein
Ages <50 Mean 7.6±0.9; N=34
Ages ≥50 Mean 7.0±1.0; N=89
P=0.02

Fig. 7

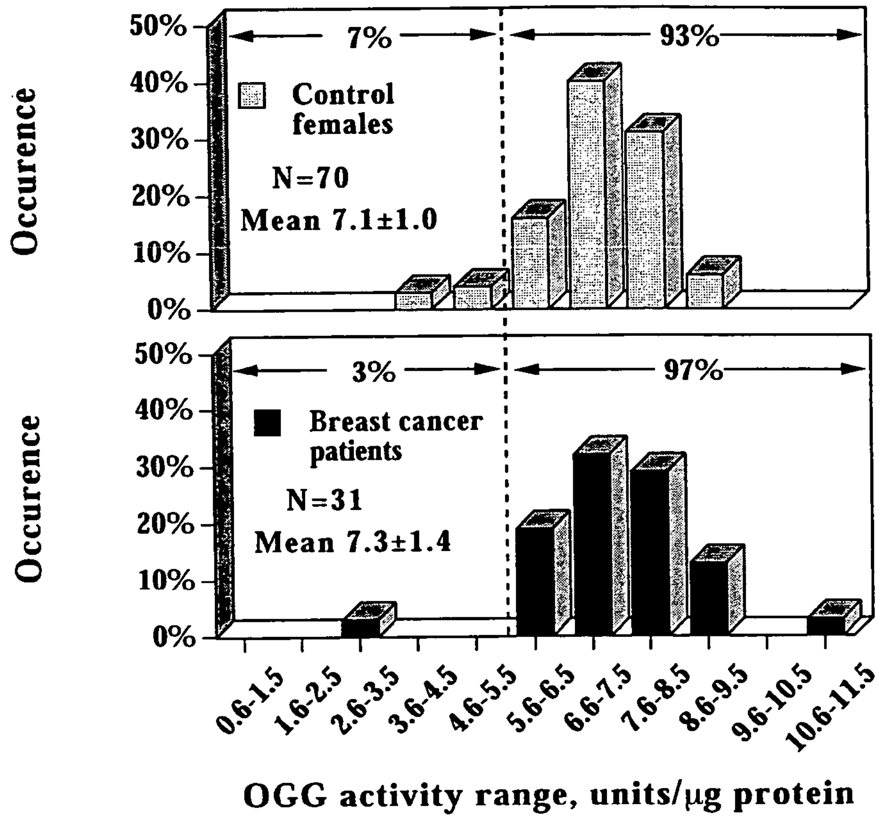


Fig. 8a

Fig. 8b

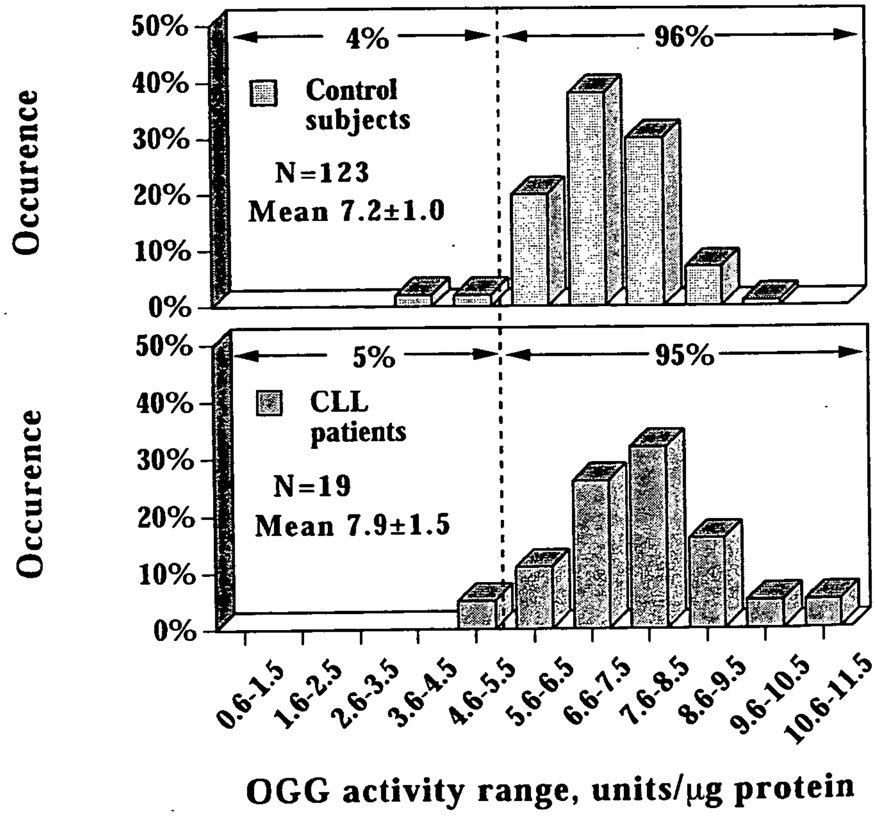


Fig. 8c

Fig. 8d

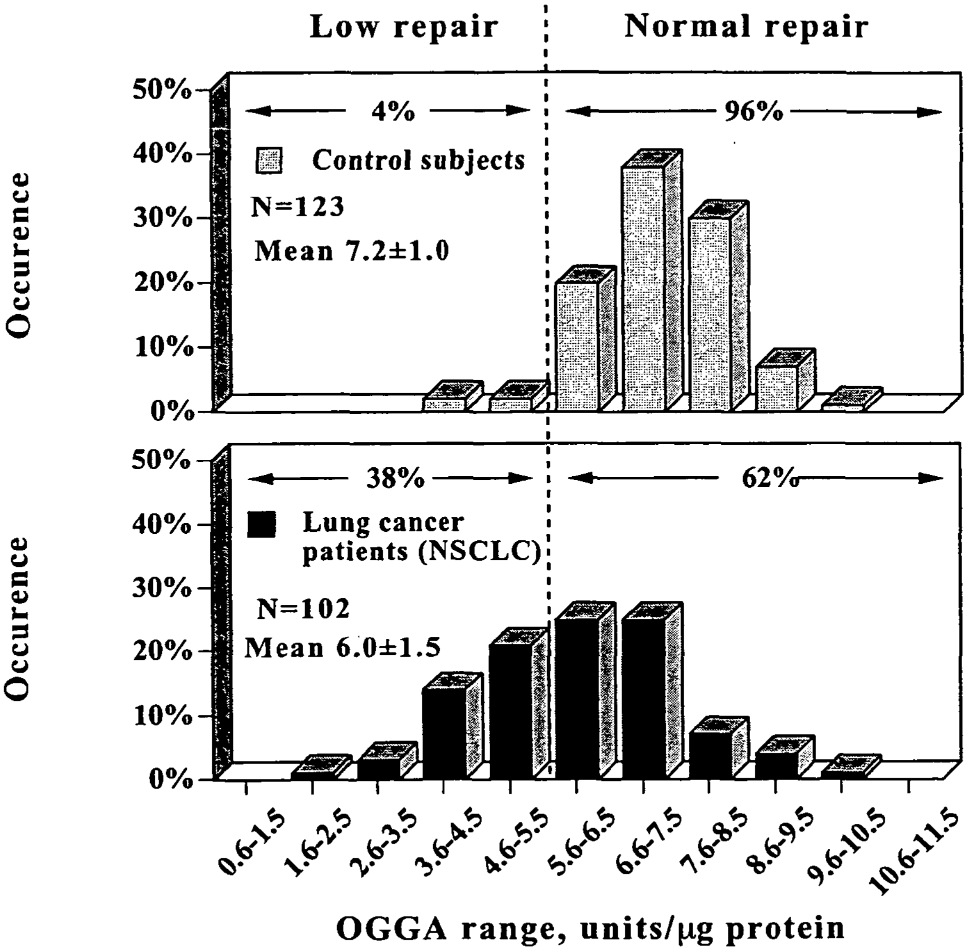


Fig. 9a

Fig. 9b

P=0.0001

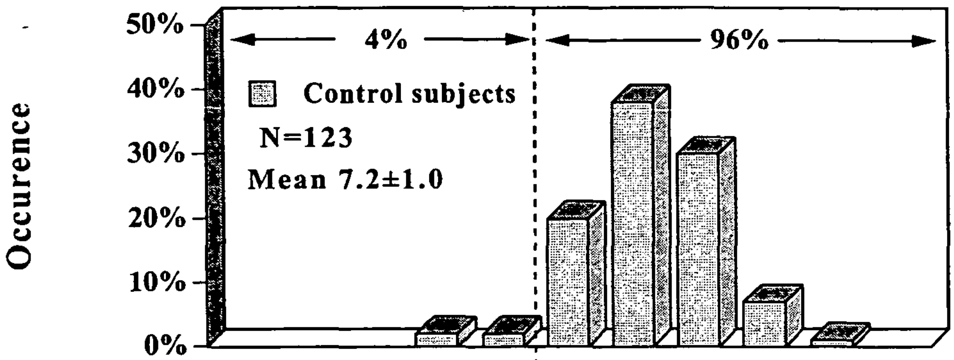


Fig. 10a

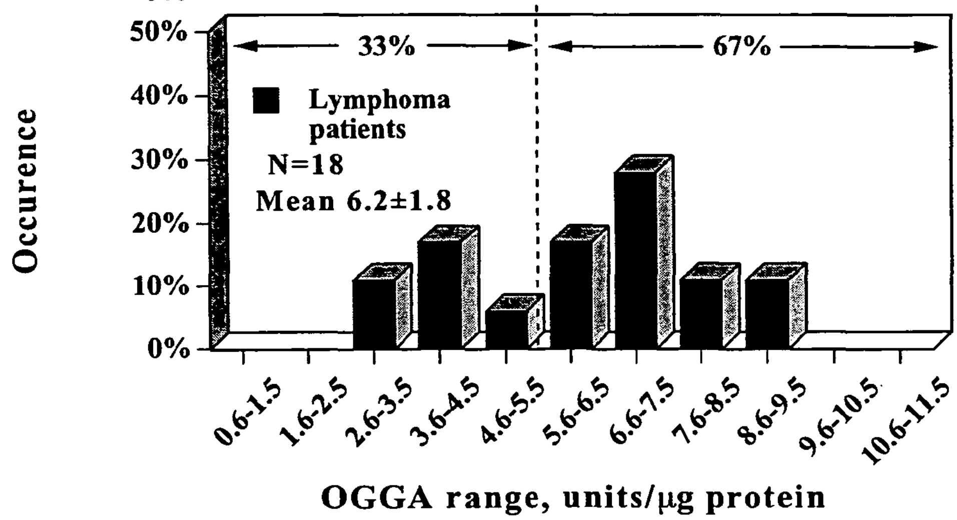


Fig. 10b

P=0.0001

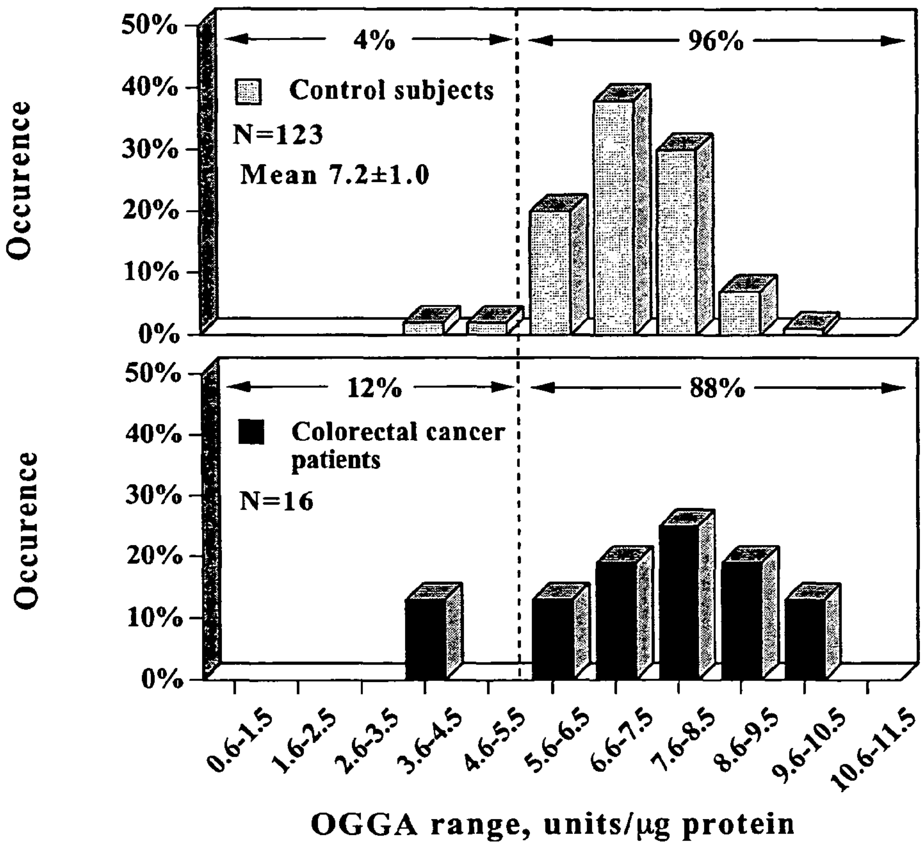


Fig. 11a

Fig. 11b

Fig. 12a

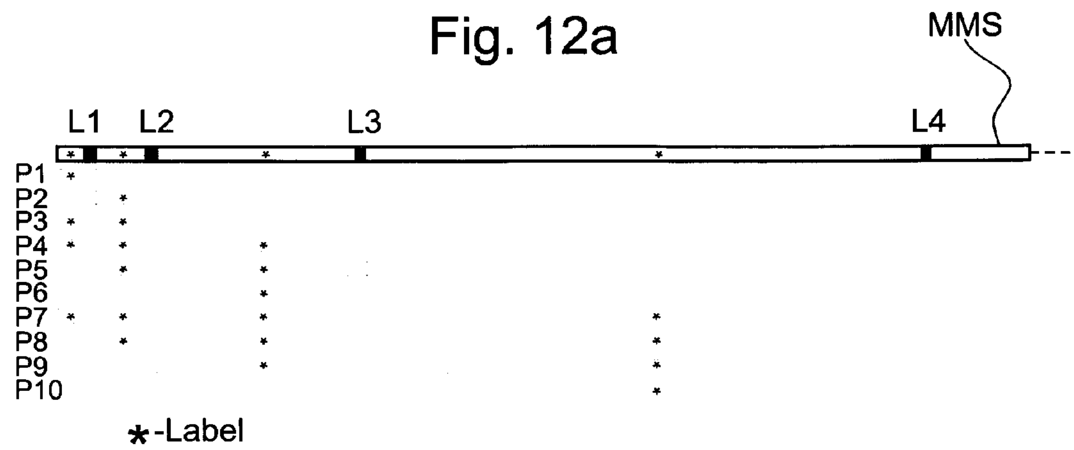


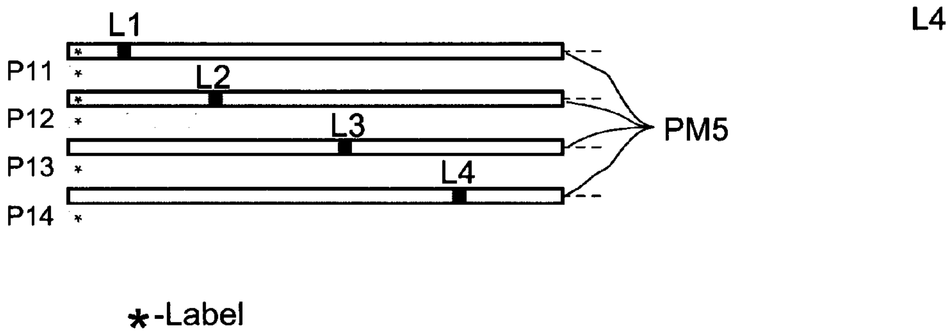


Fig. 12b

Europäisches Patentamt
European Patent Office
Office européen des brevets

# EUROPEAN SEARCH REPORT

Application Number
EP 10 18 1096

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 99/04626 A (FOX CHASE CANCER CENTER) 4 February 1999 (1999-02-04)<br>* claims 19-21, 27, 28 *<br>----- | 1-15 | INV.<br>C12Q1/68<br>C12P19/34<br>C07H21/02<br>C07H21/04 |
| A | US 5 482 833 A (PERO RONALD W [US] ET AL) 9 January 1996 (1996-01-09)<br>* the whole document *<br>----- | 1-15 | |
| A | SHINMURA K ET AL: "Infrequent mutations of the hOGGI gene, that is involved in the excision of 8-hydroxyguanine in damaged DNA, in human gastric cancer", JAPANESE JOURNAL OF CANCER RESEARCH, JAPANESE CANCER ASSOCIATION, TOKYO, JP, vol. 89, 1 August 1998 (1998-08-01), pages 825-828, XP002964057, ISSN: 0910-5050<br>* the whole document *<br>----- | 1-15 | |
| X | AUDEBERT, M. ET AL: "Effect of single mutations in the OGG1 gene found in human tumors on the substrate specificity of the Ogg1 protein", NUCLEIC ACIDS RESEARCH, vol. 28, no. 14, 2000, pages 2672-2678, XP002619409, | 1 | TECHNICAL FIELDS SEARCHED (IPC)<br>G01N<br>C12Q |
| A | * the whole document *<br>----- | 2-15 | |

The present search report has been drawn up for all claims

1

| Place of search | Date of completion of the search | Examiner |
|---|---|---|
| Munich | 31 January 2011 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 18 1096

This annex lists the patent family members relating to the patent documents cited in the above-mentioned European search report.
The members are as contained in the European Patent Office EDP file on
The European Patent Office is in no way liable for these particulars which are merely given for the purpose of information.

31-01-2011

| Patent document cited in search report | | Publication date | Patent family member(s) | | Publication date |
|---|---|---|---|---|---|
| WO 9904626 | A | 04-02-1999 | AU | 744157 B2 | 14-02-2002 |
| | | | AU | 8761398 A | 16-02-1999 |
| | | | CA | 2298980 A1 | 04-02-1999 |
| | | | EP | 1009230 A1 | 21-06-2000 |
| US 5482833 | A | 09-01-1996 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3791932 A
- US 3839153 A
- US 3850752 A
- US 3850578 A
- US 3853987 A
- US 3867517 A
- US 3879262 A
- US 3901654 A
- US 3935074 A
- US 3984533 A
- US 3996345 A
- US 4034074 A
- US 4098876 A
- US 4879219 A
- US 5011771 A
- US 5281521 A


### Non-patent literature cited in the description


- **SLUPPHAUG, G. ; EFTEDAL, I. ; KAVLI, B. ; BHARATI, S. ; HELLE, N. M. ; HAUG, T. ; LEVINE, D. W. ; KROKAN, H. E.** Properties of a recombinant human uracil-DNA glycosylase from the UNG gene and evidence that UNG encodes the major uracil-DNA glycosylase. *Biochemistry,* 1995, vol. 34, 128-138
- **MONDEN, Y. ; ARAI, T. ; ASANO, M. ; OHTSUKA, E. ; ABURATANI, H. ; NISHIMURA, S.** Human MMH (OGG1) type 1a protein is a major enzyme for repair of 8-hydroxyguanine lesions in human cells. *Biochem. Biophys. Res. Comm.,* 1999, vol. 258, 605-610
- **PARKER, A. ; GU, Y. ; LU, A. -L.** Purification and characterization of a mammalian homolog of Escherichia coli MutY mismatch repair protein from calf liver mitochondria. *Nucleic Acids Res.,* 2000, vol. 28, 3206-3215
- **KANG, D. ; NISHIDA, J. ; IYAMA, A. ; NAKABEPPU, Y. ; FURUICHI, M. ; FUJIWARA, T. ; SEKIGUCHI, M. ; TAKESHIGE, K.** Intracellular localization of 8-oxo-dGTPase in human cells, with special reference to the role of the enzyme in mitochondria. *J. Biol. Chem.,* 1995, vol. 270, 14659-14665
- **LADNER, R. D. ; CARADONNA, S. J.** The human dUTPase gene encodes both nuclear and mitochondrial isoforms. *J. Biol. Chem.,* 1997, vol. 272, 19072-19080
- **SRIVASTAVA, D. K. ; RAWSON, T. Y. ; SHOWALTER, S. D. ; WILSON, S. H.** Phorbol ester abrogates up-regulation of DNA polymerase β by DNA-alkylating agents in Chinese hamster ovary cells. *J. Biol. Chem.,* 1995, vol. 270, 16402-16408
- **SRIVASTAVA, D. K. ; HUSAIN, I. ; ARTEAGA, C. L. ; WILSON, S. H.** DNA polymerase β expression differences in selected human tumor cell lines. *Carcinogenesis,* 1999, vol. 20, 1049-1054
- Current Protocols in Immunology. 1994, vol. I-III
- Basic and Clinical Immunology. Appleton & Lange, 1994
- Selected Methods in Cellular Immunology. W. H. Freeman and Co, 1980
- Immobilized Cells and Enzymes. IRL Press, 1986
- Methods in Enzymology. Academic Press, vol. 1-317
- **MARSHAK et al.** Strategies for Protein Purification and Characterization - A Laboratory Course Manual. CSHL Press, 1996
- **SAMBROOK et al.** Molecular Cloning: A laboratory Manual. 1989
- Current Protocols in Molecular Biology. 1994, vol. I-III
- **AUSUBEL et al.** Current Protocols in Molecular Biology. John Wiley and Sons, 1989
- **PERBAL.** A Practical Guide to Molecular Cloning. John Wiley & Sons, 1988
- Recombinant DNA. **WATSON et al.** Scientific American Books
- Genome Analysis: A Laboratory Manual Series. Cold Spring Harbor Laboratory Press, 1998, vol. 1-4
- Oligonucleotide Synthesis. 1984
- Nucleic Acid Hybridization. 1985
- Transcription and Translation. 1984
- PCR Protocols: A Guide To Methods And Applications. Academic Press, 1990
- **MO, J.-Y. ; MAKI, H. ; SEKIGUCHI, M.** *Proc. Natl. Acad. Sci. USA,* 1992, vol. 89, 11021-11025

- **WILSON III, D. M. ; TAKESHITA, M. ; GROLLMAN, A. P. ; DEMPLE B.** Incision activity of human apurinic endonuclease (Ape) at abasic site analogs in DNA. *J. Biol. Chem.,* 1995, vol. 270, 16002-16007
- **PRASAD, R. ; BEARD, W. A. ; STRAUSS, P. R. ; WILSON, S. H.** Human DNA polymerase β deoxyribose phosphate lyase. Substrate specificity and catalytic mechanism. *J. Biol. Chem.,* 1998, vol. 273, 15263-15270
- **KROKAN et al.** *FEBS Letters,* 2000, vol. 476, 73-77
- **WOOD et al.** *Science,* 2001, vol. 291, 1284-1289
- **ABURATANI, H. ; Y. HIPPO ; T. ISHIDA ; R. TAKASHIMA ; C. MATSUBA ; T. KODAMA ; M. TAKAO ; A. YASUI ; K. YAMAMOTO ; M. ASANO.** Cloning and characterization of mammalian 8-hydroxyguanine-specific DNA glycosylase/apurinic, apyrimidinic lyase, a functional mutM homologue. *Cancer Res.,* 1997, vol. 57, 2151-2156
- **ARAI, K. ; K. MORISHITA ; K. SHINMURA ; T. KOHNO ; S. R. KIM ; T. NOHMI ; M. TANIWAKI ; S. OHWADA ; J. YOKOTA.** Cloning of a human homolog of the yeast OGG1 gene that is involved in the repair of oxidative damage. *Oncogene,* 1997, vol. 14, 2857-2861
- **ASAMI, S. ; T. HIRANO ; R. YAMAGUCHI ; Y. TOMIOKA ; H. ITOH ; H. KASAI.** Increase of a type of oxidative DNA damage, 8-hydroxyguanine, and its repair activity in human leukocytes by cigarette smoking. *Cancer Res.,* 1996, vol. 56, 2546-2549
- **ASAMI, S. ; H. MANABE ; J. MIYAKE ; Y. TSURUDOME ; T. HIRANO ; R. YAMAGUCHI ; H. ITOH ; H. KASAI.** Cigarette smoking induces an increase in oxidative DNA damage, 8-hydroxydeoxyguanosine, in a central site of the human lung. *Carcinogenesis,* 1997, vol. 18, 1763-1766
- **ATHAS, W. F. ; M. A. HEDAYATI ; G. M. MATANOSKI ; E. R. FARMER ; L. GROSSMAN.** Development and field-test validation of an assay for DNA repair in circulating human lymphocytes. *Cancer Res.,* 1991, vol. 51, 5786-5793
- **AUCKLLEY, D.H. ; CROWELL, R.E. ; HEAPHY, E.R. ; STIDLEY, C.A. ; LECHNER, J.F. ; GILLILAND, F.D. ; A., B.S.** Reduced DNA-dependent prorein kinase is associated with lung cancer. *Carcinogenesis,* 2001, vol. 22, 723-727
- **AUDEBERT, M. ; S. CHEVILLARD ; C. LEVALOIS ; G. GYAPAY ; A. VIEILLEFOND ; J. KLIJANIENKO ; P. VIELH ; A. K. EL NAGGAR ; S. OUDARD ; S. BOITEUX.** Alterations of the DNA repair gene OGG1 in human clear cell carcinomas of the kidney. *Cancer Res.,* 2000, vol. 60, 4740-4744
- **BESSHO, T. ; R. ROY ; K. YAMAMOTO ; H. KASAI ; S. NISHIMURA ; K. TANO ; S. MITRA.** Repair of 8-hydroxyguanine in DNA by mammalian N-methylpurine-DNA glycosylase. *Proc. Natl. Acad. Sci. USA,* 1993, vol. 90, 8901-8904
- **BISHOP, J. M.** Cancer: The rise of the genetic paradigm. *Genes & Dev.,* 1995, vol. 9, 1309-1315
- **BJORAS, M. ; L. LUNA ; B. JOHNSEN ; E. HOFF ; T. HAUG ; T. ROGNES ; E. SEEBERG.** Opposite base-dependent reactions of a human base excision repair enzyme on DNA containing 7,8-dihydro-8-oxoguanine and abasic sites. *EMBO J.,* 1997, vol. 16, 6314-6322
- **CHEVILLARD, S. ; J. P. RADICELLA ; C. LEVALOIS ; J. LEBEAU ; M. F. POUPON ; S. OUDARD ; B. DUTRILLAUX ; S. BOITEUX.** Mutations in OGG1, a gene involved in the repair of oxidative DNA damage, are found in human lung and kidney tumours. *Oncogene,* 1998, vol. 16, 3083-3086
- **COLLINS, A.R.** Oxidative DNA damage, antioxidants, and cancer. *BioEssays,* 1999, vol. 21, 238-246
- **CONNOR, F. ; D. BERTWISTLE ; P. J. MEE ; G. M. ROSS ; S. SWIFT ; E. GRIGORIEVA ; V. L. J. TYBULEWICZ ; A. ASHWORTH.** Tumorigenesis and a DNA repair defect in mice with a truncating Brca2 mutation. *Nature Genet,* 1997, vol. 17, 423-430
- **DIANOV, G. ; A. PRICE ; T. LINDAHL.** Generation of single-nucleotide repair patches following excision of uracil residues from DNA. *Mol. Cell Biol.,* 1992, vol. 12, 1605-1612
- **ECHOLS, H. ; M. F. GOODMAN.** Fidelity mechanisms in DNA replication. *Annu. Rev. Biochem.,* 1991, vol. 60, 477-511
- **ENGELWARD, B. P. ; G. WEEDA ; M. D. WYATT ; J. L. BROEKHOF ; J. DE WIT ; I. DONKER ; J. M. ALLAN ; B. GOLD ; J. H. HOEIJMAKERS ; L. D. SAMSON.** Base excision repair deficient mice lacking the Aag alkyladenine DNA glycosylase. *Proc. Natl. Acad. Sci. USA,* 1997, vol. 94, 13087-13092
- **FISHEL, R. ; M. K. LESCOE ; M. R. RAO ; N. G. COPELAND ; N. A. JENKINS ; J. GARBER ; M. KANE ; R. KOLODNER.** The human mutator gene homolog MSH2 and its association with hereditary nonpolyposis colon cancer. *Cell,* 1993, vol. 75, 1027-38
- **FORTINI, P. ; B. PASCUCCI ; E. PARLANTI ; R. W. SOBOL ; S. H. WILSON ; E. DOGLIOTTI.** Different DNA polymerases are involved in the short- and long-patch base excision repair in mammalian cells. *Biochemistry,* 1998, vol. 37, 3575-3580
- **FRIEDBERG, E. C. ; G. C. WALKER ; W. SIEDE.** DNA repair and mutagenesis. ASM Press, 1995

- **GAJEWSKI, E. ; G. RAO ; Z. NACKERDIEN ; M. DIZDAROGLU.** Modification of DNA bases in mammalian chromatin by radiation-generated free radicals. *Biochemistry,* 1990, vol. 29, 7876-7882
- **GORDIS, L.** Epidemiology. W. B. Saunders Co, 1996
- **GOWEN, L. C. ; A. V. AVRUTSKAYA ; A. M. LATOUR ; B. H. KOLLER ; S. A. LEADON.** BRCA1 required for transcription-coupled repair of oxidative DNA damage. *Science,* 1998, vol. 281, 1009-1012
- **HANAWALT, P. C.** Transcription-coupled repair and human disease. *Science,* 1994, vol. 266, 1957-1958
- **HANG, B. ; B. SINGER ; G. P. MARGISON ; R. H. ELDER.** Targeted deletion of alkylpurine-DNA -N-glycosylase in mice eliminates repair of 1, N6-ethenoadenine and hypoxanthine but not 3, N4-ethenocytosine or 8-oxoguanine. *Proc. Natl. Acad. Sci. USA,* 1997, vol. 94, 12869-12874
- **HAZRA, T. K. ; T. IZUMI ; L. MAIDT ; R. A. FLOYD ; S. MITRA.** The presence of two distinct 8-oxoguanine repair enzymes in human cells: their potential complementary roles in preventing mutation. *Nucleic Acids Res.,* 1998, vol. 26, 5116-5122
- **HELZLSOUER, K. J. ; E. L. HARRIS ; R. PARSHAD ; H. R. PERRY ; F. M. PROCE ; K. K. SANFORD.** DNA repair proficiency: Potential susceptibility factor for breast cancer. *J. Natl. Cancer. Inst.,* 1996, vol. 88, 754-755
- **HERNANDEZ-BOUSSARD, T. ; P. RODRIGUEZ-TOME ; R. MONTESANO ; P. HAINAUT, P.** IARC p53 mutation database: a relational database to compile and analyze p53 mutations in human tumors and cell lines. *International Agency for Research on Cancer, Hum. Mutat.,* 1999, vol. 14, 1-8
- **HOLLSTEIN, M. ; SHOMER, B. ; GREENBLATT, M. ; SOUSSI, T. ; HOVIG, E. ; MONTESANO, R. ; HARRIS, C.C.** Somatic point mutations in the p53 gene of human tumors and cell lines: updated compilation. *Nucleic Acids Res.,* 1996, vol. 24, 141-146
- **HUTCHINSON, F.** Chemical changes induced in DNA by ionizing radiation. *Prog. Nucleic Acid Res. Mol. Biol.,* 1985, vol. 32, 115-154
- **HYUN, J. W. ; J. Y. CHOI ; H. H. ZENG ; Y. S. LEE ; H. S. KIM ; S. H. YOON ; M. H. CHUNG.** Leukemic cell line, KG-1 has a functional loss of hOGG1 enzyme due to a point mutation and 8-hydroxydeoxyguanosine can kill KG-1. *Oncogene,* 2000, vol. 19, 4476-4479
- **ISHIDA, T. ; Y. HIPPO ; Y. NAKAHORI ; I. MATSUSHITA ; T. KODAMA ; S. NISHIMURA ; H. ABURATANI.** Structure and chromosome location of human OGG1, Cytogenet. *Cell Genet.,* 1999, vol. 85, 232-236
- **JYOTHISH, B. ; R. ANKATHIL ; B. CHANDINI ; B. VINODKUMAR ; G. SUNIL NAYAR ; D. DINESH ROY ; J. MADHAVAN ; M. KRISHNAN NAIR.** DNA repair proficiency: a potential marker for identification of high risk members in breast cancer families. *Cancer Lett.,* 1998, vol. 124, 9-13
- **KIM, K. ; S. BIADE ; Y. MATSUMOTO.** Involvement of flap endonuclease 1 in base excision DNA repair. *J. Biol. Chem.,* 1998, vol. 273, 8842-8848
- **KLEINBAUM, D. G.** Logistic Regression. Springer-Verlag, 1994
- **KLUNGLAND, A. ; I. ROSEWELL ; S. HOLLENBACH ; E. LARSEN ; G. DALY ; B. EPE ; E. SEEBERG ; T. LINDAHL ; D. E. BARNES.** Accumulation of premutagenic DNA lesions in mice defective in removal of oxidative base damage. *Proc. Natl. Acad. Sci. USA,* 1999, vol. 96, 13300-13305
- **KYRTOPOULOS, S.A. ; AMPATZI, P. ; DAVARIS, P. ; HARITOPOULOS, N. ; GOLEMATIS, B.** Studies in gastric carcinogenesis. IV. O6-methyguanine and its repair in normal and atrophic biopsy specimen of human gastric mucosa. Correlation of O6-methylguanine-DNA alkyltransferase activities in gastric mucosa and circulating lymphocytes. *Carcinogenesis,* 1990, vol. 11, 431-436
- **LAVAL, F.** Expression of the E. coli fpg gene in mammalian cells reduces mutagenicity of g rays. *Nucleic Acids Res.,* 1994, vol. 22, 4943-4946
- **LE PAGE, F. ; E. E. KWOH ; A. AVRUTSKAYA ; A. GENTIL ; S. A. LEADON ; A. SARASIN ; P. K. COOPER.** Transcription-coupled repair of 8-oxoguanine: requirement for XPG, TFIIH, and CSB and implications for Cockayne syndrome. *Cell,* 2000, vol. 101, 159-171
- **LE PAGE, F. ; V. RANDRIANARISON ; D. MAROT ; J. CABANNES ; M. PERRICAUDET ; J. FEUNTEUN ; A. SARASIN.** BRCA1 and BRCA2 are necessary for the transcription-coupled repair of the oxidative 8-oxoguanine lesion in human cells. *Cancer Res.,* 2000, vol. 60, 5548-5552
- **LEACH, F. S. ; N. C. NICOLAIDES ; N. PAPADOPOULOS ; B. LIU ; J. JEN ; R. PARSONS ; P. PELTOMAKI ; P. SISTONEN ; L. A. AALTONEN ; M. NYSTROMLAHTI.** Mutations of a mutS homolog in hereditary nonpolyposis colorectal cancer. *Cell,* 1993, vol. 75, 1215-1225
- **LEANDERSON, P. ; C. TAGESSON.** Cigarette smoke-induced DNA damage in cultured human lung cells: role of hydroxyl radicals and endonuclease activation. *Che. Biol. Interact.,* 1992, vol. 81, 197-208
- **LINDAHL, T.** Instability and decay of the primary structure of DNA. *Nature,* 1993, vol. 362, 709-715
- **LIPPMAN, S.M. ; SPITZ, M.R.** Lung cancer chemoprevention: An integrated approach. *J. Clin. Oncol.,* 2001, vol. 19, 74s-82s

- **LIVNEH, Z. ; O. COHEN-FIX ; R. SKALITER ; T. ELIZUR.** Replication of damaged DNA and the molecular mechanism of ultraviolet light mutagenesis. *CRC Crit. Rev. Biochem. Mol. Biol.,* 1993, vol. 28, 465-513
- **LU, R. ; H. M. NASH ; G. L. VERDINE.** A mammalian DNA repair enzyme that excises oxidatively damaged guanines maps to a locus frequently lost in lung cancer. *Curr. Biol.,* 1997, vol. 7, 397-407
- **MAKI, H. ; M. SEKIGUCHI.** MutT protein specifically hydrolyses a potent mutagenic substrate for DNA synthesis. *Nature (London,* 1992, vol. 355, 273-275
- **MATTSON, M. E. ; POLLACK, E.S. ; CULLEN, J.W.** What are the odds that smoking will kill you?. *Am. J. Public Health,* 1987, vol. 77, 425-431
- **MINNA, J.D. ; ROTH, J.A. ; GAZDAR, A.F.** Focus on lung cancer. *Cancer Cell,* 2002, vol. 1, 49-52
- **MINOWA, O. ; T. ARAI ; M. HIRANO ; Y. MONDEN ; S. NAKAI ; M. FUKUDA ; M. ITOH ; H. TAKANO ; Y. HIPPOU ; H. ABURATANI.** Mmh/Ogg1 gene inactivation results in accumulation of 8-hydroxyguanine in mice. *Proc. Natl. Acad. Sci. USA,* 2000, vol. 97, 4156-4161
- **MODRICH, P.** Mismatch repair, genetic stability, and cancer. *Science,* 1994, vol. 266, 1959-1960
- **MONDEN, Y. ; T. ARAI ; M. ASANO ; E. OHTSUKA ; H. ABURATANI ; S. NISHIMURA.** Human MMH (OGG1) Type 1a Protein Is a Major Enzyme for Repair of 8-Hydroxyguanine Lesions in Human Cells. *Biochem. Biophys. Res. Commun.,* 1999, vol. 258, 605-610
- **PARSHAD, R. ; F. M. PRICE ; V. A. BOHR ; K. H. COWANS ; J. A. ZUJEWSKI ; K. K. SANFORD.** Deficient DNA repair capacity, a predisposing factor in breast cancer. *Brit. J. Cancer,* 1996, vol. 74, 1-5
- **PARSONS, R. ; G. M. LI ; M. J. LONGLEY ; W. H. FANG ; N. PAPADOPOULOS ; J. JEN ; 1. C. A. DE ; K. W. KINZLER ; B. VOGELSTEIN ; P. MODRICH.** Hypermutability and mismatch repair deficiency in RER+ tumor cells. *Cell,* 1993, vol. 75, 1227-36
- **PATEL, K. J. ; V. P. C. C. YU ; H. LEE ; A. CORCORAN ; F. C. THISTLETHWAITE ; M. J. EVANS ; W. H. COLLEDGE ; L. S. FRIEDMAN ; B. A. J. PONDER ; A. R. VENKITARAMAN.** Involvement of Brca2 in DNA Repair. *Molecular Cell,* 1998, vol. 1, 347-357
- **PATEL, R. K. ; A. H. TRIVEDI ; D. C. ARORA ; J. M. BHATAVDEKAR ; D. D. PATEL.** DNA repair proficiency in breast cancer patients and their first-degree relatives. *Int. J. Cancer,* 1997, vol. 73, 20-24
- **PAVLOV, Y. I. ; D. T. MINNICK ; S. IZUTA ; T. A. KUNKEL.** DNA replication fidelity with 8-oxodeoxyguanosine triphosphate. *Biochemistry,* 1994, vol. 33, 4695-4701
- **RADICELLA, J. P. ; C. DHERIN ; C. DESMAZE ; M. S. FOX ; S. BOITEUX.** Cloning and characterization of hOGG1, a human homolog of the OGG1 gene of Saccharomyces cerevisiae. *Proc. Natl. Acad. Sci. USA,* 1997, vol. 94, 8010-8015
- **REARDON, J. T. ; T. BESSHO ; H. C. KUNG ; P. H. BOLTON ; A. SANCAR.** In vitro repair of oxidative DNA damage by human nucleotide excision repair system: possible explanation for neurodegeneration in xeroderma pigmentosum patients. *Proc. Natl. Acad. Sci. USA,* 1997, vol. 94, 9463-9468
- **ROLDAN-ARJONA, T. ; Y. F. WEI ; K. C. CARTER ; A. KLUNGLAND ; C. ANSELMINO ; R. P. WANG ; M. AUGUSTUS ; T. LINDAHL.** Molecular cloning and functional expression of a human cDNA encoding the antimutator enzyme 8-hydroxyguanine DNA glycosylase. *Proc. Natl. Acad. Sci. USA,* 1997, vol. 94, 8016-8020
- **ROSENQUIST, T. A. ; D. O. ZHARKOV ; A. P. GROLLMAN.** Cloning and characterization of a mammalian 8-oxoguanine DNA glycosylase. *Proc. Natl. Acad. Sci. USA,* 1997, vol. 94, 7429-7434
- **RUNGER, T. M. ; B. EPE ; K. MOLLER.** Repair of ultraviolet B and singlet oxygen-induced DNA damage in xeroderma pigmentosum cells. *J. Invest. Dermatol.,* 1995, vol. 104, 68-73
- **SAGHER, D. ; T. KARRISON ; J. L. SCHWARTZ ; R. LARSON ; P. MEIER ; B. STRAUSS.** Low O6-alkylguanine DNA alkyltransferase activity in the peripheral blood lymphocytes of patients with therapy-related acute nonlymphocytic leukemia. *Cancer Res.,* 1988, vol. 48, 3084-3089
- **SANCAR, A.** Mechanisms of DNA repair. *Science,* 1994, vol. 266, 1954-1956
- **SAVITSKY, K. ; A. BAR-SHIRA ; S. GILAD ; G. ROTMAN ; Y. ZIV ; L. VANAGAITE ; D. A. TAGLE ; S. SMITH ; T. UZIEL ; S. SFEZ.** A single Ataxia Telangiectasia gene with a product similar to PI-3 kinase. *Science,* 1995, vol. 268, 1749-1753
- **SCULLY, R. ; J. CHEN ; A. PLUG ; Y. XIAO ; D. WEAVER ; J. FEUNTEUN ; T. ASHLEY ; D. M. LIVINGSTON.** Association of BRCA1 with Rad51 in Mitotic and Meiotic cells. *Cell,* 1997, vol. 88, 265-275
- **SHARAN, S. K. ; M. MORIMATSU ; U. ALBRECHT ; D. S. LIM ; E. REGEL ; C. DINH ; A. SANDS ; G. EICHELE ; P. HASTY ; A. BRADLEY.** Embryonic lethality and radiation hypersensitivity mediated by Rad51 in mice lacking Brca2. *Nature,* 1997, vol. 386, 804-810

- **SHIBUTANI, S. ; M. TAKESHITA ; A. P. GROLLMAN.** Insertion of specific bases during DNA synthesis past the oxidation-damaged base 8-oxodG. *Nature,* 1991, vol. 349, 431-434
- **SHINMURA, K. ; T. KOHNO ; H. KASAI ; K. KODA ; H. SUGIMURA ; J. YOKOTA.** Infrequent mutations of the hOGG1 gene, that is involved in the excision of 8-hydroxyguanine in damaged DNA, in human gastric cancer, Jpn. *J. Cancer Res.,* 1998, vol. 89, 825-828
- **SINGHAL, R. K. ; R. PRASAD ; S. H. WILSON.** DNA polymerase beta conducts the gap-filling step in uracil-initiated base excision repair in a bovine testis nuclear extract. *J Biol Chem,* 1995, vol. 270, 949-957
- **SRIVASTAVA, S. ; Z. ZOU ; K. PIROLLO ; W. BLATTNER ; E. H. CHANG.** Germ-line transmission of a mutated p53 in a cancer-prone family with Li-Fraumeni syndrome. *Nature (London,* 1990, vol. 348, 747-749
- **STRAUSS, B. S.** Translesion DNA synthesis: polymerase response to altered nucleotide. *Cancer Surv.,* 1985, vol. 4, 493-516
- **VANDENBROUCKE, J. P. ; T. KOSTER ; E. BRIET ; P. H. REITSMA ; R. M. BERTINA ; F. R. ROSENDAAL.** Increased risk of venous thrombosis in oral-contraceptive users who are carriers of factor V Leiden mutation. *Lancet,* 1994, vol. 344, 1453-1457
- **VOGELSTEIN, B. ; K. W. KINZLER.** The multistep nature of cancer. *Trends Genet.,* 1993, vol. 9, 138-141
- **WEEDA, G. ; J. H. J. HOEIJMAKERS ; D. BOOTSMA.** Genes controlling nucleotide excision repair in eukaryotic cells. *BioEssays,* 1993, vol. 15, 249-258
- **WEI, Q. ; L. CHENG ; W. K. HONG ; M. R. SPITZ.** Reduced DNA repair capacity in lung cancer patients. *Cancer Res.,* 1996, vol. 56, 4103-4107
- **WEI, Q. ; G. M. MATANOSKI ; E. R. FARMER ; M. A. HEDAYATI ; L. GROSSMAN.** DNA repair and aging in basal cell carcinoma: a molecular epidemiology study. *Proc. Natl. Acad. Sci. U S A,* 1993, vol. 90, 1614-1618
- **WEI, Q. ; G. M. MATANOSKI ; E. R. FARMER ; M. A. HEDAYATI ; L. GROSSMAN.** DNA repair and susceptibility to basal cell carcinoma: a case-control study. *Am. J. Epidemiol.,* 1994, vol. 140, 598-607
- **WEINBERG, R. A.** Oncogenes, antioncogenes, and the molecular bases of multistep carcinogenesis. *Cancer Res.,* 1989, vol. 49, 3713-3721
- **WIKMAN, H. ; A. RISCH ; F. KLIMEK ; P. SCHMEZER ; B. SPIEGELHALDER ; H. DIENEMANN ; K. KAYSER ; V. SCHULZ ; P. DRINGS ; H. BARTSCH.** OGG1 polymorphism and loss of heterozygosity (LOH): significance for lung cancer susceptibility in a caucasian population. *Int. J. Cancer,* 2000, vol. 88, 932-937
- **WOOD, M. L. ; M. DIZDAROGLU ; E. GAJEWSKI ; J. M. ESSIGMANN.** Mechanistic studies of ionizing radiation and oxidative mutagenesis: Genetic effects of a single 8-hydroxyguanine (7-hydro-8-oxo-guanine) residue inserted at a unique site in a viral genome. *Biochemistry,* 1990, vol. 29, 7024-7032